# EUROPEAN PATENT SPECIFICATION

(11) **EP 4 590 366 B1**
(45) Date of publication and mention of the grant of the patent: **14.10.2026**
(21) Application number: 23730087.6
(22) Date of filing: 31.05.2023
(51) Int. Cl.: A61M 5/168, A61M 5/44, G01F 1/68

(54) **FLOW SENSING DEVICE AND METHOD**
VORRICHTUNG UND VERFAHREN ZUR DURCHFLUSSMESSUNG
DISPOSITIF ET PROCEDE DE DETECTION DE DEBIT

(30) Priority: 20.09.2022 US 202263376295 P; 04.10.2022 US 202263378307 P
(43) Date of publication of application: 30.07.2025
(73) Proprietor: Lingane, Paul James, Palomar Park, California 94062 (US); Nvention Ltd, London E16 2DQ (GB)
(72) Inventor: LINGANE, Paul James, Palomar Park, California 94062 (US)
(74) Representative: Elkington and Fife LLP
(86) International application number: PCT/EP2023/064607
(87) International publication number: WO 2024/061484

(56) References cited:
- EP-A1- 0 473 868
- WO-A1-89/12216
- US-A1- 2019 125 966
- US-A1- 2022 184 301

## Description

### FIELD OF THE INVENTION

The present application relates to a flow sensing device and method, in particular for use in sensing flow through a tubing.

### BACKGROUND OF THE INVENTION

Plastic or silicone tubing lines are commonly used in medicine to infuse or flush liquids into or out of a patient or to move fluids within a patient's body. The flow of fluid in such tubing can be easily interrupted by a number of causes. Movement of the patient causing a disturbance of an intravenous (IV) needle at the infusion site can stop flow, as can an occlusion of the tubing caused by a kink or valve failure or blockage. Transporting a patient connected to an IV bag may require that bag be taken down from a hanger during the transport, stopping IV flow. Flow can also be intentionally stopped during a procedure and then inadvertently kept off instead of being restarted when required. IV bags can run low or empty so infusion fluid no longer flows. Flow into arteries requires pressurizing the source fluid bag to overcome arterial pressure, and that pressure may become exhausted before the fluid bag is empty, causing flow to stop.

There is a need to have a way to monitor fluid flow in medical tubing to make sure that it is flowing when it should be.

Traditionally, flow in infusion tubing has been monitored by the use of a drip chamber which permits an observer to visually see fluid dropping in the chamber as an indication that there is flow in the tubing. There are also existing devices that clamp onto the drip chamber to optically observe the drops, such as the Drip Assist from Shift Labs or the Monidrop from Monidor. Alternatively, infusion pumps have means of detecting when fluid flow is blocked by measuring an increase in back-pressure if the tubing is occluded, or by optical or ultrasonic means.

In some situations it may be important to provide a warning if fluid flow has been stopped inadvertently, and the above methods of monitoring fluid flow have limitations.

One limitation is the cost and size of the above devices if they were to be used in a situation requiring only short-term monitoring (where a small disposable device may be the most appropriate), or in a mass casualty event where many such devices must be deployed at once, or where such a device must be carried in a crowded medic's bag and so must be small and light-weight. Transport situations in which there is vibration can result in difficulty monitoring flow using such existing devices because the vibration can affect the regularity of drops in a drip chamber.

There are situations where an infusion pump is not used, and instead simple gravity is used to flow liquids from a bag into the patient, or where a pressurized bag or vessel is used to source the liquids. In these cases, if a drip chamber is used it must be visually monitored to check that flow continues. However, this is not practical at night or in a dimly lit fluoroscopy theater, as the drops cannot be seen. This approach is also not feasible in the case that the drip chamber is not oriented vertically to allow drops to fall freely. It also requires inspection of the drip chamber at close range in order to see the drops, adding risk to nurses if the patient is infectious.

*In vivo* applications where the medical tubing is implanted in the body present other challenges such as size and reliability and how to power a device through the skin.

Sometimes, a very low flow rate, such as 5 ml per hour, is used just to keep open (TKO) a vascular access line should it be needed later to infuse drugs. TKO lines have very slow drop rates in a drip chamber, such as one drop per minute. Thus checking flow continuance in these cases is difficult; the drip chamber must be watched for a long time before any single drop will form and then fall off. The drip chamber may be overlooked if personnel are too busy with other things, such as during an emergency or in battlefield settings or mass evacuations where one medic is caring for many patients.

There are some settings where a drip chamber is not used at all, such as in blood donation centers, where, for example, the nurse can tell that flow is normal simply by monitoring the filling of the collection bag over time to see if it is progressing. Some ambulatory infusion systems such as the Baxter INFUSOR or INTERMATE systems use a pressurized vessel to propel the liquid and omit any drip chamber at all. In these cases, the only way to monitor flow is to observe emptying of the liquid in the pressure vessel over a long period of time.

Other situations where very low flow rates, or intermittent flow occurs, is when monitoring effusions from the body or fluid flow within the body such as urine, cerebrospinal fluid, blood, lymph or other fluid flows or discharges. Monitoring flow under these conditions can be difficult if there is no direct means to do so or if the flow is not continuous.

It has further been recognized by the inventor that, in certain settings, such as military or emergency settings, the ambient light level may be too low to visually see the drops in a drip chamber, and keeping a drip chamber vertical so the drops fall properly is not always possible during emergency patient transport. In military settings in a combat zone, any device that emits light or sound is prohibited as it would give away the position of the soldier.

Thermally-based mass-flow sensors, properly designed, can overcome some of the above problems and are known to practitioners of the art. For example, DE 3827444 A1 describes a flow monitor for infusion lines that uses a heater element and two or three thermal sensors to determine occurrence of flow, flow rate and direction of flow.

However, known devices have slow responsiveness to detecting flow stopping or starting, and are mostly useful for measuring a quantitative flow rate of fluid. In some situations, such as when a device is needed to monitor the intentional stopping and starting of a fluid, it is particularly beneficial to detect and report the resumption of flow as quickly as possible to avoid giving unnecessary warning alarms.

Developments in this field would be of value.

EP 0473868 A1 discloses a flow monitor for a medium flowing in a line, with a line constriction in the line run, with a heating element and with a temperature probe, the heating element and the temperature probe opposite each other at the pipe constriction.

US 2022/184301 A1 discloses a flow meter for an electric drug injection pump and a method for measuring a flow using the flow meter, the flow meter includes a lower case, an upper case and a temperature sensor part.

WO 89/12216 A1 discloses an apparatus for detecting leakage or unintended consumption in pipe systems, wherein a mode of operation is based on heat transmission.

### SUMMARY OF THE INVENTION

The invention is defined by the claims.

At least one aspect of the invention provides a device for sensing a fluid flow through a tubing, for example through a medical tubing line, comprising: a heating element and at least a first temperature sensing means, T3; and a controller or control arrangement/means operatively coupled to the heating element and at least first temperature sensor. In at least one mode of operation, the controller is adapted to control the heating element for dissipating heating power; and detect a flow parameter or condition based at least in part on an output from the at least first temperature sensor. For example, this may be based on sensing variations in a temperature signal output from the temperature sensor, or a function or correlate thereof. The controlling the heating element to dissipate heating power and the detecting the flow parameter or condition may be done simultaneously in some examples.

In some embodiments, the device is arranged in use to hold the heating element positioned in thermal communication with the fluid in the tubing at a first location, and the temperature sensor positioned in thermal communication with the fluid in the tubing at a second location. The first location may be (substantially) longitudinally aligned with the second location along the lumen of the tubing. The first location may be circumferentially offset from the second relative to the lumen of the tubing. The second location is preferably substantially radially opposite or facing the first location across the lumen of the tubing. As will be explained later, this relative positioning of the heating element and temperature sensor offers significant technical advantages.

Various aspects and embodiments of the invention are set out herein. It is to be understood that features, properties or embodiments described in relation to one aspect of the invention may be incorporated or combined or included in any other aspect of the invention. The interoperability of the different optional features to the different aspects of the invention will be apparent from reading the detailed description.

The controller may further generate an output indicative of the detected flow parameter or condition, for example a user-perceptible output or an electronic data output.

At least some embodiments of the present invention propose provision of a temperature sensor substantially radially or diametrically opposite the heater. In known thermal mass flow sensors for infusion lines, temperature sensors need to be provided upstream and downstream of the heater in order to detect speed and direction of flow. However, a problem with these known devices is that there is a high latency time between actual flow start and detection of flow start. By providing a sensor at this novel location, radially opposite the heater, rather than axially upstream or downstream from it, the thermal path between the heater and sensor is reduced, meaning that flow start can be sensed with a much shorter latency time.

Furthermore, the same effect cannot simply be achieved by moving an upstream or downstream sensor closer to the heater, for at least two reasons. First, if an up/downstream sensor is moved so that the axial distance between the heater and sensor matches the diameter distance across the tube, then the axial thermal path through the wall of the tubing is also commensurately reduced in distance. This leads to high thermal interference (heat directly travelling along the tube wall to the sensor without going through the fluid), which affects results because that interfering heat will obscure desired changes to the sensor by flow start/stop. By contrast, with a radially opposed sensor, the interfering thermal path directly through the tubing wall is longer than the thermal path through the fluid so that the fluid thermal path dominates the response of the sensor. Second, it is not just the thermal path length between sensor and heater which is significant, but also the total volume of fluid present between the heater and the sensor. With thermal mass based measurement, detected temperature changes rely on the heating of the whole volume of fluid present between the heater and the sensor. With a sensor axially displaced from the heater, the whole cylindrical volume of fluid between the heater and sensor must be heated before the sensor will detect the temperature change. By contrast, with a radially opposed heater, only the smaller volume of fluid extending radially between the heater and sensor needs to be heated. As a consequence, this further reduces time latency in detection of flow start. The optional compression of the tubing (discussed below) may further reduce the volume of fluid in the thermal path between the heater and the temperature sensor, making the system even more sensitive to flow changes at very low volumetric flow rates.

Another reason the same effect cannot be achieved by simply moving a downstream sensor closer to the heater can be explained as follows. The temperature, as sensed at the radially opposite position, goes up when flow stops as the heater warms the fluid and that warmth crosses the tubing lumen diameter by thermal conduction through the fluid. Temperature falls when flow resumes as fresh cooler fluid replaces the warmer fluid interposed between the heater and temperature sensor. The signal polarity is in the opposite direction for prior art devices that have a temperature sensor downstream: those sensors warm up when flow occurs and they return down towards ambient temperature when flow stops and they are no longer influenced by warmed fluid. If one were to move a downstream sensor closer to the heater, at some point the signal polarity becomes indeterminate as it will end up with a response with characteristics of both a downstream and an opposed sensor: fluid that is stagnant will be heated, that heat will spread out from the heater and some of that heat will reach a downstream sensor if it is right next to the heater.

The temperature modulation at the radially opposite location is an order of magnitude greater than at downstream locations, resulting in a much larger signal per unit of heater power. This larger signal and power efficiency allows for a much lower heater power, so battery power or wireless power delivery become viable options as it can now run for hours on one battery charge or be powered at a distance by wireless means. One experiment showed that a temperature sensor radially across the tubing from the heater had 12 times the temperature response than a downstream sensor.

Putting the temperature sensor radially across the lumen of the tubing from the heater optimizes the response of the device to detect start and stop of flow. But note that a temperature sensor in this radially opposed position cannot be used to measure the direction of flow and its measurement sensitivity to flow rate is best at only very low flow rates and it loses flow rate sensitivity as flow rate increases.

In some embodiments, the heater and temperature sensor are held applied against the outside of the tubing at a first and second contact area, these corresponding to the aforementioned first and second location. It is noted that, where reference is made to a first and second contact area, at minimum all that is needed is that the heating element and temperature sensor are in thermal contact with the fluid at respective points in the tubing which are substantially radially opposite. Thus, throughout this disclosure, reference to contact areas can be replaced equivalently with reference to contact points without loss of functionality.

In this disclosure, by 'radially opposite' may be understood as meaning the same as 'radially facing'.

In this disclosure, by 'radially' is meant along a radial dimension of the tubing, meaning a dimension from one point on the outside, inside or interior of the wall of the tubing to an opposite point on the outside, inside or interior of the wall of the tubing. Radially opposite in this context for example means the same as diametrically opposite or diametrically facing.

The tubing line may be understood as having an axial/longitudinal dimension, along a direction of fluid flow through the tubing, and radial and circumferential dimensions orthogonal to the axial dimension.

The first location or contact area and second location or contact area may be substantially axially aligned. In other words, both contact areas are aligned at a same axial location along the length of the tubing. In other words, there is substantially no axial displacement between the position of the first contact area and the position of the second contact area.

As noted above, the first location and second location are preferably substantially radially opposed. Substantially radially opposite may mean for example radially opposite plus or minus some tolerance, for example, +/- 5-10 degrees, i.e., the second location or contact area is positioned within an arcuate section of the tubing wall, the arcuate section having its center radially opposite the first location or contact area, and subtended by an angle at the center of the tubing (the tubing axial axis) of 5-10 degrees.

As will be explained below, the controller may be adapted, in some control modes or flow states, to control the heating element to dissipate a substantially constant heating power. Driving the heating element with a substantially constant heating power means a substantially constant heating power over time, e.g. over a duration of an operation session of the device. Substantially constant power means a substantially constant power level; in other words substantially uniform power; in other words substantially invariant power. The advantage of using a substantially constant heating power, as opposed to a constant heater temperature, is that in the former situation the temperature of the heater will rise a fixed increment above the ambient temperature based upon the amount of power being dissipated. **In** this device, changes in flow affect the difference in temperature between the heater and the temperature sensor, so having the heater temperature track any ambient temperature changes with a fixed temperature offset will tend to compensate for variation in ambient temperature. **In** an extreme example, used to illustrate this concept only, if a fixed temperature heater were used (as opposed to fixed power) and the ambient temperature were to rise to the temperature of the heater, the temperature sensor would lose all sensitivity to flow because the heater would no longer be putting energy into the fluid. **In** this scenario, if instead fixed power is used, the heater temperature would just rise as the ambient temperature rises, and the temperature sensor would still be responsive to changes in fluid flow. Thus, a constant power heater needs to only raise the fluid temperature a few degrees above whatever the ambient temperature is, whereas a constant temperature heater must always operate at a temperature substantially above the highest ambient temperature anticipated, and thus consume much more battery energy.

**In** certain applications where the ambient temperature is known, such as when the heater and tubing is embedded in a patient and its ambient temperature is the relatively fixed body temperature, a constant heater temperature that is distinctly above the ambient temperature may be used with the same effect.

Substantially constant heating power means for example constant heating power +/- 5%, more preferably +/- 3%, even more preferably +/- 1%.

Substantially constant may mean for example a constant time-average power, for example over a pre-defined moving average window. Substantially constant may mean for example a constant baseline power, for example with some fluctuation thereabout.

The controller may in certain control modes control the heating element to dissipate a substantially constant total thermal output power. Assuming that the heating element has a near 100% efficiency, then the total electrical input power to the heater can be assumed to be all converted to heating power. In this case, controlling the heating element to dissipate a substantially constant heating power simply means supplying/driving the heating element with a substantially constant electrical input power.

Detecting the fluid flow parameter or condition may be based on sensing variations in an output from the temperature sensor, or a correlate or function thereof, while the heating element is being controlled.

The controller could be provided by circuitry, by one or more microprocessors, or by a combination of both. Circuitry of the controller may be distributed, so that the controller is not formed by a single integral unit. For example, for a device where the heater and temperature sensor are embedded in the body, there may be elements of the controller inside the body and elements of the controller that are outside the body. The controller in this case may be understood as a control means or control arrangement.

In an advantageous set of embodiments, the controller is adapted to: control the heating element to dissipate heating power; and detect a flow parameter or condition based on at least an output from the temperature sensor, and wherein the controller is further adapted, in at least one phase of operation, to adjust a heating power output or dissipation of the heating element based upon changes in the detected flow parameter or condition.

It is the recognition of the inventor that a different heating pattern or state may be sufficient for detection of changes in the flow condition depending upon a current flow condition of the fluid. This may be particularly the case where detection of the flow condition comprises classification of the flow into one of a discrete set of possible flow states/conditions, for example, flow or no-flow. The general insight is that it may be possible to save power and/or reduce overheating of the fluid by adjusting the heater to only the minimum necessary in order to detect a change of the current flow state to a different flow state. The level of heating necessary to be able to do this depends on the flow state because, the faster the flow, the more heat is swept away through flow of the fluid. By using knowledge of a current flow state, more intelligent control of the heating element can be achieved.

In some embodiments, the controller is adapted to detect at least a flow stop/start condition (flow or no-flow). The adjusting of the heating power dissipation in dependence upon changes in the detected flow parameter or condition may in this case comprise reducing the heating power output or dissipation following detection of a flow stop condition.

It is the recognition of the inventor that, once flow has stopped, if the heater remains emitting heat at uniform power dissipation, the temperature of the fluid in the tubing, and the temperature recorded at the temperature sensor, will keep rising to a high level. This continually increasing temperature is not necessary for sensing purposes, and furthermore, at a certain point, may cause problems of overheating of the fluid and tubing. It is therefore proposed in accordance with one or more embodiments to bring the level of heating power down during periods where flow has ceased. Such periods could be fairly extended in practice, for example in a case where multiple infusion lines are being operated at once, and at some periods a subset are flowing and a subset are not. It would be beneficial to automatically detect a no-flow condition and bring the heating power level to a lower level for the duration thereof.

For example, following detection of a change from a flow start state to a flow stop state, the controller may lower the power dissipation to a lower state. Restart of flow in such a power state can still be adequately detected as a fluctuation or inflection in the temperature signal for example.

Advantageously, in some embodiments, the controller is adapted to selectively operate the heating element in one of at least two heating power modes: a higher/standard heating power mode and lower heating power mode, and wherein at least an average (e.g. time average) heating power dissipation in the higher heating power mode is greater than an average heating power dissipation in the lower heating power mode. The adjusting of the heating power dissipation in dependence upon changes in the detected flow parameter or condition may comprise at least switching to the lower heating power mode following detection of a flow stop condition, for example following detection of a change from a flow start to a flow stop condition (flow to no-flow). The heating power dissipation in the lower heating power mode is preferably non-zero, for example so that detection of flow re-start can still be made.

In some embodiments, when the current flow condition/state is a flow start state (i.e. there is non-zero flow), the controller may be adapted to operate the heating element in the higher power mode. For example, following detection of a flow start state, the controller may be adapted to operate the heating element in the higher power mode. The inventor has found that, when there is even relatively slow continuous flow, the temperature measurement fairly quickly trends toward an equilibrium level which is significantly below the upper levels reached if the heater is left at the same power level during flow stop. Thus overheating is not an issue when there is flow. Furthermore, during even slow flow, the heat loss to the fluid motion is very significant, meaning that a relatively higher heating power dissipation is preferred for purposes of obtaining a strong sensing signal.

In some embodiments, the controller may be adapted to operate the heating element by default in the higher heating power mode, and to switch to the lower heating power mode only upon detection of a flow stop state. In some embodiments, the device may by default operate in the higher power mode upon switch-on or start up, obtain an initial determination as to a flow condition or state of the fluid, and then switch to the lower power mode if the detected flow condition or state is a no-flow state.

In some embodiments, in the higher power mode, the controller is adapted to control the heating element to dissipate a substantially constant heating power. In other words, the amount of heat put out per unit time is substantially constant. In other words, when the flow is in a flow start state, the controller may be adapted to control the heating element to dissipate a substantially constant heating power. However, this option is not essential.

In the lower power mode, the heating power dissipation might also be controlled to be substantially constant, but at a lower level, or it might be controlled in a more variable way, for example in dependence upon signals derived from one or more temperature sensors.

In some embodiments, the controller is adapted to reduce the power dissipation of the heating element to the lower power mode responsive to detection of persistence of the flow stop condition for a threshold time period or a threshold number of temperature signal sample points, and/or responsive to detection of the output from the first temperature sensor, or a correlate/function thereof, exceeding a threshold level.

In some embodiments, when the heating power dissipation is in the lower power mode, the controller may be adapted to detect transition of the fluid flow from the flow stop condition to the flow start condition based on: detecting a negative inflection in an output from the first temperature sensor or a correlate/function thereof; or based on detecting a negative gradient in the first temperature sensor signal, or a correlate/function thereof, exceeding or crossing a pre-defined threshold; or based on the first temperature signal, or a correlate/function thereof, falling below or crossing a threshold temperature. Thus, when flow is stopped, detection of a change to a flow start condition can be detected via signal features which are detectable even at relatively low levels of heater power.

It is noted that, in this disclosure, reference to a temperature signal "or a correlate/function thereof" means the temperature signal or a function of the temperature signal, for example a computed correlate of the temperature signal. For example, it may comprise a signal devised based on an output from the relevant temperature sensor and optionally one or more further temperature sensors. For example, it may comprise an output from the first temperature sensor adjusted for ambient, and/or adjusted according to up/downstream temperatures or gravitationally higher/lower temperatures in any of the methods described later.

After the power is reduced, the temperature sensor signal may exhibit a slow downward trend, tending toward a steady state level. Upon re-start of fluid flow, the temperature will exhibit a sudden fluctuation downward, wherein the negative trend will inflect to a steeper negative gradient.

In some embodiments, the controller is adapted to return the heating element to the higher heating power dissipation mode following detection of a flow start condition. When the controller detects the flow condition change from flow stop condition to flow start condition, the previous change to the reduced power dissipation state may thus be reversed.

There are different options for how the heating element and temperature sensors may be held by the device in their positions relative to the tubing. There may be some means by which the elements are supported in use in relevant positions. In some embodiments, the device may include at least a section of the tubing, and wherein the heating element and the at least one temperature sensor are each coupled to an outside wall of the at least section of tubing, at least partially integrated/embedded inside a wall of the at least section of tubing, or are mounted inside the lumen of the at least section of tubing. In the latter case, they might be attached or fixed to an interior wall of the tubing, or might be mounted to a supporting frame which is disposed inside the lumen and wherein the frame is coupled to the tubing for example. In further embodiments, the device comprises a housing or support structure adapted to hold the heating element and the at least one temperature sensor in contact with an outside wall of the at least section of tubing at a first contact area and a second contact area respectively. This option has the benefit that the device can be provided without the tubing included, meaning that it can be fitted to pre-existing tubings, in accordance with requirements.

With regards to the latter option, in one set of embodiments, the device may comprise a housing adapted to couple, for example removably, to an outside wall of the tubing, the housing accommodating the heating element and the at least one temperature sensor. The housing, when coupled to the tubing, may be adapted to hold the heating element in contact with, e.g. an outside wall of, the tubing at a first contact area, and hold the temperature sensor in contact with, e.g. the outside wall of, the tubing at a second contact area. The second contact area is preferably substantially radially opposite the first contact area across the lumen of the tubing. In operation, the controller is adapted to (e.g., simultaneously) control the heating element to dissipate a heating power, and detect a flow parameter or condition based on an output from the temperature sensor. Any of the features or options described above or below may be combined with this embodiment.

In some embodiments, the housing may be adapted to removably couple to the tubing but this is not essential.

In some embodiments, the housing is adapted for coupling to a tubing line having an outside diameter between 1 mm and 10 mm. In some embodiments, the housing is adapted to permit coupling to tubing of a range of different outside diameters, for example between 1 mm and 10 mm.

An alternative embodiment of the device may comprise a heating element and at least one temperature sensor that are permanently affixed to the tubing on the outside wall of the tubing or inside the tubing wall or inside the lumen of the tubing. The heating element may be held in a first position on the tubing at a first contact area, and the temperature sensor might be held on the tubing at a second position at a second contact area. The second contact area may be substantially radially opposite the first contact area across the lumen of the tubing.

In at least one set of embodiments, in addition to the at least first temperature sensor, the device may include or more further temperature sensors. The flow parameter or condition may be determined using outputs from the first temperature sensor in combination with those of the one or more further temperature sensors, e.g. based on a function of respective outputs of the first temperature sensor and the one or more further temperature sensors.

For example, in some embodiments, the device may further include at least one further temperature sensor, and wherein, the at least one further temperature sensor is positioned for sensing either: an ambient temperature in the environment of the device, or a temperature of the fluid at a location upstream from the heating element. If the second temperature sensor is for sensing an ambient temperature in the environment, it may be spaced from the wall of the tubing in some cases, i.e. not in contact with the tubing. If the second temperature sensor is for sensing the temperature of the fluid upstream from the heating element, it may be positioned to be in thermal communication with the fluid at a location axially displaced from the axial location of the first temperature sensor and the heating element.

The controller may be adapted to detect the flow parameter or condition based on outputs of both the first temperature sensor and the at least one further temperature sensor, for example based on compensating a temperature change measurement of the first temperature sensor using temperature change measurements of the at least one further temperature sensor.

In other words, the second temperature sensor may be used as a baseline temperature, and temperature change measurements detected at the first temperature sensor are offset or calibrated against temperature change measurements of the baseline second temperature sensor. In particular, a temperature change at the first temperature sensor, applied against the outside or inside of the tube, reflects both changes in the ambient and changes in the flow, since both will alter the measured temperature. Only the flow-induced temperature changes are desired for the device. Therefore, providing a second temperature sensor which is positioned so as to be only sensitive to the ambient temperature changes, or changes in the inlet temperature level of the fluid (where the inlet for example means the point of inflow into the section of the tubing retained by the housing), can be of benefit as the temperature change readings at the first temperature sensor can be offset or compensated using temperature change readings at the second temperature sensor readings. The resulting compensated temperature reading is reflective of substantially only flow-related temperature modifications.

The compensation of the temperature change measurement may be achieved by a resistor divider circuit in some embodiments, wherein both temperature sensors are thermistors and the first temperature sensor and second temperature sensor are arranged in a resistor divider arrangement, and the voltage output from the resistor divider arrangement is used to determine the flow parameter or condition. For example, the controller detects changes in the output from the resistor divider arrangement. A change in the resulting output level or a change in slope may be taken as indicative of a change in flow stop/start condition for example.

In some embodiments where the device is embedded in the body of the patient or animal the assumption may be made that the baseline temperature of the ambient surrounding the heater is at the normal temperature of the body corpus.

In some embodiments, the device may be arranged in use to hold the at least one further temperature sensor at a position for sensing a temperature of the fluid at a location upstream from the heating element, and wherein the controller is adapted to detect the flow parameter or condition based on a ratio or a difference between the first temperature sensor signal, or a function/correlate thereof, and the further temperature sensor signal, or a function/correlate thereof. Preferably, the controller may be adapted to detect at least a transition from a flow start condition to a flow stop condition based on a ratio or a difference between the first temperature sensor signal, or a function/correlate thereof, and the second temperature signal, or a function/correlate thereof.

In some embodiments, the device further includes at least one further temperature sensor, and wherein the device is arranged in use such that the at least one further temperature sensor is positioned for sensing a temperature of the fluid at a location downstream from the heating element, and wherein the controller is adapted to detect the flow parameter or condition based on outputs of both the first temperature sensor and the at least one further temperature sensor. When flow changes from flow stop to flow start, there is a transient upward peak or inflection in the temperature signal at the downstream location as the bolus of fluid which has been subject to heating by the heating element passes by the downstream location. Thus, upon flow re-start, the temperature at the heater location trends downward and the temperature at the downstream location temporarily trends upward. This reciprocal correlated temperature pattern can be used to strengthen or speed up detection of flow start. In particular, in some embodiments, the controller is adapted to detect at least a transition from a flow stop condition to a flow start condition based on a ratio or a difference between the first temperature sensor signal, or a function/correlate thereof, and the further temperature sensor signal, or a function/correlate thereof.

In some embodiments, the device comprises at least a first further temperature sensor and a second further temperature sensor. The device may be adapted in use so as to hold the first further temperature sensor positioned at a location longitudinally offset from the heating element along the lumen of the tubing in a first direction (for example for sensing a temperature of the fluid either upstream or downstream of the heating element). The device may be adapted in use such that the second further temperature sensor is held at a position longitudinally offset from the heating element along the lumen of the tubing in a second direction opposite to the first (for example for sensing a temperature of the fluid either downstream or upstream).

By providing further temperature sensors disposed laterally spaced or longitudinally offset on either side of the heater element, this allows greater flexibility in the detection of the flow parameter or condition. For example, the upstream sensor may be used in detecting flow stop and the downstream sensor may be used in detecting flow start.

In some embodiments, the device can be designed to be used in a specific orientation relative to flow. Therefore, in these cases, it can be known in advance which of the further sensors is arranged for sensing temperature upstream and which is arranged for sensing temperature downstream, on the assumption that the user installs the device in a pre-defined orientation relative to flow. In this case, optionally, the controller may be configured to use the upstream sensor in ratio/difference with the first sensor, T3, to detect transition from flow to flow stop, and to use the downstream sensor in ratio/difference with the first sensor, T3, to detect transition from flow stop to flow start.

**In** other embodiments, functionality might be implemented to make the device orientation-independent, so that it does not matter which way round the sensor is installed relative to flow.

**In** this case, in some embodiments, the controller may be adapted to determine which of the first and second further temperature sensors is located upstream of the heating element and which is located downstream of the heating element.
Following this, the selection option mentioned above may be used. The detection might be done automatically (e.g. based on a comparison of temperature signal patterns of the first and second further temperature sensors) or might be done using a user input (e.g. a user might press a switch or a button to indicate direction for example).

In further embodiments, the controller may use an operation mode which is not dependent on knowing which sensor is upstream and which is downstream.

In some embodiments, the controller may be adapted to: detect at least a transition from a flow start condition to a flow stop condition based on a ratio or a difference between the first temperature sensor signal, or a function/correlate thereof, and the upstream temperature sensor signal, or a function/correlate thereof; and/or detect at least a transition from a flow stop condition to a flow start condition based on a ratio or a difference between the first temperature sensor signal, or a function/correlate thereof, and the downstream temperature sensor signal, or a function/correlate thereof.

When flow stops, the upstream temperature sensor can be expected to exhibit a slight upward fluctuation in temperature while, when flow re-starts, the downstream temperature sensor can be expected to exhibit a slight upward fluctuation in temperature. Thus, incorporating these sensor readings into the detection can make detection faster and/or more sensitive.

In addition to or instead of any of the upstream-downstream sensor selection possibilities, also the relative gravitational positioning of the further sensors (those longitudinally offset from the first sensor) can be taken into account in selecting which sensors are used in detecting changes in flow condition/state. For example, a gravitationally lower sensor may be selected to avoid issues with convective heating effects acting on the gravitationally higher sensor. In some embodiments, a non-binary selection can be made, for example by using a weighted combination of the outputs of the two further sensors with weightings dependent on angle of inclination of the device and/or upon a time since flow stopped. Alternatively, a compensation may be applied to an output from the gravitationally higher sensor to compensate for convective heating effects, and then the gravitationally higher sensor could be used by itself, in conjunction with the first sensor, or a selection could be made between the two further sensors based solely on which is upstream and which is downstream, in the manner discussed above. Which sensor is gravitationally higher can be determined for example based on including a local orientation sensor integrated in the device, or based on simply determining which of the further sensors has a lower output temperature than the other, for example during a flow stop state.

Thus, in some embodiments, the device may further comprise an orientation sensing means, for example comprising an accelerometer or inertial measurement unit (IMU) or inclinometer, for sensing an orientation of the device.

The controller may in some embodiments be adapted to determine which of the first and second further temperature sensors is located gravitationally higher than the other of the temperature sensors.

Using this information, in some embodiments, the controller may be adapted to determine the flow parameter or condition based on a difference or ratio between an output from the gravitationally lower of the first and second further temperature sensors and an output of the first temperature sensor, T3. This avoids any inaccuracy arising from convective heating effects on the gravitationally higher sensor. The controller may be adapted to use the gravitationally lower sensor in this way at least when the flow condition or state is detected to be in a flow stop condition.

In some embodiments, the information may additionally or alternatively be used to compensate for convective heating effects between the heater and the temperature sensor located gravitationally above the heater. While fluid is flowing, convective heating effects are negligible because the flow of the fluid overwhelms any convective currents that might flow. However, when flow stops, convective heating between the heater and any further temperature sensor which is gravitationally above the heater may become non-negligible. Thus, it is proposed in some embodiments to include means for measuring an angle of inclination between the heater and the gravitationally higher sensor and to compute an estimated convective heating contribution to the temperature measurements at the gravitationally higher temperature sensor.

For example, in some embodiments, the device comprises an orientation sensing means, for example comprising an accelerometer or inertial measurement unit (IMU) or inclinometer, for sensing an orientation of the device, and wherein the orientation sensing means is adapted to generate an orientation output indicative of an angle of inclination of the device. Following detection of a flow stop condition, the controller may be adapted to determine a predicted convective heating influence on an output of the further temperature sensor which is gravitationally higher based on application of a model or function which defines pre-determined mappings between: inputs comprising an angle of inclination of the device, and a time duration since a beginning of the flow stop condition; and an output comprising a predicted additional temperature component of the output of the further temperature sensor which is gravitationally higher. The controller may be further adapted to: apply a correction/compensation to a temperature output from the gravitationally higher of the further temperature sensors based on an output from the model. In particular, this correction/compensation may be to negate or offset the estimated convective heating contribution to the temperature output from the gravitationally higher temperature sensor.

Additionally or alternatively, in some embodiments, the orientation sensing means is adapted to generate an orientation output indicative of an angle of inclination of the device relative to a gravitational vertical direction. The controller may be adapted to determine the flow parameter or condition based on a ratio or difference between (i) an output from the first temperature sensor, T3, and (ii) a weighted sum of outputs of the first and second further temperature sensors. The weightings of the weighted sum may be determined based on a pre-determined mapping between (a) the weighting values, and (b) a time duration since flow was detected to have stopped and/or the angle of inclination of the device.

A further possibility, as an alternative to the above considerations regarding which of the further sensors is upstream/downstream and which is gravitationally higher or lower, would be to determine which of the two further sensors has the lower temperature output, and detect flow stop/start state based on a ratio or difference between the output of the first temperature sensor, T3, and this lower-reading temperature sensor, and based on one or more thresholds corresponding to different flow parameters or conditions.

Thus, in some embodiments, the controller may be adapted to: compare the temperature signal outputs of the first and second further temperature sensors; select, from the first and second further sensors, the sensor with the lower temperature output; and determine the flow parameter or condition based on a difference or ratio between an output from the selected sensor and an output from the first temperature sensor,T3, or a correlate thereof, and based on a set of one or more thresholds corresponding to different flow parameters or conditions.

Whichever sensor is lower in temperature will be the one which is not influenced by convection, regardless of whether it is upstream or downstream. For example, if the temperature of the first temperature sensor, T3, is close to the lowest of the first and second temperature sensors (e.g. a difference is less than a defined threshold) then there is non-zero fluid flow. This decision uses a threshold. If the temperature at the first sensor, T3, is substantially higher than the lower of first and second temperature sensors (e.g. a difference is greater than a defined threshold), then there is no flow. This decision uses a further threshold. If the temperature at the first temperature sensor, T3, drops suddenly relative to the lower of the first and second temperature sensors (e.g. a difference signal exhibits a decline of a threshold gradient), this may indicate that flow has restarted. This may use a threshold rate of change.

In some embodiments, the device may include means for directly or indirectly sensing a temperature of the heating element itself, and wherein the controller is adapted to adjust/regulate a heating power of the heating element in part in dependence upon the temperature of the heating element, for example for limiting the temperature of the heating element to stay below a threshold. This can help avoid overheating.

In some embodiments, the detected flow parameter or condition may include detection of presence of air in the tubing. The controller may in some cases be adapted to detect presence of air between the heating element and the temperature sensor based on detecting:
a rise in the first temperature sensor signal, or a correlate thereof, of a threshold gradient, or
a rise in the first temperature sensor signal, or a correlate thereof, which exceeds a threshold temperature.

Additionally or alternatively, the device may include means for directly or indirectly sensing a temperature of the heating element, and wherein the controller is adapted to detect presence of air between the heating element and the temperature sensor based on changes in the temperature of the heating element, or a function or correlate thereof. For example, this temperature may be expected to rise when air is in the tubing at the location of the heater. This may be implemented using a self-heating thermistor in some embodiments.

In some embodiments, the device may include a valve, for example a pinch valve, and wherein the device is arranged in use such that the pinch valve is actuatable to occlude flow through the tube.

In some embodiments, the controller may be adapted to actuate the valve to occlude the tubing responsive to detection of air in the tubing.

The controller can, by way of one example, detect air with a similar detection technique as for detecting flow stop, except that when air is present, the gradient incline in temperature may be steeper.. An arrangement in which the heater and temperature sensor are radially opposed can detect presence of air faster and with greater sensitivity than more traditional mass flow detection techniques.

It is noted that the valve can be positioned at any location along the tubing, either fluidly upstream or downstream from the heating element and temperature sensor, since occluding the flow at any point along the tubing will likely stop flow along the entire length of the tubing.

In one set of advantageous embodiments, the device may include a manually chargeable energy storage means, for example chargeable by manual application of a force, for example against a biasing element, and wherein the actuation of the valve may be powered by release of energy stored in the manually chargeable energy storage means. For example, this might take the form of a spring-loaded energy store, wherein the energy store can be charged by manual force.

A challenge in seeking to provide automated cut-off of fluid flow responsive to air detection is that a powered valve requires a fairly high current to drive it (albeit for only a short period of time). A small-scale, preferably battery powered, sensor would not typically have such a high current available. Thus, it is the recognition of the inventor that a separate mechanically charged actuation means could be used for the valve, and wherein the controller is adapted simply to actuate release of the actuator upon detection of air. For example, this could comprise a manually depressed plunger which biases a spring element, and wherein the spring element is then held in a charged state by a latch which is releasable electronically by control of the controller.

In one advantageous set of embodiments, the device includes a housing adapted to couple to an outside wall of the tubing; the housing accommodating the heating element and the at least one temperature sensor; and wherein the housing, when coupled to the tubing, is adapted to hold the heating element in contact with, e.g. an outside wall of, the tubing at a first contact area, and hold the temperature sensor in contact with, e.g. the outside wall of, the tubing at a second contact area, the second contact area substantially radially opposite the first contact area across the lumen of the tubing; and wherein the device is configured such that action of coupling the housing to the tubing acts to charge the energy storage means. In other words, the device harvests work done in coupling the housing to the tubing to charge the actuator energy storage means. In some embodiments, the housing is adapted to be coupled to the tubing by closing two hinged portions against one another, trapping the tube in-between, and wherein the closure of the hinge acts to charge the actuator, for example by biasing a resilient element. Various other arrangements are possible.

In some embodiments, the device may further comprise a local power source, for example a battery, for electrically powering the heating element. This allows for providing a standalone device without a need for connection to an external power source. The device may be provided without the power source actually included in the device. For example, the device may simply comprise an electrical connection site adapted to connect with a local power source such as a battery. There may be a means for mechanically retaining the local power source within or attached to the housing during operation, while the power source is connected to said electrical connection site.

In some embodiments, such as where the heater and temperature sensors are embedded *in vivo,* the power source may be external to the body and the power is wirelessly or inductively or capacitively coupled to the heater and temperature sensor in a manner that does not require piercing the skin.

In some embodiments, in at least one power control mode, the controller is adapted to control the heating element to dissipate a substantially constant heating power; and wherein the controlling of the heating element to dissipate a constant heating power comprises driving the heating element with a substantially constant power supply (i.e. constant input power to the heating element over time). As discussed above, in some embodiments, the device may be operable in a set of two or more power modes, e.g. a higher power mode and a lower power mode, dependent upon flow state. In some embodiments, the controller may be adapted to control the heating element to dissipate a substantially constant heating power in only a subset of the two or more modes, e.g. in just the higher power mode.

In some embodiments, the device includes a local power source, and wherein the controller is adapted to convert an electrical output from the local power source into a pulse width modulated (PWM) electrical supply for driving the heating element, and to provide the pulse width modulated electrical supply to the heating element. The controller may be adapted, in at least one control mode, to adjust a duty cycle of the PWM electrical supply in dependence upon the voltage of the power source so as to maintain a (substantially) constant power dissipation in the heating element. Maintaining a constant power dissipation by the heating element means for example maintaining a substantially constant (substantially uniform) power supply to the heating element. As a battery drains, its output voltage decreases. If the heating element is a resistor, where its power dissipation is the voltage squared divided by the resistance value, a decreasing voltage results in a decreasing power. Therefore, in such a circumstance, in order to maintain a constant power level to the heating element, an adjustment to the power output from the power source can be performed so as to keep the power level to the heating element constant. A pulse width modulation scheme is a simple and efficient means of achieving this. As the voltage drops, the controller can increase the duty cycle (the high/ON phase of the duty cycle) so as to compensate for the drop in voltage and thereby compensate for the corresponding drop in power, and thus maintain a substantially constant input power supply to the heating element.

A constant power input to the heating element may be a power level which is lower than the maximum power output possible from the battery when fully charged. **In** this way, the power level is kept lower from the start (by modulating the duty cycle), which provides the scope to maintain this lower power, even as the voltage output from the battery drops. For example, a typical supply power to the heating element may be less than one watt and in some embodiments may be less than 100 milliWatts.

As noted above, the controller is adapted to detect a flow parameter or condition. In some embodiments, the detected flow parameter or condition includes a flow stop/start condition. This may mean a binary detection of whether flow has stopped or started. This may be a flow stop/start event detection (i.e. detection of flow starting or stopping), and/or may be a flow status detection (i.e. continuous detection of whether flow is zero or non-zero). The detection may be based at least in part on sensing variations in an output from the first temperature sensor, or a function or correlate thereof, while the heating element is being controlled. More generally, detection of the flow parameter or condition may be based on sensing variations in a composite signal which is a function of multiple different temperature sensor readings, including that of the first temperature sensor, T3. For example, as discussed above, in some cases, the first temperature sensor output is compensated for ambient temperature changes. **In** some cases, this compensated first temperature sensor signal may be further processed by determining a ratio or difference between it and an upstream or downstream temperature sensor signal (as discussed above).

In some embodiments where the heater and first temperature sensor are implanted into the body it may not be necessary to compensate the measurement of the first temperature sensor if the assumption is made that the milieu is at normal body temperature.

The device may use a thermal mass flow technique to detect flow stop/start.
For example, if the radially opposed temperature sensor, or a function/correlate thereof, measures a rise in temperature above a baseline value or above a threshold value or faster than a certain slope, the controller detects that flow has stopped. If the temperature sensor, or a function/correlate thereof, measures a fall in temperature by more than a certain amount or by more than a certain rate, the controller detects that flow has started.

For example, in some embodiments, the controller may be adapted to detect a flow stop condition of the fluid based on detecting a temperature signal from the first temperature sensor, or a function or correlate thereof, exhibiting a rising trend for a threshold time duration or threshold number of signal sample points; or the first temperature sensor signal, or a correlate thereof, exceeding a threshold; or a rise in the first temperature sensor signal, or a correlate thereof, measured over a specific time duration, exceeding a threshold. The controller may be adapted to detect a flow start condition of the fluid based on detecting a temperature signal from the first temperature sensor, or a correlate thereof, exhibiting a declining trend for a threshold time duration or threshold number of signal sample points; or the first temperature sensor signal, or a correlate thereof, falling below a threshold; or a fall in the first temperature sensor, or a correlate thereof, measured over a specific time duration, exceeding a threshold.

In some embodiments, the controller may be adapted to generate an alert signal after a pre-set non-zero time delay following detecting flow stopping, and to terminate the alert signal immediately responsive to detecting flow starting. The alert signal may be a user perceptible alert signal, e.g. a sensory output such as an audible alarm, one or more visual indicators such as light indicators, or any other sensory output. The alert signal may alternatively be an electronic signal that is communicated to a user or other device to be interpreted in a manner of their choosing.

As will be discussed in more detail to follow, it is a realization of the inventor that, in actual clinical practice for certain applications, it is most useful to generate an alert only after a certain time delay following detection of flow stopping. In particular, it is normal in some endovascular procedures for there to be multiple infusion lines into a patient simultaneously, each at a different vascular access port. During the procedure, the clinician selectively turns certain infusions off and then back on as different tools are repeatedly introduced and then removed. If one of the infusions is turned off for more than e.g. 1 minute there is a serious risk to the patient of blood clot formation and stroke. With the various different tubing lines being turned off and then on, it is easy for the doctor to lose track of which lines are stopped and for how long. If a device is configured to provide a flow alarm that sounds immediately upon flow stoppage, this simply adds to the workflow clutter since flow stoppage in a line is intentional so long as it lasts no longer than 1 minute. What is more valuable clinically is a device that sounds after a time delay of a duration which corresponds to the maximum time delay before there is risk to the patient. The alarm should then switch off immediately once flow restarts. This is more in accordance with clinical preference.

In some embodiments, the device may include means for applying a compression to the tubing wall, wherein the compression reduces a radial distance between the first location and the second location. Radial here means relative to the radial dimension of the tubing. Thus the compression reduces the radial distance between the first location and second location across the lumen of the tubing. For example, the compression squashes or partially flattens (i.e. deforms) the tubing at the axial location of the heater and temperature sensor, so that the heater and temperature sensor are brought (radially) closer together. This shortens the radial thermal path length and fluid volume between the temperature sensor and the heating element, which increases the sensitivity to flow stop/start.

In some examples, in the case where the device includes a housing for holding the heating element and temperature sensor against the wall of the tubing, the means for applying a compression may be adapted such that coupling the housing to the tubing has the effect of causing the compression to be applied. In other examples the section of tubing that holds the heater and first temperature sensor may be manufactured to have a non-circular, flattened cross section upon which the heater is attached to one flattened side and the first temperature sensor is applied to the opposing flattened side.

In particular examples, the means for applying a compression may be provided by the aforementioned optional housing, wherein the housing is structured such that, when coupled to the tubing, the compression is applied by the housing to the tubing wall. In other words, in this example, when coupled to the tubing, a compression is applied by the housing to the tubing wall for reducing a radial distance between said first contact area of the heating element and said second contact area of the temperature sensor. An alternative means for applying a compression might include an actuatable compression application means, e.g. electronically actuatable or manually actuatable. There may be for instance a squeezing mechanism which is actuatable to apply the compression. This might be actuated automatically upon coupling the housing to the tubing in some instances.

It is noted that in this disclosure, 'compression' of the tubing may mean applying a squeezing action or force to the tubing, with opposed application points on either side of the tubing. 'Compression' may also mean a section of tubing that is manufactured with a non-circular cross section such that the heater and the first temperature sensor are closer together than they would be if the cross section where instead circular.

In one particular set of embodiments, in the case where the device includes a housing for holding the heating element and temperature sensor against the wall of the tubing, the housing may comprise first and second parts, e.g. hinged together, and the housing operable to couple to the tubing by moving the housing into a closed position with the tubing trapped between the first and second parts, and the housing structured to accommodate the tubing when so closed, and to hold the heating element and temperature sensor at the first and second contact areas. In some cases, the first part may accommodate the heating element and the second part accommodate the temperature sensor. A variety of other arrangements are also possible.

In some embodiments, the device may be configured such that, when coupled to the tubing, a respective axial section of the tubing wall either side of the first and second contact areas is not in contact with the device, for example exposed to the air or the surrounding milieu.

In some embodiments, the housing may define an internal cavity or space within which an axial section of the tubing is received, and wherein the first contact area and second contact area are the only (or major) areas of contact between the device and the tubing inside the cavity or space. The remainder of the tubing may be exposed to an environment inside the cavity or space.

In some embodiments, the device may be configured such that, when coupled to the tubing, the first and second contact area form the only areas of contact between the device and the tubing (or, for example, at least of the axial section of the tubing which is received inside the housing).

In certain embodiments, such as when the device is embedded in the body and the heater and temperature sensors are surrounded by interstitial fluid and tissue in close thermal contact with the heater and temperature sensors, it may be preferable to provide thermal insulation around the heater and temperature sensors to thermally separate the heater and temperature sensors from the milieu, such that the heat from the heater flows principally into the fluid in the tubing and not into the milieu and the first temperature sensor measures primarily the temperature of the fluid and not the temperature of the surrounding milieu.

It has been described above how using the first temperature sensor radially opposed to the heater can be used to determine if fluid flow has stopped or started. This sensor can be used to measure flow rate at very low flow rates, but it loses sensitivity to flow rate as flow rate increases. In some embodiments, the device may include at least one further temperature sensor arranged to be held at a position axially/longitudinally displaced from the heating element along the length of the tubing (for example for sensing temperature either upstream or downstream of the heating element in operation), and wherein the controller is further operatively coupled with the at least one further temperature sensor, and wherein the controller is adapted to determine a flow rate and/or a flow direction using the first temperature sensor and the at least one further temperature sensor.

If the at least one further temperature sensor is located downstream from the heater during normal forward flow, and flow restarts after being stopped, the reported temperature at the one further temperature sensor will rise as the reported temperature at the first temperature sensor falls, due to the volume of warmed fluid moving downstream away from the heater towards the new sensor. If that one further temperature sensor is kept at that position on the tubing, and flow had been stopped but then starts in the reverse direction, its reported temperature will not rise as the reported temperature at the first temperature sensor falls. In this way adding at least one further temperature sensor can determine the direction of flow. The flow rate can be inferred from the magnitude of the reported temperature change from the one further temperature sensor placed downstream, at or near the time when the first temperature sensor indicates that flow is occurring.

Inclusion of a further one or more temperature sensors can also improve speed and sensitivity of detection of a flow stop/start condition as explained earlier.

In some embodiments, the device is for *ex vivo* use, for example for coupling to an infusion or effusion line outside of the body.

As mentioned briefly above, in some embodiments, the device may be provided as an implantable device for use inside the body of a subject, i.e. for implantation in a subcutaneous region of the body.

Various further consideration may apply in such cases.

In some embodiments, the device may include at least a section of the tubing.

In some embodiments, the device may further include a thermally insulating enclosure, for impeding heat dissipation from the heating element to an outside of the device, and wherein the heater and temperature sensor are housed inside of the thermally insulating enclosure.

In some embodiments, the thermally insulating enclosure is coupled or mounted to an exterior of the tubing. The thermally insulating enclosure may at least partially surround an exterior wall of the tubing, for example encircling the exterior wall of the tubing.

According to one or more embodiments, there may be provided an apparatus, comprising: the device in accordance with any embodiments described in this document or in accordance with any claim of the application; and an external interface unit; wherein the controller comprises an internal controller portion disposed in the implantable device and an external controller portion disposed in the external interface unit, and wherein the heater and temperature sensor are each electrically connected to the internal controller portion, and wherein the internal controller portion comprises first inductive power coils for inductively receiving power from second inductive power coils comprised by the external controller portion.

The internal controller portion may be adapted to communicate wirelessly with the external controller portion to send temperature information from the temperature sensor or sensors.

This may be done either with a separate wireless communication link, e.g. near field communication (NFC), Bluetooth, or any other suitable wireless communication protocol, including for example a proprietary protocol, or can be done by modulation of the inductive power supply signal.

In some embodiments, the device is a standalone device. In other embodiments the device may be one element in a larger system.

A further aspect of the invention provides a system comprising a medical tubing line and a device in accordance with any of the embodiments or examples in this disclosure, the device coupled to the infusion or effusion tubing line.

A further aspect of the invention provides a kit of parts comprising a medical tubing line and a device in accordance with any of the embodiments or examples in this disclosure.

A further aspect of the invention provides a method for sensing fluid flow through a tube, for example through a tubing line, e.g. a medical tubing line. The method comprises holding a heating element in thermal communication with a lumen of the tubing at a first location and simultaneously holding a temperature sensor in thermal communication with a lumen of the tubing at a second location, preferably wherein the second location is substantially radially opposite the first location across the lumen of the tubing. The method further comprises controlling a heating power dissipation of the heating element. The method further comprises sensing a temperature output from the temperature sensor, for example while the heating element is active. The method further comprises detecting a flow parameter or condition of fluid in the tubing based on at least an output from the temperature sensor.

For example, the method may comprise holding a heating element in thermal contact with the fluid at a first location and simultaneously holding a temperature sensor in thermal contact with the fluid at a second location, wherein the second location is substantially radially opposite the first location across the lumen of the tubing.

In some embodiments, the method comprises adjusting a heating power dissipation of the heating element based upon changes in the detected flow parameter or condition.

The method may be a method for *ex-vivo* sensing of fluid flow.

The method may be a method for *in-vivo* sensing of fluid flow.

In some embodiments, the method may further comprise compressing or squeezing the tubing or manufacturing the tubing such that, at the location of the heater and temperature sensor, the radial distance between the first location and second location is reduced. The compression or squeezing or manufacturing is to deform the tubing away from a circular cross sectional shape to thereby reduce the thermal path length through the tubing at the location of the compression or deformation for the duration that the deformation exists.

In some embodiments, the force applied to compress the tubing may simultaneously act to hold the temperature sensor and heater in contact with the tubing at first and second contact areas.

In some embodiments the heater and temperature sensors and the section of fluid in the tubing between them are located within a thermally insulated housing to minimize heat flow between the device and the surrounding milieu.

A further aspect of the invention is an implantable device for implantation in the body of subject for sensing a fluid flow through a tubing within the body. The device comprises a heating element and at least one temperature sensor. The device further comprises a controller or control arrangement operatively coupled to the heating element and temperature sensor. The device is arranged in use to hold the heating element positioned in thermal communication with the fluid in the tubing at a first location, and the temperature sensor positioned in thermal communication with the fluid in the tubing at a second location.

The first location may be (substantially) longitudinally aligned with the second location along the lumen of the tubing. The first location may be circumferentially offset from the second relative to the lumen of the tubing.

The second location may be substantially radially opposite/aligned with the first location across the lumen of the tubing. The controller may be adapted to, simultaneously: control the heating element to dissipate heating power; and detect a flow parameter or condition based on an output from the temperature sensor.

Any of the features or embodiments described in this document in relation to any of the other aspects of the invention may be combined or integrated with or applied to the present aspect of the invention.

In some embodiments, the device includes at least a section of the tubing.

In some embodiments, the device may further include a thermally insulating enclosure, for impeding heat dissipation from the heating element to an outside portion of the device, and wherein the heater and temperature sensor are housed inside of the thermally insulating enclosure.

In some embodiments, the thermally insulating enclosure is coupled or mounted to an exterior of the tubing, and optionally wherein the thermally insulating enclosure at least partially surrounds an exterior wall of the tubing, for example encircling the exterior wall of the tubing.

In some embodiments, there may be provided an apparatus, comprising: the implantable device of any preceding claim; and an external interface unit. The previously mentioned controller may comprise an internal controller portion disposed in the implantable device and an external controller portion disposed in the external interface unit. The heater and temperature sensor may each be electrically connected to the internal controller portion, and wherein the internal controller portion comprises first inductive power coils for inductively receiving power from second inductive power coils comprised by the external controller.

The internal controller portion may further be adapted to communicate wirelessly with the external controller portion to send temperature information from the temperature sensor.

Another aspect of the invention is a device for sensing a fluid flow through a tubing (e.g. a medical tubing), comprising: a heating element and at least one temperature sensor; and a controller operatively coupled to the heating element and temperature sensor. The device is arranged in use to hold the heating element positioned in thermal communication with the fluid in the tubing at a first location, and the temperature sensor positioned in thermal communication with the fluid in the tubing at a second location. The first location may be (substantially) longitudinally aligned with the second location along the lumen of the tubing. The first location may be circumferentially offset from the second relative to the lumen of the tubing. The second location is preferably substantially radially opposite/aligned with the first location across the lumen of the tubing. The device includes at least a section of the tubing, and wherein the heating element and the at least one temperature sensor are each coupled to an outside wall of the at least section of tubing, at least partially integrated/embedded inside a wall of the at least section of tubing, or mounted inside the lumen of the at least section of tubing. The controller may be adapted to simultaneously: control the heating element to dissipate heating power; and detect a flow parameter or condition based on at least an output from the temperature sensor.

Another aspect of the invention is a device for sensing a fluid flow through a tubing (e.g. a medical tubing), comprising: a heating element and at least one temperature sensor; and a controller operatively coupled to the heating element and temperature sensor. The device is arranged in use to hold the heating element positioned in thermal communication with the fluid in the tubing at a first location, and the temperature sensor positioned in thermal communication with the fluid in the tubing at a second location. The first location may be (substantially) longitudinally aligned with the second location along the lumen of the tubing. The first location may be circumferentially offset from the second relative to the lumen of the tubing. The second location is preferably substantially radially opposite/aligned with the first location across the lumen of the tubing. The controller may be adapted to simultaneously: control the heating element to dissipate heating power; and detect a flow parameter or condition based on an output from the temperature sensor, and wherein the flow parameter or condition is a presence of air in the tubing

The device may further comprise a valve, for example a pinch valve, and wherein the device is arranged in use such that the pinch valve is actuatable to occlude flow through the tube, and wherein the controller is adapted to actuate the valve to occlude the tubing responsive to detection of air in the tubing. The device may further include a manually chargeable energy storage means, for example chargeable by manual application of a force, for example against a biasing element, and wherein the actuation of the valve may be powered by release of energy stored in the manually chargeable energy storage means.

For example this might take the form of a spring-loaded energy store, wherein the energy store can be charged by manual force. For example, this could comprise a manually depressed plunger which biases a spring element, and wherein the spring element is then held in a charged state by a latch which is releasable electronically by control of the controller.

In one advantageous set of embodiments, the device includes comprises a housing adapted to couple to an outside wall of the tubing; the housing accommodating the heating element and the at least one temperature sensor; and wherein the housing, when coupled to the tubing, is adapted to hold the heating element in contact with, e.g. an outside wall of, the tubing at a first contact area, and hold the temperature sensor in contact with, e.g. the outside wall of, the tubing at a second contact area, the second contact area substantially radially opposite the first contact area across the lumen of the tubing; and wherein the device is configured such that action of coupling the housing the tubing acts to charge the energy storage means. In other words, the device harvests work done in coupling the housing to the tubing to charge the actuator energy storage means. In some embodiments, the housing is adapted to be coupled to the tubing by closing two hinged portions against one another, trapping the tube in-between, and wherein the closure of the hinge acts to charge the actuator, for example by biasing a resilient element. Various other arrangements are possible.

Embodiments discussed above have been described with reference to a sensing configuration comprising a heating element and temperature sensing element arranged substantially radially opposed to one another. However, various of the options and features discussed above can also be advantageously applied to a variant configuration in which a flow condition or parameter is detected based on sensing a temperature of the heating element itself.

Thus, another aspect of the invention is a flow sensing device for sensing a fluid flow through a tubing, comprising a heating unit, the heating unit comprising a heating element and means for directly or indirectly sensing variation in a temperature of the heating element, wherein the device is arranged in use to hold the heating element in thermal communication with a lumen of the tubing, for transferring heat to fluid flowing in the tubing. The device further comprises a controller operatively coupled to the heating element and sensing means, and wherein the controller is adapted to detect a flow condition or parameter (e.g. stop/start condition, or presence of air in the tubing at the location of the heating element), based on: controlling the heating element to dissipate a heating power, and simultaneously and using the sensing means to sense variations in the temperature of the heating element while the heating element is being controlled.

The heating unit may be a single unitary component.

In some embodiments, the heating unit comprises a self-sensing heating element, the sensing of the temperature of the heating element being achieved by sampling an electrical characteristic of the heating element. For example by sampling a voltage across the heating element, to detect a resistance of the heating element, the resistance being indicative of temperature. In some embodiments, the self-sensing heating element is a thermistor, and wherein heating is achieved by application of an electrical supply to the thermistor, and sensing is achieved by sampling an electrical characteristic of the thermistor while said electrical supply is being applied. For example, the thermistor is driven with a constant current, and a voltage across the thermistor is simultaneously sensed, whereby the voltage gives an indication of the resistance, and whereby the resistance gives an indication of temperature. An alternative approach is to alternately switch between heating and sensing in a duty cycle operation, e.g. at a rapid rate.

Any of the features or embodiments described in this document in relation to any of the other aspects of the invention may be combined or integrated with or applied to the present aspect of the invention.

As mentioned above, one realization of the inventor is that detection of flow parameters or conditions may be improved by employing use of two additional temperature sensors, disposed axially displaced from the heating element on either axial side of the heater. Thus, this represents a further aspect of the invention. In particular, one aspect of the invention is a device for sensing a fluid flow through a medical tubing line, comprising: a heating element and at least a first temperature sensor, and a controller operatively coupled to the heating element and the at least first temperature sensor, wherein the device is arranged in use to hold the heating element positioned in thermal communication with the fluid in the tubing at a first location, and the temperature sensor positioned in thermal communication with the fluid in the tubing at a second location. Preferably, the second location is substantially radially opposite the first location across the lumen of the tubing. The device comprises at least a first further temperature sensor and a second further temperature sensor. The device is arranged in use such that the first further temperature sensor is positioned at a location longitudinally offset from the heating element along the lumen of the tubing in a first direction, and the device is arranged in use such that the second further temperature sensor is positioned longitudinally offset from the heating element along the lumen of the tubing in a second direction, opposite to the first direction.

The controller may be adapted to simultaneously: control the heating element to dissipate heating power; and detect a flow parameter or condition based on at least an output from the first temperature sensor, and at least one of the first and second further temperature sensors.

This aspect of the invention may be combined advantageously with the additional features of any of the dependent claims of the other aspects of the invention, and particularly claims 10-20 (optionally excluding the features of claim 1).

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a layout of a heater and temperature sensor in a prior art device;
Fig. 2 shows a layout of a heater element and temperature sensor according to one or more embodiments of the invention, wherein the heater element is at one position on the tubing and a temperature sensor is directly across the tubing from the heater;
Fig. 3 shows a layout of heating element and temperature sensors according to a further one or more embodiments;
Fig. 4 shows a layout of heating element and temperature sensors according to a further one or more embodiments, wherein three temperature sensors are included touching the IV tubing, one downstream and two upstream from the heating element and one ambient temperature sensor;
Fig. 5 shows an example flow sensing device in operation, coupled to an IV line tubing;
Fig. 6 shows a housing of an example device according to one or more embodiments;
Fig. 7a illustrates the dimensions of the tubing and illustrates a tolerance range for positioning of the radially opposed temperature sensor;
Fig. 7b shows a layout of heating element and temperature sensor according to one or more embodiments, and further shows paths of heat flow from the heating element to the temperature sensor;
Fig. 8 shows, as a comparison with Fig. 7b, paths of heat flow longitudinally from the heater through the fluid to a hypothetical temperature sensor downstream from the heater;
Fig. 9a shows example electrical connections used in a prototype for sensing flow based on temperature readings;
Fig. 9b shows an electrical topology for compensating for ambient temperature variation, using an ambient temperature sensor that is not touching the tubing;
Fig. 10 shows the output of a thermistor resistive divider circuit in a prototype device when liquid fluid is flowing for the first 60 seconds, then it stops for 120 seconds, then it restarts flowing at 180 seconds;
Fig. 11 shows a simple electronics topology that can be implemented at low cost;
Fig. 12 illustrates the difference between using a constant power heater element (in accordance with embodiments of the present invention) and a constant temperature resistive heater element;
Fig. 13 shows an electronic circuit topology in which a power transistor is used as a heating element, and the power dissipated in the transistor remains approximately constant over the range of expected battery voltage;
Fig. 14 shows a simulation output of a transistor heater element controlled for a near constant (0.25 Watt) power dissipation over the range of expected battery voltage;
Fig. 15 illustrates steps of an example firmware algorithm to produce an approximately constant power in a resistive heater element as battery voltage changes, using a resistance-capacitance (RC) network to control pulse-width modulation (PWM) timing;
Fig. 16a shows a simulation output of the average power in a resistive heater element over a range of battery voltage using the firmware algorithm of Fig. 15;
Fig. 16b shows a simulation output of the power in a resistive heater element, expressed as a percentage of nominal, over a range of battery voltage using the firmware algorithm of Fig. 15;
Fig. 17 is an example algorithm showing an asymmetrical delay introduced when starting an alert following detecting flow stoppage and no delay when clearing an alert following detecting flow restarting;
Fig. 18 shows an example device comprising means to apply an artificial flattening of the tubing through compression to decrease the radial thermal path length between heater and temperature sensor;
Fig. 19 is a cross sectional view of an example device adapted to receive and position different diameter sizes of tubing between a heater and temperature sensor, such that they have a same thermal path length radially;
Fig. 20 is a cross sectional side view of a prototype device;
Fig. 21 is a cross sectional end view of the prototype device of Fig. 20;
Fig. 22 is a schematic diagram of an example fluid path in operation;
Fig. 23 shows an exterior view of an example device according to one or more embodiments;
Fig. 24 shows a schematic representation of a device that measures fluid flow while embedded in the body;
Fig. 25 shows an exemplary illustration of possible positions for temperature sensors;
Fig. 26 illustrates temperature response at the location of the heater and upstream and downstream of the heater following a transition from a flow start state to a flow stop state;
Fig. 27 illustrates temperature response at the location of the heater and upstream and downstream of the heater following a transition from a flow stop state to a flow start state;
Fig. 28 illustrates a differing temperature response upstream and downstream of the heater responsive to flow stop when the upstream sensor is gravitationally raised relative to the downstream sensor; and
Fig. 29 shows an example temperature readout from a sensing means arranged to sense a temperature of the heating element itself.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

It is to be understood that when used in this specification, the terms intra-venous tubing, IV tubing, intra-arterial tubing, infusion tubing, effusion tubing, line or tubing all refer to the same thing, and can be any tubing that is intended to transport material into the body or out of the body or within the body or not related to a body at all. The term body refers to a human or animal body corpus.

Embodiments of the invention provide a device and method of action for a device that abuts the outside of a tubing , e.g. an infusion or effusion tubing, or is integrated in a wall of the tubing, or is disposed within the lumen of a tubing, and uses thermal mass-flow techniques to determine a flow state or condition, for example if fluid is flowing or stopped within the tubing. The device includes a heater and temperature sensor arranged, when the device is in use, in thermal communication with the tubing lumen and positioned at opposite sides of the tubing wall, in facing relationship. They are for example approximately diametrically opposed or aligned.

Optionally, in some embodiments, the controller is further adapted, in at least one phase of operation, to adjust a heating power dissipation of the heating element based upon changes in a detected flow parameter or condition, for example switching the heating element to a lower power dissipation state following detection of cessation of flow.

Optionally, in some embodiments, if fluid flow is detected to have stopped, the device will alert the operator after a specifically defined delay time, and if fluid subsequently restarts flowing, the alert is terminated promptly, e.g. approximately instantly.

Optionally, in some embodiments, there is applied an intentional distortion of the flexible tubing to reduce the diametric distance between the heater and the temperature sensor, to optimize the function.

Techniques are further described in accordance with some embodiments to address issues arising from battery operation, from changes in ambient temperature and from the presence of interfering fluids and drips.

By way of introduction, it is a motivation behind at least some embodiments of the present invention to improve upon known methods of monitoring fluid flow in infusion tubing (some of which have been discussed above) by providing a relatively small, very low-cost device that can easily clip onto IV tubing to monitor fluid flow, and that does not actually touch the infusate directly, and thus does not need to be sterile. The same principles may be applied to flow sensors for other applications, including implantable flow sensors.

In preferred examples, this device may give an alert if fluid flow stops for longer than a predetermined length of time. The alert could either be visible, audible, a silent mechanical indicator, by wired or wireless electronic communication or some combination of these. The alert would clear immediately upon resumption of fluid flow.

To be most universally useful, in some embodiments, the device is configured such that it is operable with different types (e.g. shapes) and diameters of infusion or effusion or medical tubing. For example, this could include suitability for use with high-pressure tubing used for arterial infusions, which are less than optically clear due to a reinforcing braid in the tubing wall. Typically, infusion tubing outer diameters range from 3.6 mm to 5.7 mm. Thus, by way of example, some embodiments provide a device which is operable to fit to tubing of any diameter between about 3 mm and about 6 mm.

It would also be of advantage for a device to be operable with a variety of fluids, including opaque liquids such as blood or suspensions. It would also be of advantage for a device to be operable in any position or orientation with respect to gravity, even in Space with no gravity, be insensitive to vibration during transport, and be battery powered to operate for several hours while in the field.

The above represent a range of advantageous features and effects, which are not essential to the inventive concept, and some, all or none of which may be features of embodiments of the present invention.

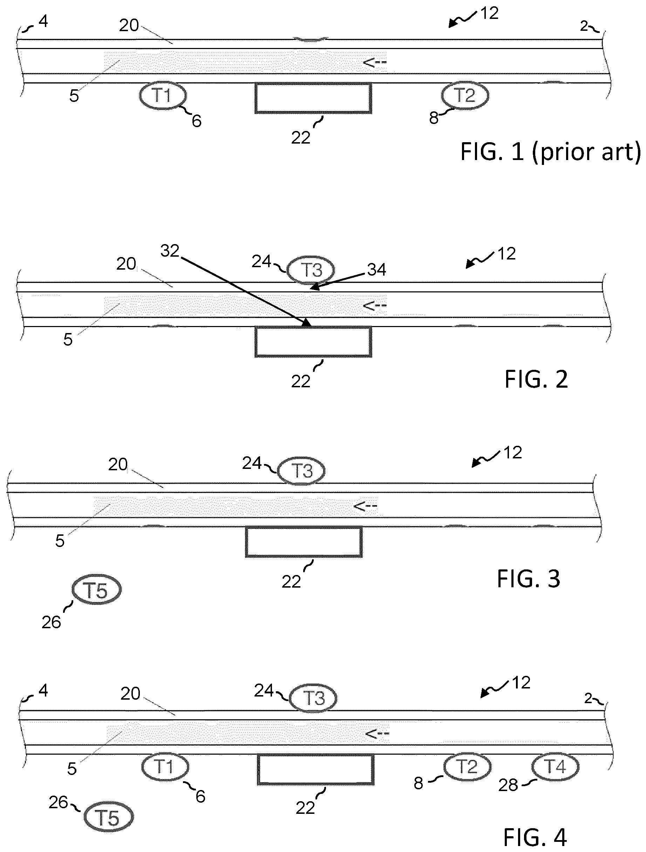

By way of explanatory background, Fig. 1 shows an example arrangement of a heater 22, and first 6 (T1) and second 8 (T2) temperature sensor of a known flow sensing device. The heater and temperature sensors are all arranged in contact with an outer tubular wall 20 of an infusion or effusion line tubing 12. Fig. 1 shows an inlet 2 and outlet 4 to the section of tubing which is illustrated. A fluid flows through the lumen 5 of the tubing along the direction indicated by the arrow. Temperature readings from the upstream and downstream sensors can be compared so as to derive a fluid flow direction and rate. An example device which uses this configuration is described in DE 3827444 A1 for example.

Fig. 2 schematically illustrates an example layout of components in accordance with one or more embodiments of the present invention. The device comprises at least a first temperature sensor 24 (T3) held against the tubing wall 20 at a contact area 34 substantially radially opposite the contact area 32 of the heater to the tubing wall. The heater and temperatures sensor might alternatively be integrated or at least partially embedded in the wall, or positioned inside the lumen. The temperature sensor is labelled T3 to contrast it with the prior art arrangement comprising sensors T1 and T2, which are absent in the particular arrangement shown in Fig. 2. As will be explained later, this arrangement permits detection of flow start and stop with significantly reduced latency compared to the arrangement of Fig. 1 due to the shorter thermal path between the heater and temperature sensor, and with a significantly larger signal level.

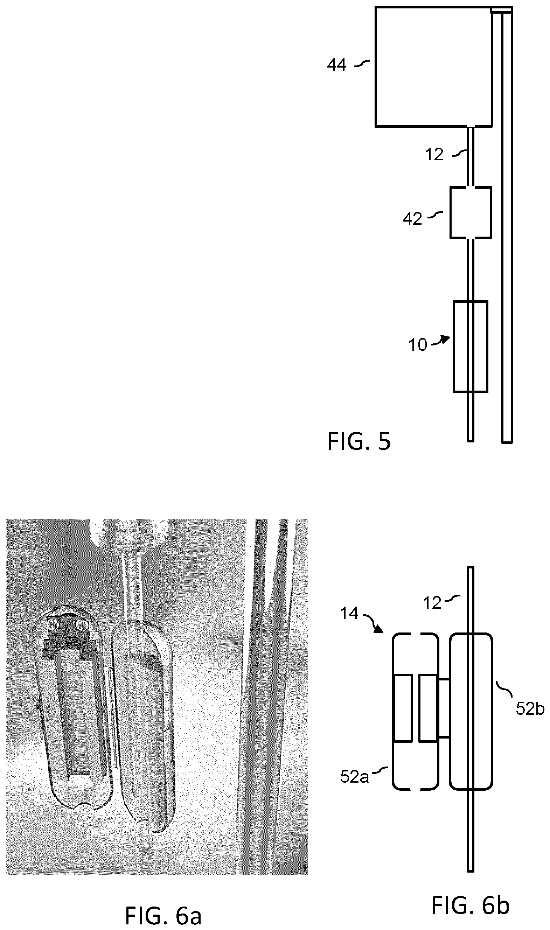

In more detail, in accordance with one or more embodiments, and with reference also to Fig. 5 and Fig. 6, there is provided a device 10 for sensing a fluid flow through a tubing 12, e.g. a medical tubing, e.g. an infusion or effusion tubing line.

The device comprises a heating element 22 and at least a first temperature sensor 24, T3. The device further comprises a controller (not shown) operatively coupled to the heating element 22 and temperature sensor 24, T3.

The device is arranged in use to hold the heating element positioned in thermal communication with the fluid in the tubing at a first location 32, and the first temperature sensor T3 positioned in thermal communication with the fluid in the tubing at a second location 34. The first location 32 may be (substantially) longitudinally aligned with the second location along the lumen of the tubing. The first location may be circumferentially offset from the second location 34 relative to the lumen of the tubing.

In preferred embodiments, the second location 34 is substantially radially opposite the first location 32 across the lumen 5 of the tubing.

In operation, the controller is adapted to: control the heating element 22 to dissipate heating power; and detect a flow parameter or condition of the fluid flow based on at least an output from the temperature sensor 24, T3, or a function or correlate thereof.

In one set of embodiments, the device comprises a housing 14 (see for example Fig. 6) adapted to couple to an outside wall 20 of the tubing. The device may be adapted to removably couple to the tubing. The housing accommodates the heating element 22 and the at least one temperature sensor 24, T3. The housing 14, when coupled to the tubing, is adapted to hold the heating element 22 in contact with an outside wall 20 of the tubing 12 at a first contact area 32, and hold the temperature sensor 24, T3 in contact with the outside wall of the tubing at a second contact area 34. The second contact area 34 is preferably substantially radially/diametrically opposite the first contact area 32 across the lumen 5 of the tubing.

However, other options are possible. For example, the heating element and the at least one temperature sensor may each coupled/attached to an outside wall of the section of tubing, at least partially integrated/embedded inside a wall of the section of tubing, or are mounted inside the lumen of the section of tubing.

The temperature sensors can be standard sensors familiar to someone skilled in the art, including but not limited to thermistors, silicon sensors and resistance-temperature detectors (RTDs).

The device will operate in total darkness, will operate in any physical orientation with respect to gravity, may be small, very low cost, lightweight and may be battery powered so it is self-contained. It may work on a variety of fluids including saline, water, electrolyte solutions, blood, blood products, blood expanders, cerebrospinal fluid, urine and liquid medications, either in solution or in suspension.

As will be described in more detail later, and as schematically illustrated in Fig. 3, Fig. 4 and Fig. 25, in some embodiments, one or more further temperature sensors may be provided in addition to the first temperature sensor T3.

For example, in some embodiments, further temperature sensor 26 (labelled T5 in Fig. 3) may be provided which is arranged for sensing an ambient temperature. Additionally, or alternatively, in some embodiments, a further temperature sensor may be provided (e.g., T2 or T4 in Fig. 4) for sensing a temperature of the fluid upstream from the heater.

In more detail, in accordance with some embodiments, the device further includes a second temperature sensor 26, 28 (e.g., T5 or T4) and wherein the device is arranged in use such that the second temperature sensor is positioned for sensing either: an ambient temperature of the air in the environment of the device, or a temperature of the fluid at a location upstream from the heating element, preferably at a location substantially uninfluenced by the thermal output of the heating element. In these embodiments, the controller may be adapted to detect the flow condition or parameter based on outputs of both the first temperature sensor 24, T3 and the second temperature sensor based on compensating the temperature change measurement of the first temperature 24, T3 sensor using temperature change measurements of the second temperature sensor T5, T4. This is described in more detail later.

As schematically illustrated in Fig. 4, in accordance with some embodiments, there may be provided at least one further temperature sensor arranged for sensing a temperature of the fluid at a location upstream and/or downstream of the heating element 22. For example, as illustrated in Fig. 4, a downstream temperature sensor 6, T1 similar to that shown in Fig. 1 could be provided. Additionally or alternatively, an upstream temperature sensor 8, T2 similar to that shown in Fig. 1 could be provided. Additionally or alternatively, a further temperature sensor 28, T4 could be provided for sensing a temperature of the fluid close to an inlet 2 from the infusion or effusion fluid source, for use in calibrating or compensating temperature measurements. This will be described in greater detail to follow. The device may be arranged such that at least one further temperature sensor 6, 8, 28 is arranged in use at a position either upstream or downstream of the heating element, and wherein the controller is further operatively coupled with the at least one further temperature sensor, and wherein the controller is adapted to determine a flow parameter or condition based on a function of respective outputs of the first temperature sensor (T3) and the at least one further temperature sensor. As will be explained below, the flow parameter or condition may include a flow stop/start condition. As will be explained below, here, a ratio or difference between the first temperature sensor signal T3 and one of the upstream or downstream temperature sensors might be used. The flow parameter or condition may additionally include a flow rate and/or direction.

Fig. 5 illustrates an example embodiment of the device 10 in situ, coupled to an infusion line tubing 12. An IV bag 44, containing liquid, is shown hanging from a pole. The IV tubing 12 drains the bag into a drip chamber 42 which is shown below the bag. The drip chamber allows visual determination of the amount of flow in the tubing by counting the rate at which drops of known volume are released into the chamber. The device 10 is shown removably coupled, e.g. clipped, to the tubing at a position downstream from the drip chamber, e.g. directly below the drip chamber. As an alternative arrangement, the device 10 may be integrated with the drip chamber itself, thereby providing a 'smart drip chamber'.

The device 10 can be provided as a standalone unit. The device may in some embodiments touch the outside of tubing, such as infusion tubing, and will monitor the presence of flow of a liquid fluid within that tubing, for example detecting when liquid fluid flow is stopped for a period of time, or when liquid fluid flow has been restarted.

Fig. 6 further illustrates a design of an example housing in accordance with a particular embodiment. Fig. 6 shows the device opened up prior to being clipped onto the outside of the IV tubing. Fig. 6a shows a 3D CAD rendering of the example housing. Fig. 6b shows a cross-section through the housing.

In the illustrated example, the housing 14 comprises first 52a and second 52b parts hinged together. As illustrated, the housing is adapted to accommodate the tubing running through it. By way of one possible example, the first part 52a may accommodate the heating element 22 and the second part 52b may accommodate the temperature sensor 24, T3, and the housing may be operable to couple to the tubing by hinging the housing into a closed position with the tubing 12 trapped between the first 52a and second 52b parts, and the housing structured to accommodate the tubing when so closed, and to hold the heating element and temperature sensor at the first and second contact areas. In other examples, both the heating element 22 and temperature sensor 24, T3 could be accommodated in the same half of the housing but held in radially opposed positions across the tubing. This arrangement permits easily clipping onto IV tubing as shown in Fig. 6 and does not touch the fluid inside the tubing, thus does not need to be sterile. This device can be easily unclipped and removed from the IV tubing.

The principles behind the detection of flow using one or more embodiments of the present invention will now be briefly outlined.

The flow sensing device 10 according to embodiments of the invention can be used to detect whether fluid is flowing inside the tubing 12. By way of example, detection of flow start and flow stop (a flow start/stop condition) may be made. This may be useful for example in a device whose principal purpose is to sound an alarm if liquid fluid flow stops, for example for an excessive period of time, as described above. This sensing principle however is not restricted to such use. The sensing method could even be embedded as an OEM (original equipment manufacturer) component in another finished device, to monitor for flow of fluid in that device.

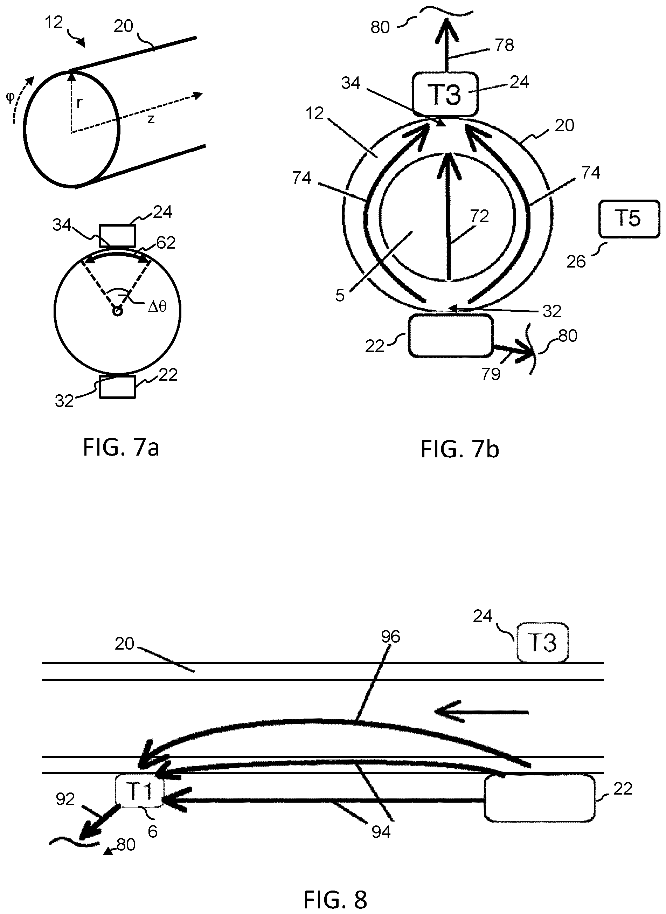

With reference to Fig. 7, the method of flow detection comprises using the surface heater element 22 to heat a small section (a first contact area 32) on one sidewall 20 of the infusion tubing 12 and to measure the temperature at a location 34 on the opposing sidewall of the tubing using the temperature sensor 24, T3. Using this method, at minimum, only one temperature sensor is required, labeled T3 in Fig. 7, and it is located substantially across the diameter of the tubing from the heater 22, for example directly radially across the tubing on the tubing wall opposite where the heater is located. Instead of having the heating element and temperature sensor T3 applied to an outside surface of the tubing, these can be at least partially embedded or integrated in a wall of a tubing, or located inside a lumen 5 of the tubing.

It is to be noted that when the words "opposite side" or "opposed" or "across" are used it is not required that the temperature sensor 24, T3 be located exactly at the point that is the farthest around the tubing from the heater; the temperature sensor should be substantially around the tubing from the heater but there is some latitude on how far around it must be.

For example, Fig. 7 (bottom left) schematically illustrates an example tolerance range for the location of the temperature sensor 24, T3 relative to the heater 22. Substantially radially opposite may mean for example radially opposite plus or minus some tolerance, for example, +/- 5-10 degrees, i.e. the second contact area 34 is positioned within an arcuate section 62 of the tubing wall, the arcuate section 62 having its center radially opposite the first contact area 32, and subtended by an angle Δθ at the center of the tubing (the tubing axial axis) of 5-10 degrees. Another example tolerance range for the positioning of the second contact area 34 might be defined by a circumferential section 62 of the tubing circumference, having its center radially opposite the heating element, and the length of the circumferential section being a defined percentage of the total circumference, for example less than 25%, preferably less than 20%, more preferably less than 10%, for example less than 5%.

Furthermore, it is noted that the heating element may have a heat output area from which heat is dissipated, and the first temperature sensor T3 may have a sensing area which is sensitive to temperature. In some embodiments, the heat output area of the heating element may be sized such that, when the device is in use, the heat output area spans no more than for example 20% of a circumference of the tubing, preferably no more than 10%. In some embodiments, the sensing area of the temperature sensor T3 may be sized such that, when the device is in use, the sensing area spans no more than for example 20% of a circumference of the tubing, preferably no more than 10%.

As illustrated in Fig. 7 (top left), the tubing line 12 may be understood as having an axial dimension, z, along a direction of fluid flow through the tubing, and a radial, r, and circumferential, φ, dimension orthogonal to the axial dimension. The radial, r, and circumferential, φ, dimension define a radial plane perpendicular to the axial (longitudinal) axis, z, of the tubing.

The first contact area 32 and second contact area 34 may be substantially axially aligned. In other words, both contact areas are aligned at a same axial location along the length of the tubing 12. In other words, there is substantially no axial displacement between the position of the first contact area and the position of the second contact area.

The thermal resistance between the heating element 22 and temperature sensor T3 through the radial fluid path 72 when the fluid is not flowing should ideally be lower than the thermal resistance along the circumferential thermal path 74 through the tubing wall from the heater to sensor, and ideally is also lower than the thermal resistance along the path 78 from T3 to the ambient 80 . This has the effect that a change in the thermal resistance through the radial fluid thermal path 72 has the greatest influence on sensor T3.

Based on a measured relationship between the temperature sensor 24, T3 signal and time, it is possible to determine whether fluid is flowing or has stopped.

For example, if the temperature at T3 starts to rise, then fluid flow in the tubing may be considered to be stopped, since the heater 22 is raising the temperature of the stagnant fluid. If the temperature at T3 starts to fall, then fluid flow in the tubing may be considered to have restarted since fresh cooler fluid is interposed between the heater 22 and temperature sensor 24, T3.

A novel feature of embodiments of the invention is that by positioning temperature sensor T3 substantially across the tubing from the heater 22, the signal T3 can be used to clear a flow alarm quickly when flow resumes because the thermal path 74 is immediately and profoundly interrupted even with a small amount of flow. Any liquid heated by the heater is swept downstream before its heat can reach sensor T3, so T3 promptly returns to the temperature of the incoming liquid. This (radially opposed) location of T3 results in the largest change in temperature as a function of flow of any possible location for a temperature sensor, resulting in a large temperature signal, which in turn simplifies the connected electronic circuitry (to be discussed later).

Referring again to Fig. 7, as mentioned, this schematically shows paths of heat flow from the heating element 22 to the temperature sensor T3, through the walls of the tubing (path 74), through the fluid inside the tubing (path 72), from the temperature sensor T3 to ambient (path 78) and from the heater 22 to ambient 80 (path 79). Fig. 7 also shows an optional ambient temperature sensor 26, T5 (mentioned above) which does not touch the tubing.

As mentioned, if the infusion tubing 12 is heated at one point 32 on one side of the wall 20 of the tubing, and if a temperature sensor 24, T3 is placed radially across (e.g. directly radially across) the tubing from the heater 22, in contact with the wall 20 on the opposite side of the tubing 12, then it is possible to determine whether fluid is flowing in the tubing or whether fluid is not flowing based on the signal output from the temperature sensor as a function of time.

Prior art devices do not use this sensor location. This may be because prior art devices have a primary focus on quantifying the flow rate, and this sensor location is less sensitive for measuring rate of flow. However, for a binary flow/no-flow detection, the inventor has found that this is the best position for the sensor.

It is to be noted that the same effect cannot be achieved simply by moving a downstream temperature sensor closer to the heater 22. This can be understood for the following reasons.

The temperature at the substantially radially opposite location goes up when flow stops as the heater 22 warms the fluid and that warmth crosses the tubing diameter (along path 72) by thermal conduction. Temperature falls when flow resumes as fresh cooler fluid replaces the warmer fluid interposed between the heater 22 and temperature sensor 24, T3. By contrast, the signal polarity is in the opposite direction for prior art devices that have a temperature sensor downstream: the temperature downstream increases when flow occurs and returns back down towards ambient temperature when flow stops, since the temperature is no longer influenced by warmed fluid. As a result of this, it is not possible to achieve the same effect as embodiments of the present invention by simply moving a downstream sensor closer to the heater (i.e. to thereby reduce the thermal path length between the heater and the sensor). This is because, as the sensor were to move closer to the heater, at some point the signal polarity would become indeterminate, since the signal would show a response with characteristics of both a downstream and an opposed sensor. In particular, fluid that is stagnant will be heated, that heat would spread out sideways from the heater and influence the temperature of the downstream sensor if it is very close to the heater, thus interfering with the temperature change response due to flowing fluid.

Additionally, a downstream temperature sensor which is close to the heater would be more strongly influenced by stray, unintended thermal pathways directly between the heater and sensor through the wall 20 of the tubing. This is illustrated in Fig. 8 which shows paths of heat flow longitudinally from the heater 22 through the fluid (path 96) to a hypothetical sensor T1 downstream, from the heater to sensor T1 by paths other than through the fluid (paths 94) and from temperature sensor T1 to the ambient 80 (path 92). These stray thermal pathways will dominate the temperature sensor 6, T1 response if it is positioned next to the heater. So, in prior art devices, a downstream sensor must be positioned far enough away from the heater to avoid being warmed when fluid flow is stopped.

In the particular case of detecting a binary flow start/stop condition, the radially opposed sensor location provides a much simpler signal response to start/stop of flow than a comparable downstream sensor arrangement. The thermal signal at a downstream temperature sensor depends upon the flow rate. While this makes it useful to quantitate the flow rate in prior art devices, this variation in output with flow rate only adds an additional, unwanted variable uncertainty when seeking specifically to make a binary flow/no-flow detection. If the flow rate is slow, the downstream sensor temperature will rise, but if the flow rate is fast enough the signal downstream will actually decrease back towards ambient, because the heater will not have time to heat a unit of fluid before it sweeps past the heater. This is taught in DE3827444A1. US 2020/061290 A1 further refers to this inversion of the signal at high flow rates as a "turning point". By contrast, the sensor that is radially opposed to the heater quickly cools toward ambient temperature with the start of even a very small flow, and it stays cool as flow increases, thus leading to a large temperature signal response that is not very dependent upon flow rate.

Thermal interference through direct thermal conduction along the tubing wall is reduced for a device which touches the tube along only a limited length of the tubing. In particular, it is in general preferable to minimize extraneous contact with the tubing in order to minimize undesired stray thermal pathways that can interfere with the desired thermal path signals. A device with a temperature sensor axially displaced from the heater must be in contact with the device along a relatively extended section of the tubing. By contrast, a device in accordance with at least a subset of embodiments of this invention need only contact the tubing at a very restricted axial location.

However, as mentioned above, in accordance with some embodiments, one or more further temperature sensors may be provided in addition to the first sensor T3, for sensing one or more of: a temperature of fluid upstream from the heating element 22; a temperature of fluid downstream from the heating element 22; a temperature of an ambient air or environment around the device; a temperature of fluid at an inlet of the device. Any combination of such further temperature sensors may be used. As will become clear, the outputs from such sensors may be used in combination to improve detection or measurement of a target flow parameter or condition. Different combinations of the sensor outputs, in conjunction with an output from the first sensor T3, may be used depending upon circumstances, e.g., depending upon a current flow condition or state, or depending upon an orientation of the device, or depending upon a target flow parameter or condition.

In some embodiments, an additional temperature sensor (e.g. T2 or T4 or T5), is provided which is arranged to sense a temperature of the environment, and/or of the fluid upstream from the heater, for use in calibrating temperature change measurements at the location of the main temperature sensor T3.

The operation of the embodiment of Fig. 2 assumes that the heater 22 power is large enough to make the resulting heater surface temperature always stay well above any ambient temperature around the device, even if ambient temperature varies over a range. In this case, only a single temperature sensor T3 is needed to detect whether fluid is flowing or not, since the variation in signal with ambient temperature is a small proportion of the signal change with flow. However, especially in a battery powered device, there is an incentive to operate the heater at as low a power as possible in order to extend the battery life and avoid excessive heating and damage to the fluid, so the heater temperature may only operate at a few degrees above the ambient temperature. However, the ambient temperature may vary, and its range may be greater than the temperature change induced in a fluid by the low power heater. In this situation, accuracy is improved by compensating the temperature sensor T3 reading for changes in ambient temperature. Using simply a fixed temperature threshold to detect whether fluid is flowing may not work optimally if the ambient temperature variation is larger than the variation due to flow.

A second temperature sensor may be introduced to facilitate this ambient temperature compensation. An example arrangement including an ambient temperature sensor T5 was shown in Fig. 3. The second temperature sensor may be accommodated in the housing, if one is provided.

If the temperature sensors report temperature directly in degrees, such as semiconductor sensors that output 10mV/degree C or semiconductor junctions that change -2mV/degree C, then ambient temperature compensation can be achieved by simply taking the difference between two sensors T3 and T5 to derive a sensor difference signal, and making flow/no-flow detections based upon this difference signal, or a function thereof (e.g. by looking for a change in the difference signal level or slope compared to a threshold).

Alternatively, often less expensive thermistor temperature sensors may be used which may change their resistance as a percent per degree C, rather than a specific resistance per degree. Thus, if thermistors are used, these may be connected in ratio to compensate for ambient temperature. A simple voltage divider with one thermistor in the upper arm and one thermistor in the lower arm will achieve this, as ambient temperature change affects both sensors by the same percentage and thus the output voltage of the divider does not change as ambient temperature changes.

Of course, use of a microprocessor which can convert input sensor signals to equivalent numerical temperature signals is also possible, in which case a simple difference signal between the converted temperature signals of T3 and T5 could be computed and monitored to detect flow condition or parameters.

Additionally or alternatively to the use of an ambient air temperature sensor T5 to compensate the first temperature sensor T3 output, a reading from a temperature sensor upstream or downstream from the first temperature sensor T3 could be used to apply compensation. For example, with reference to Fig. 4, compensation for ambient temperature may be achieved by using temperature sensor at the location of T1 (see Fig. 1 or Fig. 4), i.e. arranged in thermal communication with fluid at a location downstream from the heater, or at the location of T4, i.e. arranged in thermal communication with fluid at a location upstream from the heater. With reference to T1, this will respond thermally to flow rate in the opposite direction from T3: when there is fluid flow then T1 warms up as the warmed fluid flows downstream, at the same time as T3 cools down as new, cooler fluid is interposed between the heater and T3. Because the two sensors operate in opposite directions, one can use them in ratio to create the maximum signal modulation due to flow.

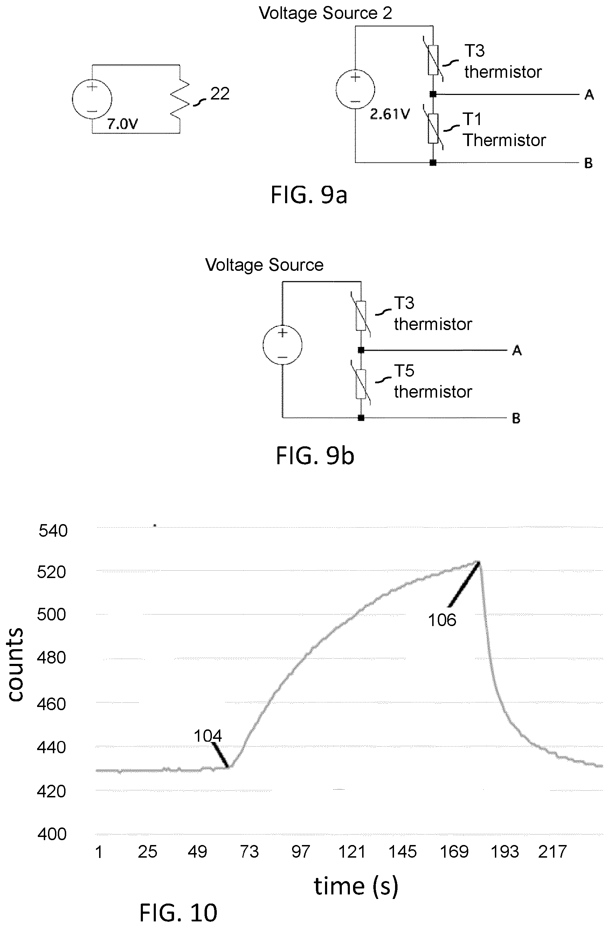

If the sensors are thermistors, where the resistance percentage change is proportional to temperature, then using a resistive divider gives both the largest temperature signal modulation and also automatically compensates for ambient temperature variation, since such variation changes the resistance of each thermistor by the same percentage, resulting in no change in voltage output with ambient temperature variation. An example circuit with a suitable resistive divider arrangement is shown in Fig. 9a (right). This shows an experimental sensor module that was constructed to test the invention, where T3 is negative temperature coefficient (NTC) thermistor radially opposed to the heater and T1 is a NTC thermistor touching the tubing downstream from the heater. Fig. 9a (left) shows the heater as a resistor, for example of 200 ohms.

Fig. 9b shows an electrical topology to inexpensively compensate for ambient temperature variation, using an ambient temperature sensor T5 that is not touching the tubing.

Illustrated voltages are exemplary only.

When the device compensates for variation in ambient temperature, the heating element 22 needs, at minimum, to only dissipate a fraction of one Watt of power (i.e. generate a heating power of less than 1 Watt) and the temperature change of the fluid induced by the heater need only be a few degrees or even a fraction of a degree. In experiments proving the concept of this invention, a heater power dissipation of 0.25 Watts and even as low as 0.02 Watts was sufficient to provide a useful signal.

Fig. 10 illustrates the output of a thermistor resistive divider circuit in a prototype device, with T3 as the upper resistor in the divider and T1 the lower resistor (see Fig. 9a (right)), when the heater is dissipating a constant heating power of 0.25 Watts. Water is the fluid. It flows for the first 60 seconds at about 200 milliliters/hour. It then stops for 120 seconds. It then restarts flowing at the 180 second point. The x-axis is time in seconds and the y-axis is counts out of a 10-bit analog-to-digital converter. The change when water is flowing versus not flowing is clearly visible. In particular, inflection point 104 indicates the point at which flow stops, and inflection point 106 indicates the point at which flow starts.

The measurements of the inventor demonstrate that the thermal signal at T3 due to changes in flow is an order of magnitude larger than the thermal signal downstream at T1 due to changes in flow. In a continuously flowing regime, by the time the heat reaches the T1 location there is only a slight increase in temperature of T1 due to flow, whereas T3 is in close thermal proximity to the heater through path 72 (see Fig. 7). However, ambient temperature variation affects both sensors equally. Thus the advantage of including optional sensor T1 is primarily to compensate for ambient temperature.

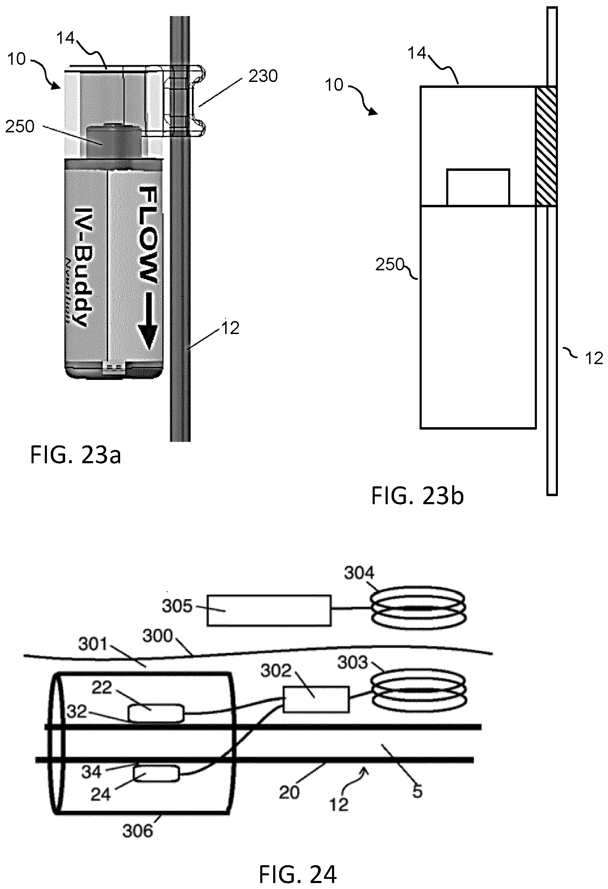

Since the primary advantage to using optional sensor T1 is thus to compensate for ambient temperature variation when operating the heater at a lower power, in some embodiments, a separate dedicated ambient temperature sensor T5 can be substituted for sensor T1 (see e.g. Fig. 3). T5 only measures ambient temperature and does not need to touch the tubing. A major advantage of this arrangement is that T5 can be spaced from the tubing, which reduces thermal interference. Using T5, the same compensation for ambient temperature variation is achieved, at the cost of only a small reduction in signal modulation due to flow. The advantage of using a sensor T5 remote from the tubing is that the whole device can be made considerably smaller since the only required physical connection to the tubing is at a single axial point along the tubing with the heater 22 on one side and T3 on the other. This significantly reduces the size of the device as it does not need to also touch the tubing downstream. This also for example enables greater options for the location of the battery and electronic circuits with respect to the tubing. For example, Fig. 23 (described later) shows that the whole device can be made only slightly larger than a one cell AAA battery used to power it, and only touch the tubing at one point.

It is noted that, in the above embodiment, the compensation is achieved by analog circuitry rather than by a microprocessor. Reference in this disclosure to a controller compensating the temperature output from sensor T3 should be understood as covering the option that the controller is a control assembly which includes the analog compensation circuitry.

Thus, at least some embodiments of the invention include an electrical circuit topology that automatically compensates for changes in ambient temperature and that simplifies the electronic design used to detect cessation or resumption of flow of liquid in the tubing.

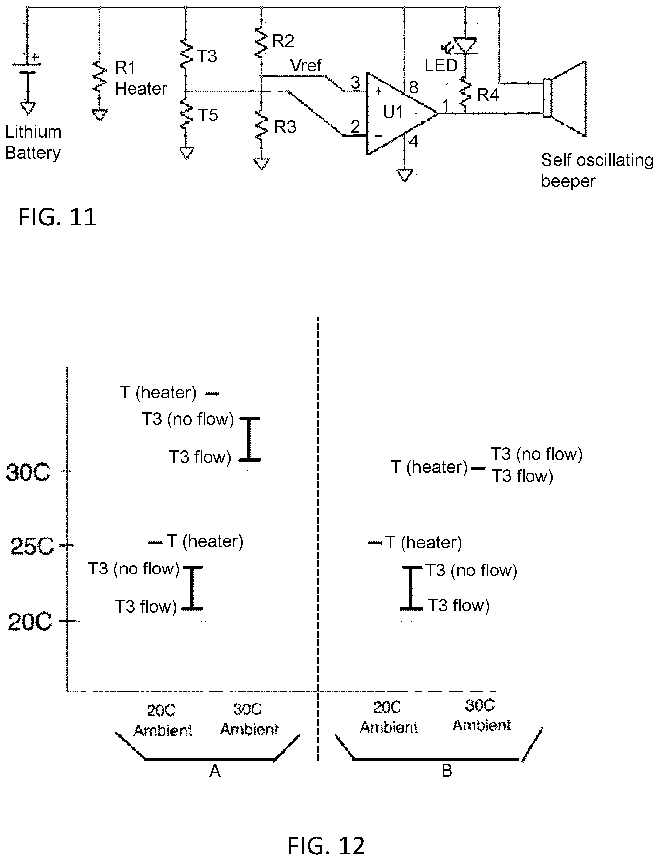

Fig. 11 shows a very simple electrical circuit that reports a no-flow condition following the principals of one or more embodiments of this invention. Thermistor temperature sensors T3 and T5 are used in a resistive divider arrangement so that compensation for ambient temperature is automatically achieved. In the illustrated example, these thermistors are each 1K Ohm at 25 degrees C. If using a lithium battery, the voltage is approximately constant over much of its capacity discharge, so the power in the resistor heater element R1 is more constant than using an alkaline battery chemistry. Resistor divider R2 and R3 create a threshold voltage Vref above which the flow will be considered stopped, and that threshold automatically adjusts for the small voltage change in the battery voltage as it discharges. Electronic comparator U1, a red light emitting diode and a beeper alert when flow has stopped. This design relies on the natural thermal response time of the fluidics to provide the desired delay when reporting a stopped-flow condition, with some adjustment of this time being possible by changing the ratio of resistances R2 and R3. In this example, the controller previously discussed is provided by the circuit shown in Fig. 11.

The simplicity of this circuit means that it can be manufactured at very low cost, consistent with a disposable single-use device.

In accordance with one or more embodiments, temperature sensor 28, T4 shown in Fig. 4 may optionally be included in the device and may be used to compensate the algorithm for fluid that enters the inlet at a temperature different than ambient temperature. For example, in blood donation situations the inlet temperature may be closer to body temperature and thus higher than ambient temperature, or if the IV bag has been refrigerated, the inlet temperature may be lower than ambient temperature. The value of T4 can be used to adjust the value of the temperature measured at the other sensors to compensate for the inlet temperature difference. The amount of this adjustment depends on the thermal resistances between the different sensors. It is noted that this thermal resistance depends upon the fluid flow rate. For example, if the temperature T4 is greater than T5 then the measured value of T5 could be adjusted upwards somewhat to compensate for a higher inlet temperature. An alternative arrangement would be to position temperature sensor T5 to measure the temperature of the tubing at position T4, rather than ambient temperature elsewhere, to automatically compensate for inlet fluid temperatures that are different than ambient.

For the most consistent performance with variation in ambient temperature when operating at lower power settings, it is preferable that the heater dissipate a constant power, not operate at a constant temperature. With constant power dissipation, any change in ambient temperature will result in the same change of temperature for both the heater element 22 the temperature sensor T3. This is not the case if a resistive heater were controlled to a fixed temperature setpoint, where, in an extreme example for illustration purposes only, if the ambient temperature were to rise above the heater temperature setpoint then the heater would no longer have the desired effect of heating the fluid inside the tubing above ambient. This is illustrated in Fig. 12. Fig. 12 illustrates the difference between using a constant power heater element (in accordance with embodiments of the present invention) and a constant temperature heater element.

The left half (A) corresponds to a case of constant power dissipation from a heater. The right half (B) corresponds to a case of constant temperature output from the heater.

The left half (A) shows the temperature of the heater element ("T (heater)") and the temperature of sensor T3 both under flow ("T3 (flow)") and no-flow ("T3 (no flow)") conditions, when a constant power is dissipated in the heater. In this this case, changes in ambient temperature (x-axis) just shift all temperatures by the same amount.

The right half (B) shows the alternative case where a resistive heater is controlled to a constant temperature setpoint, such that if the ambient temperature rises above that setpoint the heater will turn off until temperature drops. This has the result that there is no change to sensor T3 between flow and no-flow conditions when the ambient temperature is higher than the setpoint temperature (in this case 30 degrees), as T3 and the heater will just sit at the ambient temperature. It would be possible to operate a constant temperature heater at temperature higher than the highest anticipated ambient temperature (with the drawbacks of using more battery energy or potentially damaging the fluid), but it will never have as much signal modulation as with a constant heater power arrangement.

The power dissipated in the heater is the product of the voltage times the current, or in a resistive heater element it is the voltage squared divided by the resistance. Near constant power dissipation in this heater can be achieved in a number of ways, outlined below.

In some examples, a resistive heater can be powered from a constant voltage or constant current supply in order to dissipate a constant power.

In some examples, if powered from a battery whose voltage drops as it is discharged, the battery chemistry can be chosen with the flattest discharge voltage curve to minimize the power change as it is discharged. For example, lithium chemistries would be preferred over alkaline chemistries as their voltage droops less as they are discharged.

In some embodiments, a pulse-width modulation technique can be used in conjunction with a hardware circuit or with microcontroller firmware that measures the supply voltage and adjusts the duty cycle of the heater to, on average, dissipate a constant power over the expected range of battery voltages.

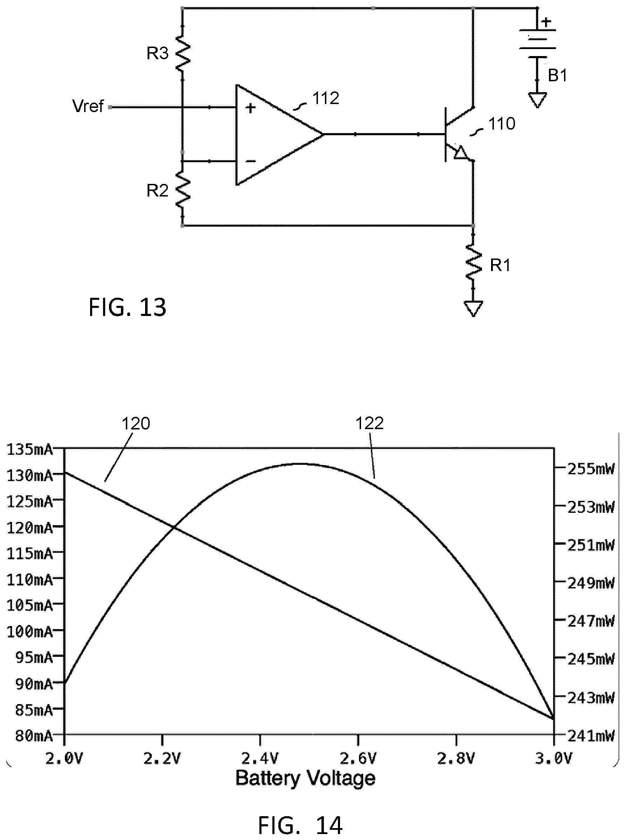

In some embodiments, a power transistor with a heat-spreading pad can be used as the heater element. A circuit topology such as that shown in Fig. 13 can be used to drive the transistor 110 so that its power dissipation is nearly constant over the expected range of battery voltage, as shown in Fig. 14. Feedback to an operational amplifier 112 is the sum of a voltage component across the transistor 110 and a current component through it. This method was tested by the inventor in prototypes. With this arrangement, the power dissipated in the transistor 110 remains approximately constant despite changing battery (B1) voltage. Fig. 14 shows a simulation output of the transistor heater element controlled for a near constant (0.25 Watt) power dissipation over the range of expected battery voltage. The x-axis shows battery voltage. Line 120 shows the current in the transistor (y-axis, left). Line 122 shows the power dissipated in the transistor (y-axis, right).

In accordance with one or more embodiments, a preferred method for achieving a constant heating power by the heating element 22 is to use a pulse width modulation (PWM) technique.

For example, the device may comprise a local power source (e.g. a battery), and the controller may be adapted to convert an electrical output from the local power source into a pulse width modulated (PWM) electrical supply for driving the heating element, and to provide the pulse width modulated electrical supply to the heating element. The controller may be adapted to adjust a duty cycle of the PWM electrical supply in dependence upon the voltage of the power source so as to maintain a constant power dissipation in the heating element. This may in some cases mean maintaining a constant power input to the heating element (e.g. if the heating element is a resistor).

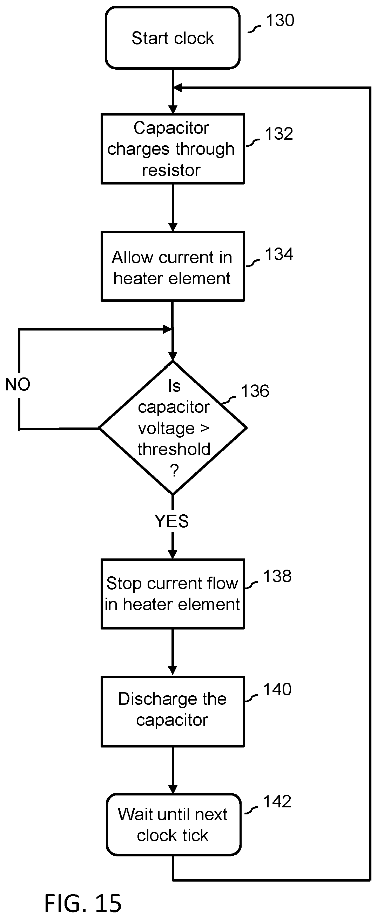

One advantageous set of embodiments may use a PWM technique to repeatedly charge a capacitor through a resistance connected to the battery voltage, where the resulting timing of the capacitor voltage is monitored using the algorithm shown in Fig. 15. This approach would be suitable if a microcontroller was not available.

Fig. 15 illustrates steps of an example firmware algorithm to produce an approximately constant power in a resistive heater element as battery voltage changes, using a resistance-capacitance (RC) network to control pulse-width modulation (PWM) timing.

The steps are as follows.

In step 130, a clock is started, for example with a 1 ms tick time/interval.

In step 132 a capacitor is allowed to charge through a resistor from the (e.g. battery) voltage source driving the heater.

In step 134, current is allowed through the heater element.

In step 136, it is determined whether the capacitor voltage exceeds a threshold. This decision step is looped until the result is YES, at which point, in step 138, current to the heater element is stopped. In step 140, the capacitor voltage is discharged to zero. The method then waits 142 until the next clock tick.

It can be seen that this will create a PWM scheme in which the pulse width will be automatically adjusted (via decision step 136) to provide an approximately constant power input to the heater

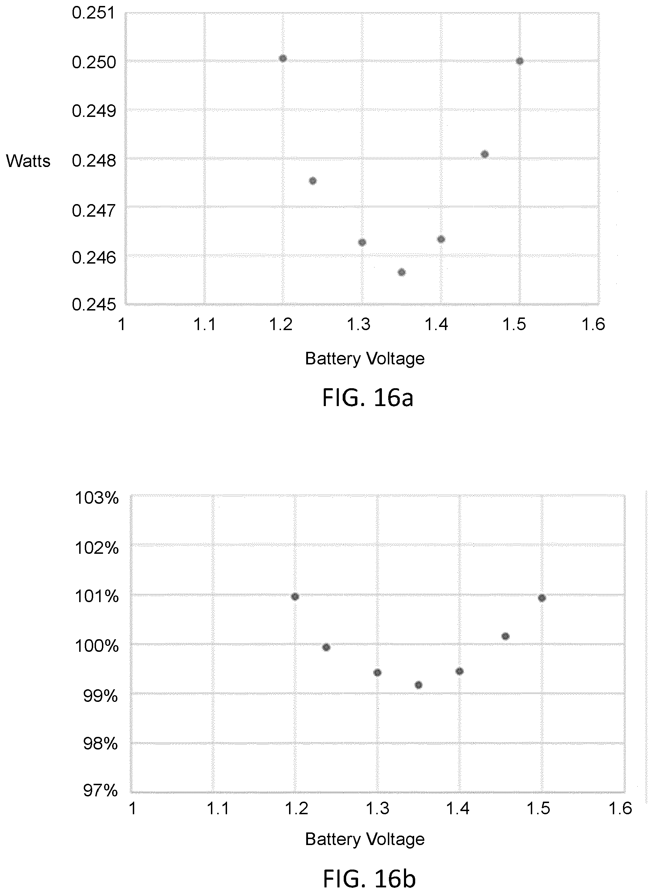

In this example, the power supply is a battery. The battery in this specific example is an Energizer L92 lithium cell whose cell potential ranges from 1.5 V to 1.2 V as it is discharged. The heater is a standard electronic resistor element of 5.49 Ohms, the RC time constant for the algorithm is 617 microseconds and the threshold voltage used in the algorithm is 0.945 V. Fig. 16a shows the resulting average heater power dissipation (y-axis, Watts) is approximately 0.25 Watts. Fig. 16b shows that the resulting heater power dissipation as the battery voltage (x-axis) declines varies by less than approximately +/- 1%. Without this method (or something with equivalent effect) the heater power variation would be +/- 18% over the same range of battery voltage.

In some embodiments, the controller is adapted to selectively operate the heating element 22 in one of at least two different heating power modes, and wherein substantially constant heating power is provided in only one of the two modes. For example, the controller may be adapted to operate the heating element in a higher heating power mode when there is non-zero flow, and to switch to a lower heating power mode following detection of no-flow, wherein at least an average heating power dissipation in the higher heating power mode is greater than an average heating power dissipation in the lower heating power mode. In the higher power mode, optionally the heating element may be controlled to provide substantially constant heating power. In the lower power mode, optionally substantially constant heating power may be provided or the heating power may be controlled differently.

As mentioned briefly above, in accordance with one or more embodiments, the device may be adapted to generate a user-perceptible alert to signal flow stoppage, and wherein a delay is imposed between detecting flow stopping and generating the alert, for example of approximately 60 seconds.

By way of background, it has been realized by the inventor that certain neurovascular procedures reveal another important limitation of traditional flow monitoring methods: they report a flow stoppage immediately. This is not consistent with the workflow of such procedures. It is normal in neurovascular procedures for there to be three to six bags of anticoagulant (and sometimes more) being infused into the patient simultaneously, each in a different catheter at a different vascular access port. During the procedure, the clinician selectively turns certain infusions off and then back on as different tools are repeatedly introduced and then removed from the catheters. If one of the infusions is turned off for more than 1 minute there is a serious risk to the patient of blood clot formation in the catheter and resulting stroke. With the various different tubing lines being turned off and then on it, is easy for the clinician to lose track of which lines are stopped and for how long. If a device is configured to provide a flow alarm that sounds immediately upon flow stoppage, this simply adds to the workflow clutter since flow stoppage in a line is intentional so long as it lasts no longer than 1 minute.

What a clinician in fact requires is to be alerted in case of an exception to proper practice, for example to sound an alarm only if one tubing line is shut off for more than 30 seconds so that the clinician can react to resume flow at less than the 1 minute time point. This would mean that alarms would be rare, and only occur if an actual problem exists. However, when flow is restarted, the alarm should stop immediately, since in this case the problem has been cleared. Thus, it is a realization of the inventor that there would be significant benefit in providing an asymmetrical delay: a delay when flow stops before creating an alert, but no delay when clearing the alert.

Depending upon the clinical scenario, different specific asymmetrical delay times may be appropriate. For example, if infusions are disrupted during patient transport or during home care it may be appropriate to have a longer delay before providing an alarm. However the principle is applicable in all scenarios: namely, generate an alert in the event that further delay could harm the patient, but stop the alert immediately when flow resumes.

There are existing thermal methods to measure flow and direction of flow in infusion tubing, but they do not provide asymmetrical delay time in reporting the stop of flow and immediately detect and report the resumption of flow.

Thus, in accordance with one or more embodiments, the controller may be adapted to generate an alert signal after a pre-set non-zero time delay following detecting flow stopping, and to terminate the alert signal immediately responsive to detecting flow starting. The alert signal may be a user-perceptible alert signal, e.g. a sensory output.

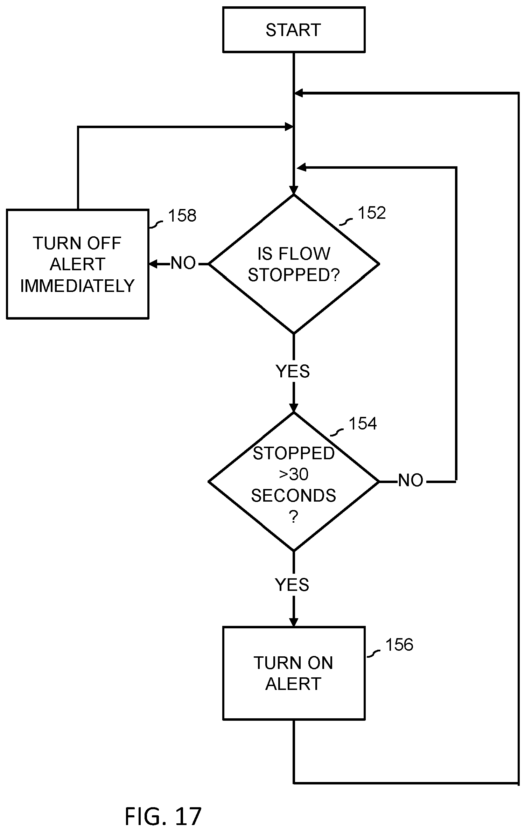

By way of example, Fig. 17 shows an example algorithm for introducing an asymmetrical delay when starting an alert following detecting flow stoppage and no delay when clearing an alert following detecting flow restarting.

The algorithm comprises a step 152 of detecting whether liquid fluid is flowing in the infusion tubing or not, for example based on any of the techniques discussed in this disclosure. If flow has stopped, a further decision step 154 assesses whether a pre-set time delay has passed (e.g. 30 seconds in the illustrated example). Once the time delay has passed, the alert is activated 156. The method then returns to checking whether flow is still stopped. As soon as flow restarts, the alert is deactivated 158 immediately. The time delay of step 154 may be set, by way of example, in the range of 20 seconds to 10 minutes or more, typically with a time in the 30 - 60 second range. When fluid flow is restored, the alert condition is cleared promptly without delay.

It is noted that for a device adapted only to make a binary detection of a flow/no flow condition, the circuitry can be made simpler and the device physically smaller than prior art devices that seek to quantify an actual rate of flow.

As briefly mentioned above, the technical advantages of the device can be further enhanced in accordance with one or more embodiments by providing means for compressing a section of the tubing when the device is attached thereto, to reduce the radial distance between the heater and the sensor.

In particular, according to one or more embodiments, the device includes a means for applying a compression to the tubing wall, wherein the compression reduces a radial distance between the first contact area and the second contact area. The compression is for example a squeezing action. The compression results in inward deformation (crushing) of the tube at the axial location of the heater 22 and temperature sensor T3, so that they are brought radially closer together.

The means for applying a compression may be adapted such that coupling the housing to the tubing (where one is provided) has the effect of causing the compression to be applied.

In preferred embodiments, the means for applying the compression is provided by the housing, wherein the housing is structured such that, when coupled to the tubing, the compression is applied by the housing to the tubing wall.

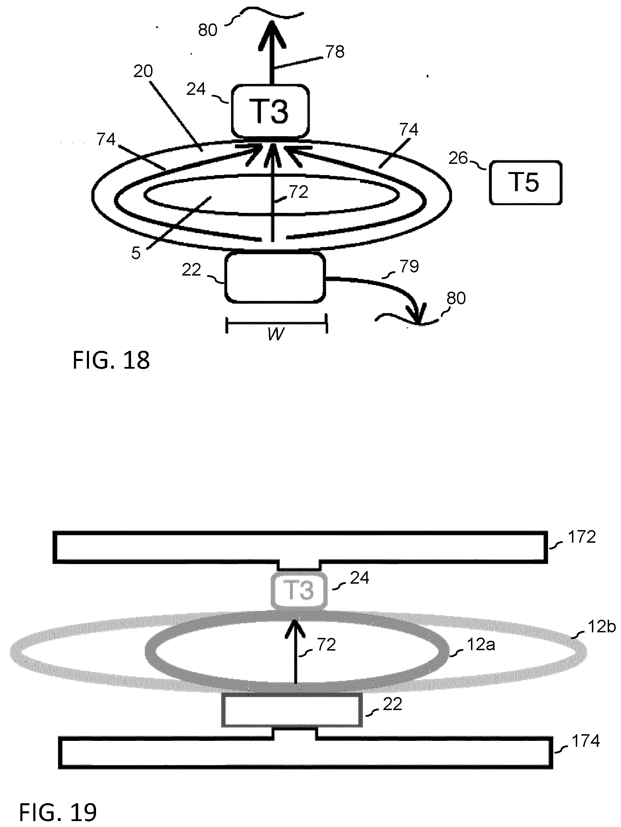

Fig. 18 illustrates the same arrangement as in Fig. 7, but wherein a compression has been applied as described above so that tubing is flattened at the axial location of the heating element 22 and the temperature sensor T3. This decreases the thermal path length (path 72) radially through the fluid from the heater within the lumen of the tubing, and moreover decreases this radial thermal path length as a ratio of the circumferential thermal path length 74 through the wall of the tubing. This therefore also decreases thermal interference via thermal conduction through the wall 20.

Introducing an intentional deformation of the tubing wall has a number of benefits, outlined below.

First, it shortens the thermal path length 72 radially through the fluid between the heater 22 and the sensor T3 so that radial path 72 becomes a more significant influence on the temperature at T3. This has the beneficial effect of increasing the thermal signal at T3. Second, it results in the temperature sensor at T3 heating more quickly when flow stops because there is just a smaller volume of fluid between the heater and T3, so there is less fluid in that region to heat up. This has the effect of reducing the response time between flow stop and detection of flow stop by means of an inflection in the temperature signal.

Third, it likewise has the result that the temperature sensor at T3 responds more quickly to resumption of fluid flow due to the shortened radial path length 72. By way of example, this can also have the benefit that any "flow stopped" alarm can be cleared quickly. This can be seen in Fig. 10 where the signal drops immediately and precipitously when flow starts (point 106).

Fourth, it makes the system very sensitive to very slow flow rates, such as for the TKO ('to keep open') lines discussed previously, because it reduces the volume of fluid between the heater and T3. Even very slow flow rates will sweep the smaller volume of fluid out quickly and replace it with fresh fluid.

Fifth, it can in some cases be a useful means for making the device performance consistent when working with a variety of tubing diameters. In particular, if the tubing compression is achieved by two plates that, in operation, are a fixed specific distance apart, then the radial thermal path length 72 between the heater and T3 will be fixed length, regardless of the initial diameter of the tubing. This principle is illustrated in Fig. 19 which illustrates a means for applying a compression to the tubing 12 in the form of a pair of opposing plates 172, 174, which might be accommodated in the housing in such a way that when the housing is coupled to the tubing 12, the tubing is received between the plates, and the plates are set at a pre-defined spacing apart from one another, which may be smaller than an expected minimum diameter of tubings with which the device is to be used. This has the effect of applying a compression to received tubing which results in a consistent radial path length 72 between the heater 22 and sensor T3 regardless of the initial diameter of the tubing. For example, Fig. 19 schematically illustrates two different tubings 12a, 12b of different uncompressed diameters within the device.

A further refinement to the device is now discussed by referring again to Fig. 10. It can be seen that the signal rises with a gradual ramp when the fluid flow stops at point 104, since heat from the heater traverses the lumen of the tubing and reaches the radially opposed temperature sensor. Then when flow restarts at point 106 the signal drops precipitously. One can imagine that at some signal threshold level, such as at 480 counts, the device may conclude that flow has stopped and alert the user. It would be possible to reduce the power dissipation in the heater once that threshold has been crossed, as there is no value in the signal continuing to rise beyond the threshold as long as it stays above the threshold when flow stays stopped. Resumption of flow, shown as 106 in Fig. 10, could still be determined when the signal drops precipitously; it just would be dropping from a lower signal level than if the heater power were maintained at a constant power. The advantages of lowering the heater power when flow has been detected as stopped is that it would use less energy from a power source (e.g. a battery) and it could avoid overheating and damaging the fluid in the tubing. This technique of adjusting the heater power dissipation depending upon detected flow condition might have particular advantage when the fluid is flowing intermittently, in which circumstance it spends a larger percentage of the time in the no-flow condition. The advantage of using a higher heater power dissipation is that the response time when the flow stops is faster, but the advantage of switching to a lower heater power dissipation when flow is detected as stopped is reduced energy use and lower fluid temperature.

In view of these observations, as a more general principle, it is proposed that, in some embodiments, the controller is further adapted, in at least one phase of operation, to adjust a heating power output or dissipation of the heating element based upon changes in the detected flow parameter or condition.

In some embodiments, the detected flow parameter or condition includes a flow stop/start condition; and wherein said adjusting the heating power dissipation in dependence upon changes in the detected flow parameter or condition comprises reducing the heating power dissipation following detection of a flow stop condition.

The controller may be adapted to selectively operate the heating element in one of at least two heating power modes: a higher/standard heating power mode and lower heating power mode, wherein at least an average (e.g. time average) heating power dissipation in the higher heating power mode is greater than an average heating power dissipation in the lower heating power mode, and wherein said adjusting the heating power dissipation in dependence upon changes in the detected flow parameter or condition comprises at least switching to the lower heating power mode following detection of a flow stop condition.

Preferably, when the flow condition/state is a flow start state, the controller is adapted to operate the heating element in the higher power mode. For example, following detection of a flow start state, the controller is adapted to operate the heating element in the higher power mode. In this higher power mode, when the flow condition is a flow start state, the controller may be adapted to control the heating element to dissipate a substantially constant heating power. In the lower power mode, the heating power may be substantially constant, at a lower level, or may be controlled dynamically in dependence upon the signals derived from one or more temperature sensors.

In some examples, the controller is adapted to reduce the power dissipation of the heating element to the lower power mode responsive to detection of persistence of the flow stop condition for a threshold time period or a threshold number of temperature signal sample points, and/or responsive to detection of the first temperature sensor signal, T3, or a function or correlate thereof, falling below a threshold level.

As explained above, when the heating power dissipation is in the lower power mode, the controller can detect transition of the fluid flow from the flow stop condition to the flow start condition based on detecting a negative inflection in the first temperature sensor signal, or a function or correlate thereof, or based on detecting a negative gradient in the first temperature sensor signal (or a function or correlate thereof) crossing a pre-defined threshold, or based on the first temperature sensor signal (or a function or correlate thereof) crossing a threshold temperature.

The controller is adapted to return the heating element to the higher heating power dissipation mode following detection of a flow start condition.

By way of further illustration, one particular example device built as a prototype by the inventor will now be discussed. The details of this device do not limit the scope of the overall inventive concept and are provided by way of illustrative example.

A breadboard SENSOR MODULE was constructed to test the method of flow detection. The materials and equipment were as set out in Table 1 below:

**Table 1**

| | |
|---|---|
| VOLTAGE_SOURCE_1 | Leader LPS-151 |
| VOLTAGE_SOURCE_2 | Power Designs Model 5020 |
| VOLTMETER | Fluke 189 |
| HEATER_RESISTOR | Yaego RSF200JB-73-200R |
| THERMISTOR T1 | EPCOS B57540G0104F000 |
| THERMISTOR T3 | EPCOS B57540G0104F000 |
| FOAM BOARD | Elmer's Products, Inc. #950109 |
| IV BAG | Hospira 7951-12 |
| IV tubing set | Millpledge HB06536A |
| Timer | Radio Shack 63-1601 |

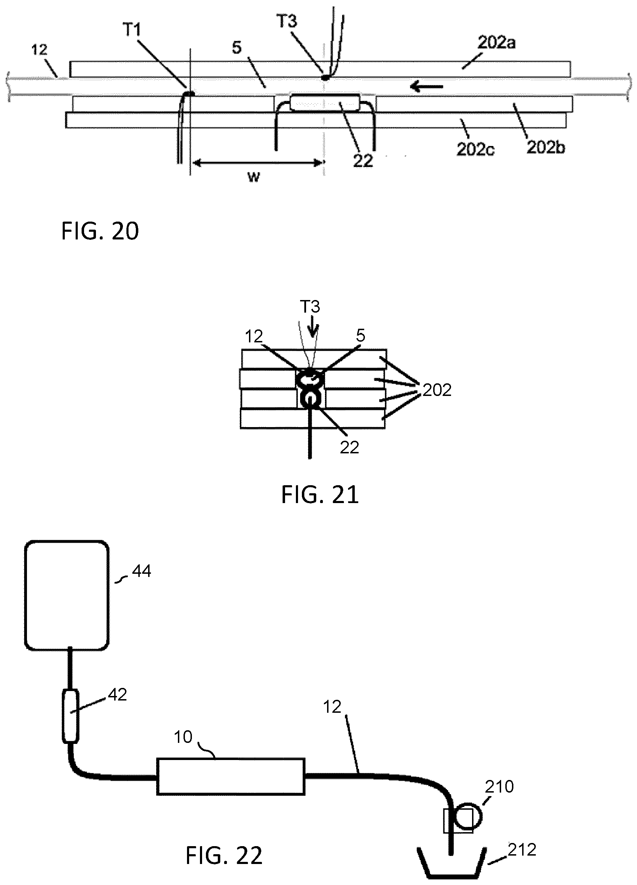

Construction of the device was as follows. A 5 mm thick foam board was initially cut into multiple pieces 202 approximately 150 mm long and 38 mm wide. Then multiple pieces 202a, 202b, 202c,..., 202n were stacked together as shown in the side cross-sectional view Fig. 20 and end cross sectional view Fig. 21.

A pocket was cut in one foam board piece 202b to accept the body of the resistor (indicated at 22). The wire leads of the resistor 22 were punched through to exit the bottom foam board. Another piece of the foam board was cut in two longitudinally to create a channel down the middle to accept the IV tubing 12. Two thermistors T1, T3 were placed as shown in Fig. 20, squeezed between the compliant IV tubing 12 and the foam board 202. The wire leads of thermistor T1 were punched through to exit the bottom foam board. The wire leads of thermistor T3 were punched through to exit the top foam board. The whole assembly was held together with adhesive tape.

Fig. 22 shows a schematic of the fluidic layout. An IV bag 44 was filled with tap water and hung upright with a drip chamber 42 hanging vertically below it. The tubing exited the drip chamber and then travels through the sensor module 10 and then through an adjustable shutoff valve 210, and then to a waste bucket 212. The shutoff valve can be adjusted to control the flow rate as measured by the number of drops per second visible in the drip chamber. This value can be set to completely stop the flow. The calibration of the particular drip chamber used is 60 drops per milliliter, or 17 microliters per drop.

The electrical circuitry shown in Fig. 9a, and discussed above, was used.

Fig. 9a shows the electrical connections, with the resistor 22 powered from a 7.0V power supply called VOLTAGE_SOURCE_1 so that the resistor dissipates approximately 0.25 Watt of power. A VOLTMETER was used to measure the voltage between points A and B. The VOLTAGE_SOURCE_2 was a precision adjustable power supply whose output voltage was adjusted to give 1.00V on the VOLTMETER across A-B when fluid flow was stopped; VOLTAGE_SOURCE_2 ended up being set to 2.61V to make this happen.

A measurement procedure was performed comprising stopping all fluid flow and then adjusting VOLTAGE_SOURCE_2 to produce 1.00V from A-B. Then flow was started at about 1 drop per second in the drip chamber and the voltage across A-B was measured and recorded after it equilibrated. Flow was increased to approximately 2 drops per second and the voltage across A-B was measured and recorded after sufficient time passed for it to equilibrate.

The results are as follows:
Voltage when fluid flow is stopped: 1.00 V
Voltage at 1 drop/second rate: 0.81 V
Voltage at 2 drop/second rate: 0.81 V

The next measurement procedure was to measure the response time. An experimental threshold of 0.90 V was used as the point where flow is considered to have started or to have stopped. If flow is established and then flow is stopped, after some period of time which might be termed "Time to Alarm" the voltage from A to B will rise above the 0.90 V threshold. If flow has been stopped and then flow is restarted again, after some period of time that might be called "Time to Clear" the voltage from A to B will drop below the 0.90 V threshold. The objective was to measure how long it takes before an alarm condition is measured (the Time to Alarm) after flow becomes stopped, and how long it takes to clear an alarm after flow is resumed. Two trials were performed under the same conditions. The flow rate when flowing was approximately 1.4 drops per second.

The results are as follows:
Trial 1 - Time to Alarm: 37 seconds
Trial 1 - Time to Clear: 4 seconds
Trial 2 - Time to Alarm: 35 seconds
Trial 2 - Time to Clear: 4 seconds.

This shows that it takes a little more than half a minute after flow stops before it can be determined that there is a problem with flow being stopped. Note that in a device, this time can be easily extended to a longer time by triggering another delay before an alarm is reported to a user. This delay can be provided in firmware or by other means such as an analog or digital timer. So, for example, an alarm can be delayed so that an alarm will not be given to the user until 1 minute has passed with no flow. This data also shows that once flow resumes the alarm can be cleared quickly, in only a few seconds. If an alarm is given to the user and the user makes changes to the tubing to restart flow, the device should then respond quickly to any flow that starts up and proceed to clear the alarm.

By way of illustration of Fig. 23a shows an exterior view of an example device according to one or more embodiments, and Fig. 23b shows a cross-section through the same example device. This example device comprises a heater and sensor arrangement as shown in Fig. 2 or Fig. 3, with only one temperature sensor touching the tubing. As a consequence, the section of the device which touches the tubing (indicated at 230) can be very short, as it only needs to touch the tubing at one point. There is no need for the additional length that would be required if a channel were used to accept and hold the tubing against multiple sensors that are axially displaced along the tubing. This allows the device to be very compact. The size of the housing pieces that close over the tubing need only be big enough to allow the user's fingers to conveniently close the housing. Fig. 23a shows the size of this part relative to the size of a single AAA battery 250 which is used to power the device. The housing 14 of the device in this example is not much larger than the battery 250, wherein the housing accommodates the battery inside, and retention means for holding the housing and battery mechanically coupled together. The battery electrically connects to the device at an electrical connection site inside the housing.

As mentioned briefly above, in accordance with any of the above-decribed embodiments, the device can be provided as an implantable device. This also represents a further aspect of the invention. For example, there may be provided an implantable device for use inside the body of a subject for sensing a fluid flow through a tubing within the body, for example through a medical tubing. The device comprises a heating element 22 and at least one temperature sensor 24. The device comprises a controller operatively coupled to the heating element 22 and temperature sensor 24. The device is arranged in use to hold the heating element positioned in thermal communication with the fluid in the tubing at a first location 32, and the temperature sensor positioned in thermal communication with the fluid in the tubing at a second location 34, wherein the second location is substantially radially opposite the first location across the lumen 5 of the tubing. The controller is adapted to: control the heating element to dissipate heating power; and detect a flow parameter or condition based on at least an output from the temperature sensor.

Any of the example features or embodiments discussed above or below may be incorporated or applied to the implantable device.

**In** some embodiments, the device includes at least a section of the tubing. **In** some embodiments, the device further includes a thermally insulating enclosure, for impeding heat dissipation from the heating element to an outside portion of the device, and wherein the heater and temperature sensor are housed inside of the thermally insulating enclosure. **In** some embodiments, the thermally insulating enclosure is coupled or mounted to an exterior of the tubing. **In** some embodiments, the thermally insulating enclosure at least partially surrounds an exterior wall of the tubing, for example encircling the exterior wall of the tubing.

By way of one example, Fig. 24 shows an embodiment of the invention where the tubing 20 is embedded in the body in a subcutaneous region 301 below the surface of the skin 300. The heater 22 is positioned on one side of the fluid lumen 5 and the temperature sensor 24 is positioned on the radially opposed side of the fluid lumen. A thermally insulating enclosure 306 thermally isolates the heater and temperature sensor from rest of the region inside the body. An internal controller 302 provides a substantially constant power to the heater and measures the signal from the temperature sensor. An external controller 305 provides power to the internal controller 302 via external induction coil 304 and internal induction coil 303. The internal controller 302 is in two-way communication with external controller 305 via signals carried between coils 303 and 304.

The thermally insulating enclosure may act to hold the heating element 22 and temperature sensor 24 at their respective locations. Alternatively they may be attached or mounted to an outside portion of the tubing. Alternatively, they may be embedded or integrated at least partially in a wall of the tubing. Alternatively they may be disposed inside the lumen of the tubing, for example attached to an inner wall of the tubing.

The embodiment described above has a wireless power delivery system.

Thus, more generally, there may be provided in accordance with one or more embodiments an apparatus, comprising the implantable device of any preceding claim; and an external interface unit. The previously mentioned controller may comprises an internal controller portion 302 disposed in the implantable device and an external controller portion 305 disposed in the external interface unit, and wherein the heater and temperature sensor are each electrically connected to the internal controller portion, and wherein the internal controller portion comprises first inductive power coils 303 for inductively receiving power from second inductive power coils 304 comprised by the external controller.

The internal controller portion is adapted to communicate wirelessly with the external controller portion to send temperature information from the temperature sensor.

Wireless communication between the internal and external controller could be achieved in a variety of different ways.

One approach may be to apply modulation to the wireless/inductive power signal, such as is utilized in the Qi standard for charging mobile computing devices.

A further approach may be to utilize the internal and external inductive coils as radio frequency antennas, whereby wireless communication may be achieved by coupling of radio signals between the inductive coils.

A further approach may be to include a separate internal and external radio antenna for facilitating radio communication.

A further option may be to provide an optical communication interface between the internal and external controller portions. Optical signals are able to penetrate skin, particularly if a thin section of skin is used as the interface area. An optical wavelength may be utilized which has optimal transmission through the skin. One option is infra-red or near infra-red light. Another option is visible light. A respective photo-sensitive detector and LED emitter may be included in both the internal controller portion and the external controller portion to thereby permit two-way communication. This provides a low cost and simple option.

Another option may be to use ultrasonic communication.

Another option may be to provide means for triggering communication with an in vivo switch. In other words, the communication may be physical or tactile. For example, a clinician would press on the skin surface to start the communication process. For example, it is to be noted that a communication session may only be needed very occasionally such as when the patient goes to the clinic once a month to have their extra ventricular drain shunt checked.

With regards to an implantable flow sensing device, there are numerous possible applications.

One particularly advantageous application of an in vivo device is for extra ventricular drain (EVD) shunts. Tubing may be put into a ventricle of the brain and run down to the peritoneum to drain. This relieves pressure in the brain. This tubing can easily get clogged so needs to be checked periodically that it will still flow. The patient would lie down for a certain period, e.g.15 minutes. The external controller may be positioned on the patient's chest for supplying power to the device. Flow through the tubing may then be monitored over the time period to check that fluid is flowing properly. If no flow is detected, then the tubing is clogged and must be replaced.

A variety of further optional features which may be incorporated in one or more embodiments will now be discussed.

Power is consumed by the heater element and if the device is battery powered then attention needs to be paid to how long the battery will last. For some applications such as home healthcare, battery run time may be extended by periodically turning off the heater to save some power, and then turning it on to make the measurement and then turning it off again. The degree to which this duty cycle technique can be used depends upon how quickly the heater warms the tubing and the time constant of the thermal path between the heater and the temperature sensor when flow is stopped. For example, if it takes 2 minutes for the temperature sensor reading to equilibrate once the heater is turned on, and if it is acceptable to check for flow only once every 5 minutes, then the heater may be kept off for 3 minutes then turned on, and two minutes after that the temperature is measured to determine if there is flow and the results reported. Then the heater is turned off again and the cycle repeated. In that example the power dissipated in the heater has been reduced to 40% of the power if the heater were to run continuously, and the battery run time extended by approximately 2.5 times.

The "alarm" or "alert" mentioned earlier in this disclosure may take several possible forms. Audible or visual alarms are possibilities. Another possibility is wireless communication of the flow results to other devices or to a central monitoring center. Wireless can for example be in the form of microwave, radio, infra-red or other optical communication, ultrasonic or other acoustic communication. Mechanical alarms are possible either to activate an indicator or to actuate or trigger another device. Alarm indicators that are silent (e.g. haptic) could be of benefit in military applications where flashing lights or noisy beeps that could give away a soldier's position are too dangerous in combat environments.

An alternative or additional way to report the stoppage of fluid flow is to report to the user the length of time that flow has been stopped. Practitioners of the art can readily design a readout that will display that time in seconds or other time-related units. Users of the device can then use their professional judgement to evaluate the consequences of the time duration of that stoppage of flow in the situation at hand.

Note that in this disclosure, the word "cooler"/"cooling element" may be substituted for the word "heater"/'heating element" in any of the description above and, if so, then the polarity of the decision thresholds should be adjusted appropriately. The idea is that the temperature of the fluid is modified at one point and then the influence of that temperature change is measured across the tubing on the opposing side.

The methods discussed here are not limited to medical products but are applicable to any liquid fluid flow situation, such as veterinary applications or applications in agriculture or as an embedded sensor in other products such as automobiles.

An aspect of the invention also provides a method for sensing fluid flow through a medical tubing line, comprising: holding a heating element in thermal communication with a lumen of the tubing at a first location; simultaneously holding a temperature sensor in thermal communication with a lumen of the tubing at a second location, wherein the second location is substantially radially opposite the first location across the lumen of the tubing; controlling the heating element such that it dissipates a heating power; sensing a temperature output from the temperature sensor while the heating element is active; detecting a flow parameter or condition of fluid in the tubing based on an output from the temperature sensor.

The method may in some embodiments further comprise compressing or manufacturing the tubing at the location of the heater and temperature sensor so that the radial distance between the first contact area and second contact area is reduced from a circular cross section. **In** some embodiments, the force applied to compress the tubing simultaneously acts to hold the temperature sensor and heater in contact with the tubing at the first and second contact areas.

Embodiments described above provide a thermally-based mass-flow sensor. As already discussed above, various embodiments may comprise a combination of one or more of the following advantageous features.

In some embodiments, the controller is adapted to perform only a binary flow detection: is there flow, yes or no? It may not include functionality for quantitating the rate of flow, which thereby simplifies operation and reduces the required number of temperature sensors.

In some embodiments, responsive to detecting flow stopping, the controller may generate a user-perceptible alert following a delay of a specific amount of time, and responsive to detecting flow restarting, the controller may immediately report the restart (e.g. by deactivating the alarm).

The temperature sensor is located substantially radially opposite the heater. This optimizes the sensor position to respond quickly when flow resumes, for example in order to promptly clear a reported flow stoppage.

The optimized sensor position also allows the device to be physically smaller than prior art devices, an advantage in the intended use cases.

In some embodiments, the shape of the tubing is intentionally distorted by the device to reduce the radial distance between the heater and the sensor, to thereby further optimize the response time of the temperature sensor, and improve the sensitivity to very low flow rates and adapt the system to a range of tubing diameters.

According to example circuit designs discussed above, the design of the electronic circuits for temperature sensing can be implemented at very low cost in a compact package.

A number of further possible advantageous features will now be outlined. These may be advantageously combined with any of the features, options or embodiments already outlined above, or outlined later below.

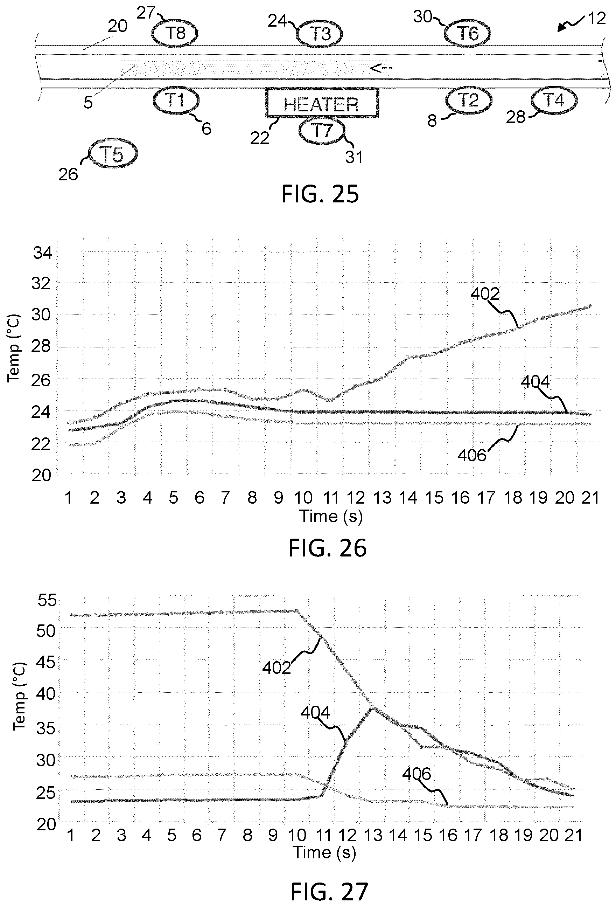

For ease of description of these features, Fig. 25 shows a representation of an example set of positions for possible temperature sensors relative to the heating element 22 which might be incorporated into a flow sensing device in accordance with one or more embodiments. It is noted that a flow sensing device may include all of these temperature sensors or may just include any selected sub-set. Inclusion of any one or more of these temperature sensors is compatible with any embodiment and any aspect of the invention described in this document. In addition to temperature sensors previously discussed, also shown in Fig. 25 is a further upstream temperature sensor T6, 30. This is disposed radially opposite the potential position of upstream temperature sensor T2, 8 previously discussed. In the illustrated example, the further temperature sensor 27 (T8) is shown positioned for sensing a temperature downstream of the fluid.

In an envisaged preferred embodiment, only one of the shown pair of sensors T1 and T8 may be provided and only one of the shown pair of sensors T2 and T6 may be provided. For example, instead of providing all of the set of sensors T1, T2, T8, T6, just T1 and T2 might be provided or just T8 and T6 might be provided. All sensors are shown in Fig. 25 to illustrate the different possible options. It may be advantageous for manufacturing to have all temperature sensors on one side of the tubing, connected all to one wire harness for example.

Fig. 25 further shows sensor T4. As discussed previously, this may have utility as an upstream sensor for purposes of sensing a temperature of fluid flowing into the device, wherein this temperature can be used to compensate temperature change measurements made at sensor T3 for example. This sensor could however be omitted.

In some embodiments, just one upstream sensor (e.g. T2 or T6) and just one downstream sensor, e.g. T1 or T8 may be provided (in addition to T3). The ambient temperature sensor T5 may optionally be additionally provided.

Also shown is a possible further optional temperature sensor 31 (T7), arranged for sensing a temperature of the heating element 22 itself.

As mentioned previously, sometimes fluid bags are brought from the refrigerator to be infused and thus the fluid enters the device at a temperature lower than ambient. Sometimes fluid bags are warmed to body temperature before infusion and thus the fluid enters the device at a temperature higher than ambient. Simply comparing the temperature at T3 to ambient may thus not be adequate to determine flow start/stop, and a higher heater power may have to be used to make the desired signal larger than the undesired temperature uncertainty at the inlet. However, if the tubing temperature is measured on the tubing upstream of T3, such as T2 or T6 or T4 in Fig. 25, that upstream signal may be used to compensate the signal at T3 and make the device more sensitive to flow stop. In this way, heater power may be minimized.

This can be seen in the graphs of Fig. 26. This graph shows the temperature of the fluid in the tubing upstream of the heater location by 1.6 cm (line 406), at the radially opposed temperature sensor T3 (line 402) and then downstream of the heater by 1.6 cm (line 402). Flow is running initially. After 10 seconds, the flow stops. Before the flow stops, it can be observed that the upstream temperature 406 has been varying and that the sensor at T3 402 varies along with the upstream sensor. Thus, taking their difference or ratio would be relatively unchanged while flow is occurring. Then, two seconds after the flow stops, the sensor at the heater begins to deviate from the upstream sensor signal. Thus taking their difference or ratio compensates for the incoming fluid temperature and has good sensitivity to flow stoppage.

Thus, in some embodiments, the device includes at least one further temperature sensor 8, 30, 28 positioned for sensing a temperature of the fluid at a location upstream from the heating element 22 (e.g. T2, T6, or T4 in the illustrated example), and wherein the controller is adapted to detect the flow parameter or condition based on a ratio or a difference between a signal of the first temperature sensor 24, T3 and a signal of the further temperature sensor, or a function or correlate thereof. For example, the controller may be adapted to detect at least a transition from a flow start condition to a flow stop condition based on a ratio or a difference between the signal of the first temperature sensor, or a function or correlate thereof, and the signal of the second temperature sensor, or a function or correlate thereof.

When the flow restarts after having been stopped, there is a momentary bump up in the temperature measured downstream (e.g. at T1 or T8 in Fig. 25) because the bolus of heated fluid flows downstream. This bump is in the opposite direction as the temperature at T3, which would be dropping. Thus the difference between, or ratio of, the temperatures at T3 and a position downstream, e.g. T1 or T8 in Fig. 25, yields a bigger signal change than simply looking at T3 alone. This results in a more sensitive and faster sensing of flow resumption. This is illustrated in Fig. 27 which shows the temperature of the fluid in the tubing upstream of the heater location by 1.6 cm (line 406), at the sensor T3 (line 402) and at a location downstream of the heater by 1.6 cm (line 404). Immediately after the flow restarts, at the 10 second point, the temperature upstream 406 and at T3 402 starts to drop and there is a transient local uptrend of the temperature signal downstream 404 shortly thereafter.

Thus, in some embodiments, the device further includes at least one further temperature sensor, the device is arranged in use such that the at least one further temperature sensor is positioned for sensing a temperature of the fluid at a location downstream from the heating element (e.g. T8 or T1 in the illustration of Fig. 25). The controller may be adapted to detect the flow parameter or condition (e.g. flow stop/start) based upon outputs of both the first temperature sensor T3 and the at least one further temperature sensor 6, 27, for example based on a ratio or difference between the first temperature sensor T3 reading, or a computed correlate/function thereof, and the downstream temperature reading, or a computed correlate thereof.

One or both of the upstream and downstream temperature sensors might be used.

In view of the considerations discussed above, there may be value in using an upstream temperature sensor for detecting occurrence of a flow stop condition and to use a downstream temperature sensor for detecting a flow start condition. The threshold used for detecting the flow stop condition may be different than the threshold used for detecting the flow start condition.

For example, in some embodiments, the controller may be adapted to detect at least a transition from a flow start condition to a flow stop condition based on a ratio or a difference between the first temperature sensor signal T3 and an upstream temperature sensor signal (e.g. T2, T6 or T4), or a correlate thereof (and preferably without reference to the downstream sensor). The controller may be adapted to detect at least a transition from a flow stop condition to a flow start condition based on a ratio or a difference between the first temperature sensor signal T3 and a downstream temperature sensor signal (e.g. T1 or T8), or a correlate thereof (and preferably without reference to the upstream temperature sensor).

It is further noted that, for determining the ratio or difference between the first sensor T3 and either the upstream sensor (e.g. T2, T6, or T4) or the downstream sensor (e.g. T1 or T8), a microprocessor can be used, or analog circuitry could be used. In the latter case for example, reference is again made to Fig. 9 and also Fig. 11 which each show circuit arrangements which incorporate respective resistor divider arrangements for generating a signal indicative of a ratio between an output of T3 and an output of a further temperature sensor (T1 in Fig. 9a, T5 in Fig. 9b and T5 again in Fig. 11). The same circuit arrangements could be used in the present set of embodiments, just replacing T1 or T5 as appropriate with the upstream or downstream sensor for which a ratio with T3 is desired to be determined.

Fig. 25 illustrates various possible placements for temperature sensors. In some embodiments, the upstream and downstream temperature sensors located on the same side of the tubing as the heater (i.e. T1, T2) might be omitted and just the upstream and downstream temperature sensors on the opposite radial side of the tubing to the heater elements (i.e. T8, T6) may be used, i.e. on the same side of the tubing as T3. This may have some manufacturing advantages in simplifying the mechanical design or wiring with all temperature sensors on the same side of the tubing.

In some embodiments, both upstream and downstream temperature sensors are included so as to allow the device to adapt to fluid flow in either direction. This may occur if the user attaches the device to the tubing in the wrong orientation. It would also potentially simplify usability if the device was direction insensitive and could be attached in either orientation to a tubing. For example, the controller may be adapted to automatically determine an orientation relative to flow based on analysis of the temperature sensor signals (methods for determining flow direction have already been discussed above). However, this is not essential, and instead the device may be designed for use in a pre-determined orientation relative to flow, so that it is known in advance which of the sensors is upstream and which is downstream of the heater.

Thus, in some embodiments, the device may comprise at least a first further temperature sensor (e.g. T2, T6 and/or T4) and a second further temperature sensor (e.g. T1 and/or T8), and wherein the device is arranged in use such that the first further temperature sensor is positioned at a location longitudinally offset from the heating element in a first direction, e.g. for sensing a temperature of the fluid either upstream or downstream of the heating element, and wherein the device is arranged in use such that the second further temperature sensor is positioned longitudinally offset from the heating element in a second direction opposite to the first, e.g. for sensing a temperature of the fluid either downstream or upstream.

In some embodiments, the controller may be adapted to determine which of the first and second further temperature sensors is located upstream of the heating element and which is located downstream of the heating element. This might be done based on a comparison of temperature signal patterns of the first and second further temperature sensors. It might be done based on use of an orientation sensor, as discussed further below. It might be done with a user input, such as a switch. However, this is not essential.

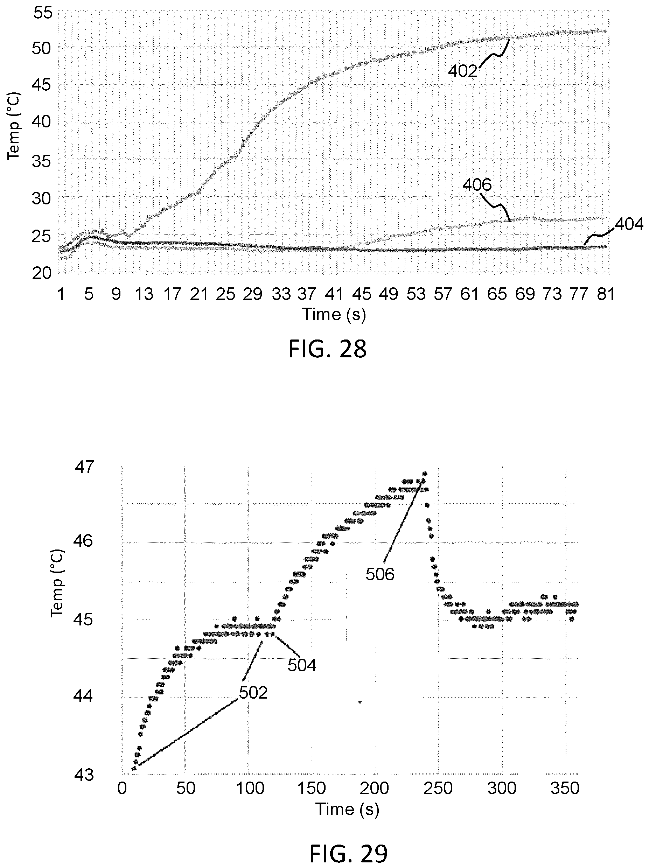

A further realization of the inventor is that, when the flow sensor is arranged for sensing flow through a tubing which is vertically oriented, e.g. a hanging IV line, gravity causes a small amount of convective heat flow upward when the fluid flow is otherwise stopped. When there is gross flow of the fluid, the convective effects are overwhelmed. However, when flow stops, the data shows that the temperature sensor that is gravitationally higher than the heater will start to warm up due to convection. This can be seen in the illustrative graph of Fig. 28. This shows the temperature of the fluid in the tubing upstream of the heater location by 1.6 cm (line 406), at T3 (line 402), and at a downstream location from the heater by 1.6 cm (line 404).

The tubing in this example is hanging vertically below a drip chamber so the upstream sensor 406 is physically above the heater and the downstream sensor 404 is physically below the heater. It is noted that the sensor which is gravitationally higher may not always be the upstream sensor, such as when the tubing is hanging down from a patient bed. Thus, for consideration of convective heating effects, it is the relative gravitational positioning of each sensor relative to the heater which is the relevant factor.

In Fig. 28 the gross flow stops at 10 seconds and, after another 25 seconds, the temperature of the fluid in the upstream sensor 406 starts to rise due to convection. Though this effect is relatively small compared to the heat at T3, it can be very significant if the upstream sensor is being used as a reference or ambient temperature for the sensor T3 radially opposed to the heater. This is particularly the case when one is trying to lower the flow start/stop decision threshold temperature in sensor T3 to be closer to the temperature of the upstream sensor.

This challenge may be at least partially addressed by the above-proposed use of both an upstream and downstream temperature sensor and wherein the upstream sensor is used as a reference or ambient temperature sensor to determine flow stop, and the downstream sensor is used to determine flow restart. It is to be noted that, in the graph of Fig. 28, the downstream sensor 404 is not affected by long periods of flow stoppage because it is gravitationally lower than the heater so does not receive convective flow. Thus, once the flow has been determined to be stopped, the controller may switch to using the downstream sensor in combination with T3 to detect flow restart.

One challenge with this approach is that it is difficult to be certain *a priori* the physical or gravitational orientation of the upstream and downstream sensors relative to the heater. If the device has been installed on the tubing upside down or if it was installed correctly but the tubing is hanging down from the patient bed such that the device is inverted, then it would be the downstream sensor that is gravitationally above the heater. To address this, it may be beneficial to provide an orientation sensor integrated in the device. This might include an accelerometer, e.g. a 3D accelerometer, or inclinometer to measure the direction of gravity. This can be used by the controller to determine which of the various sensors to use in combination with T3 for the flow stop determination and for the flow start determination.

Thus, in some embodiments, the device may further comprise an orientation sensing means, for example comprising an accelerometer or IMU or inclinometer, for sensing an orientation of the device, and wherein the controller is adapted to determine which of the first and second further temperature sensors is located gravitationally higher than the other of the first and second further temperature sensors based on an output from the orientation sensing means.

In some embodiments, the controller is adapted to determine the flow parameter or condition based on a difference or ratio between an output from the gravitationally lower of the first and second further temperature sensors and an output of the first temperature sensor (T3). This may be done in some cases only when the flow is in a flow stop condition, since only then are convective heating effects significant.

Thus, in some embodiments, the controller may be adapted to detect at least a transition from a flow start condition to a flow stop condition (when there is no convection effect) based on a ratio or a difference between the first temperature sensor T3 and the upstream temperature sensor (whether this is gravitationally lower or higher), or a correlate thereof (and preferably without reference to the other further sensor) and adapted to detect at least a transition from a flow stop condition to a flow start condition (when there is the potential for convective flow) based on a ratio or a difference between the first temperature sensor T3 and the gravitationally lower temperature sensor, or a correlate thereof (and preferably without reference to the other further temperature sensor).

It is possible that the physical orientation of the device may change with time. It would be of benefit to filter the time response of any accelerometer signal to be the same or close to the time response of any convective flow so that the choice of which sensor to use for start/stop determination will be closely matched to how the convective heat is actually flowing. In effect one can add signal filtering to the accelerometer signal to model the convective heat flow. Another way of saying this is that one may process the gravitationally higher sensor output to compensate for any convective heating effects at any given time.

Thus, the controller may in some embodiments be adapted to determine which of the first and second further temperature sensors is located gravitationally higher than the other. In some embodiments, this information may be used to compensate for convective heating effects between the heater and a temperature sensor located gravitationally above the heater.

For example, in some embodiments, the device comprises an orientation sensing means, for example comprising an accelerometer or IMU or inclinometer, for sensing an orientation of the device, and wherein the orientation sensing means is adapted to generate an orientation output indicative of an angle of inclination of the device (e.g. relative to a gravitational vertical). Following detection of a flow stop condition, the controller may be adapted to apply a (e.g. pre-determined) model or function configured to provide an output indicative of a predicted convective heating influence on the further temperature sensor which is gravitationally higher than the heating element as a function of: an angle of inclination of the device, and of a time duration since a beginning of the flow stop condition. The controller may be further adapted to: apply a correction/compensation to a temperature output from the gravitationally higher of the further temperature sensors based on an output from the model. In particular, this correction/compensation may be to negate or offset the estimated convective heating contribution to the temperature output from the gravitationally higher temperature sensor.

The model or function may define mappings between inputs comprising: an angle of inclination of the device, and a time duration since a beginning of the flow stop condition, and an output comprising a predicted additional temperature component of the output of the further temperature sensor which is gravitationally higher.

To arrive at the model or function of the convective flow, the following may be done. For a given tubing size, measurements may be made of a typical time response between flow stoppage and first registering a convective heating influence in the temperature output of a gravitationally higher upstream or downstream temperature sensor. Furthermore, one can record a magnitude of the convective heating effect on the temperature output of the gravitationally higher sensor as a function of time from flow stoppage. This can furthermore be done for each of a series of different angles of inclination of the device. In this way, a reference dataset is obtained which permits mapping from a measured angle of inclination from the device (using an integrated orientation sensor) in combination with a measured time duration since flow stoppage, onto an estimated temperature contribution to the temperature output from the gravitationally higher temperature sensor. This estimated contribution can then be subtracted or offset from the temperature output of the gravitationally higher temperature sensor. In this way, the effect of the convective flow can be removed.

Thus, this allows creation of a model relating the time since flow stopped, the angle of inclination and the convective heating contribution. This assumes a static longitudinal spacing between the heating element 22 and the sensor which is gravitationally higher. Values of the model can be interpolated to derive estimated temperature contributions for angles of inclination or time points which lie in-between the values recorded in the reference dataset.

Furthermore, it may be possible to create an equation that predicts the temperature rise of the gravitationally higher sensor from the inclination of the device and the time since flow has stopped, and thus compensate for the convective heat flow.

Furthermore, based upon the convective heating model discussed above, in some embodiments, a selection of the sensors used to determine the flow stop/start condition may be adapted. For example, a scenario may be considered in which a convective heating effect at a gravitationally higher temperature sensor is insignificant during a period of 30 seconds after flow stop, and becomes significant after 30 seconds. In this case, to determine flow start the controller may utilize the gravitationally higher temperature sensor in combination with the first sensor T3 during the 30 second period, and switch to the gravitationally lower sensor in combination with the first sensor T3 thereafter.

Furthermore, this switching between use of the temperature outputs of the upstream vs downstream sensors need not be a binary selection. In some embodiments, the controller may be adapted to determine the flow parameter or condition based on a weighted combination of the outputs from the upstream and downstream temperature sensors. This can be done in different ways. In some embodiments, the controller may be adapted to determine the flow parameter or condition based on an output from T3 in combination with a weighted sum of the outputs of the upstream and downstream sensors. In some embodiments, a separate determination may be made of the flow parameter or condition using each of: T3 in combination with the upstream sensor; and T3 in combination with the downstream sensor, and wherein each separate determination is weighted. The weightings may be determined based for example on a time duration since flow was detected to have stopped and/or the gravitational angle of the device. For example, the flow parameter or condition being determined may be a transition from a flow stop condition to a flow start condition. The ultimate binary decision of whether flow has started may be based on a 'vote' between the weighted decision A factor (using the upstream sensor) and the weighted decision B factor (using the downstream sensor). Note that the weightings may change over time if, for example, the time since flow stop is one of the weighting factors.

A further possibility, as an alternative to any of the above considerations regarding which of the further sensors is upstream/downstream and which is gravitationally higher or lower would be to determine which of the two further sensors has the lower temperature output, and detect flow stop/start state based on a ratio or difference between a temperature output of T3 and this lower-reading temperature sensor, and using one or more thresholds. This is based on a realization that a simpler way to make the flow start/stop decision not be influenced by convection may be to simply compare the temperature reading of sensor T3 to the lower of the temperature readings of sensors T1 or T2 (or any other pair of upstream/downstream sensors). Whichever sensor is lower in temperature will be the one which is not influenced by convection, regardless of whether it is upstream or downstream. For example, if the temperature of the first temperature sensor, T3, is close to the lowest of the first and second further temperature sensors (e.g. a difference is less than a defined threshold) then there is non-zero fluid flow. This decision uses a threshold. If the temperature at the first sensor, T3, is substantially higher than the lower of first and second further temperature sensors (e.g. a difference is greater than a define thresholds), then there is no flow. This decision uses a further threshold. If the temperature at the first temperature sensor, T3, drops suddenly relative to the lower of the first and second further temperature sensors (e.g. a difference signal exhibits a decline of a threshold gradient), this may indicate that flow has restarted. This may use a threshold rate of change.

Thus, in some embodiments, the device comprises at least a first further temperature sensor and a second further temperature sensor, the first further temperature sensor longitudinally offset from the heating element along the lumen of the tubing in a first direction and the second further temperature sensor longitudinally offset from the heating element along the lumen of the tubing in a second direction opposite to the first. The controller may be adapted to: compare the temperature signal outputs of the first and second further temperature sensors; select, from the first and second further sensors, the sensor with the lower temperature output; and determine the flow parameter or condition based on a difference or ratio between an output from the selected sensor and an output from the first temperature sensor (T3), or a correlate thereof, and based on a set of one or more thresholds corresponding to different flow parameters or conditions.

For example, the controller may be adapted to compute a difference or ratio signal by taking a difference or ratio between an output of the selected one of the first and second further temperature sensors and the output of the first temperature sensor . The controller may determine that flow has stopped if a difference signal is less than a first pre-defined threshold. The controller may determine that flow is non-zero (flow is present) if the difference signal is less than a greater pre-defined threshold (the same threshold or a different threshold). The controller may be adapted to determine that flow has transitioned from a stop state to a start state responsive to detecting a drop in the difference signal of a negative gradient which exceeds a negative gradient threshold.

A further realization of the inventor is that it may be advantageous to limit the temperature of the heater to avoid overheating. This could lead to damaging of the tubing. For example, overheating might occur where there is air in the tubing or if the heater is turned on without the tubing present, or to avoid overheating the fluid when it first flows into the heater region, or to not be a temperature hazard if the operator were to touch it if no tubing is present. To this end, the device may include means for directly or indirectly sensing a temperature of the heating element, and wherein the controller is adapted to adjust/regulate a heating power of the heating element in part in dependence upon the temperature of the heating element, for example for maintaining the temperature of the heating element below a threshold. For example, a separate temperature sensing element may be provided for sensing the heater temperature, e.g. sensor T7 in the illustration of Fig. 25. The heater power may be controlled in a feedback loop to keep temperature below a threshold. Alternatively, a heating element may be provided having means for sensing its own temperature, for example a thermistor, wherein power to the thermistor induces self-heating, and wherein the measured resistance across the thermistor provides an indication of the temperature of the thermistor.

The various embodiments of the flow sensing device discussed in this document can also be used to detect air-in-line.

In other words, in some embodiments, the controller is adapted to detect flow parameters or conditions which includes detection of air in the tubing.

For example, this can be detected based on detecting a rising temperature signal from the temperature sensor T3 radially opposite the heating element, or a correlate or function thereof.

For example, the controller may be adapted to detect presence of air between the heating element 22 and the temperature sensor T3 based on detecting a rise in the temperature sensor signal, or a correlate thereof, for a threshold time period, or which exhibits a threshold gradient, or which exceeds a threshold temperature.

An alternative approach is to use a heating element with means for sensing directly or indirectly the temperature of the heating element, for example a heating thermistor, wherein variation in a voltage across the thermistor is used as an indicator or temperature of the thermistor. Alternatively, a separate temperature sensor may be provided for sensing the temperature of the heating element 22. The temperature of the heater can be used to detect air in the tubing. In this case, the temperature of the heater distinctly increases when liquid fluid is replaced with air. The response to air using this configuration has been found to be faster than the response exhibited using the radially opposed temperature sensor T3. Thus, this signal may be preferably for detecting the air before it gets infused into the patient.

Thus, in some embodiments, it is proposed to include and use the radially opposed temperature sensor T3 for sensing fluid flow conditions such as flow stop/start, or flow direction, and to include and use, in addition, a means for directly or indirectly sensing a temperature of the heating element 22 to detect presence of air.

This combination would thus effectively detect the cessation and resumption of liquid flow as well as quickly detect the presence of air in the tubing.

Once air has been detected in the tubing, it would be preferable to stop the flow of liquid in the tubing to avoid the introduction of dangerous air into an artery.

To this end, in some embodiments, the device may further include a valve, for example a pinch valve, and wherein the device is arranged in use such that the pinch valve is actuatable to occlude fluid flow through the tube, and wherein the controller is adapted to actuate the valve to occlude the tubing responsive to detection of air in the tubing. The valve preferably is at a location fluidly downstream from the heating element and temperature sensor.

One problem to be overcome is that the energy needed to actuate a mechanical pinch valve could require more energy from the battery than is available, particularly near the end of its service life. To overcome this problem, the inventor proposes that in some embodiments, the device may include a manually chargeable energy storage means, chargeable by manual application of a force, for example against a biasing element, and wherein the actuation of the valve is powered by release of energy stored in the manually chargeable energy storage means, e.g. by releasing a catch/latch.

For example, energy might be stored in a spring while the enclosure lid is being closed by the operator's hand. In this way, the only electrical energy required would be that needed to release the mechanically charged actuator, e.g. by releasing a catch/latch.

In some embodiments, the device may include a housing adapted to couple to an outside wall of the tubing; the housing accommodating the heating element and the at least one temperature sensor, and wherein the housing, when coupled to the tubing, is adapted to hold the heating element in contact with the tubing and hold the temperature sensor in contact with the tubing; and wherein the device is configured such that the action of coupling the housing the tubing acts to charge the energy storage means. For example, closing hinged parts of the housing about the tubing may charge the energy storage means.

In some embodiments, the device may include a switch arranged to be activated upon coupling of the device to a tubing, e.g. upon coupling a housing of the device to the tubing. For example, the switch may be arranged to activate when a lid of the housing is closed. Activation of the switch may be configured to trigger power ON to the device, and begin sensing operations. In embodiments where the housing incorporates a cavity or groove to accommodate the tubing, there may be a physically depressable switch within the cavity or groove which is activated upon proper insertion of the tubing, and wherein the device automatically powers ON, and/or provides sensory feedback to the user, upon activation of this switch. There may additionally or alternatively be a switch on the outside of the device to manually turn the power ON to the device.

There may be a button on the device to stop an alarm if desired, or to switch an audible alarm between soft and loud, or to switch a visible alarm between ON and OFF.

It is desirable to stop the sounding of an alarm quickly once an acceptable amount of flow is detected. However, it may be desirable to have different amounts of time before starting an alarm when fluid stops, depending upon the circumstances. This may be achieved by either having a means to select the activation time on one device, or to have multiple device models, each with its own activation time. Activation times ranging between 30 seconds and 5 minutes would be useful.

Thus, in some embodiments, where the device comprises a housing which holds the temperature sensor and heater element (as opposed to these being integrated in the tubing or in the lumen for instance), one or more features may be provided to the housing to minimize the disruption caused by liquids originating outside the tubing.

Consistent with any of the embodiments described in this document, the device may further include any one or more of the following advantageous features.

In some embodiments, the device described in this application could be integrated into a drip chamber to form a "smart drip chamber". This design would be less complicated since the orientation with respect to gravity would be known. The device may be provided sterile, such that the heater and sensor(s) may be arranged in use touching, or nearly touching, the fluid itself. In this way, the measured signal levels may be higher, or the heater power could set at a lower level to achieve the same signal strength, thereby reducing power consumption, or the temperature rise in the fluid could be less. It would also work to put sensor T3 at a location circumferentially adjacent to the heater, neither upstream nor downstream, so that the volume of fluid between them would be very small, such that it would be highly sensitive to flow start and stop.

In some embodiments, the device may include a human-readable readout display, and wherein the controller is adapted to control the readout display to display an indication of an amount of time that has elapsed since flow has stopped. This could be a digital or analog display of the time in seconds or minutes or hours for example. It could also be a non-numeric display to give a graphical or qualitative indication of time, such as a bar graph or pie chart, or a display that changes color in response to the elapsed time.

In some of the embodiments described previously, the device employs close thermal contact between the heater 22 and the tubing 20 at location 32. It would be of benefit in some embodiments therefore to determine before use that close thermal contact is present and to alert the user if better positioning of the tubing is required. If the temperature of the heater element 22 can be measured, it is possible to detect the quality of that thermal contact by putting a known amount of energy into the heater and monitoring the temperature response of the heater. A poor thermal connection between the heater and the tubing will result in more rapid rate of change of the heater temperature, or a higher resulting asymptotic temperature, than if a good thermal connection exists, because there is no thermal "load" on the heater if it is not touching the tubing. Thus in some embodiments, the controller is adapted to control the heater to generate a defined thermal stimulus, e.g. a thermal pulse, and to measure a temperature response of the heater (e.g. by monitoring a voltage across the heating elements, e.g. where the heating element is a thermistor), and to compare the temperature response against a threshold or other criterion to determine a quality of thermal contact.

In some embodiments, the controller may be adapted to control generation of an alert responsive to detection that flow has stopped for longer than a predetermined time, such as 30 seconds. If an additional amount of time has passed with flow still stopped, such as 60 seconds, the alert level could escalate to indicate greater urgency. Examples of this escalation are a flashing of lights, faster flashing of lights, a different color of lights, louder or more noticeable audible indication, or additional information provided over a wireless link. In other words, the controller may be adapted to implement a multi-stage alert, wherein an alert having first sensory characteristics is generated responsive to detection of flow having stopped for a first threshold time period, and an alert having second sensory characteristics is generated responsive to detection of flow having stopped for a second threshold time period, longer than the first. For example, the second sensory characteristics may include, compared to the first sensory characteristics, a greater volume of an audible alert, a greater beeping frequency of a beeping alert, a different pitch or tone of an audible alert, a different color of a visible alert, a greater flashing frequency of a visible alert.

Embodiments discussed above have been described with reference to a sensing configuration comprising a heating element and temperature sensing element arranged substantially radially opposed to one another. However, various options and features discussed above can also be advantageously applied to a variant configuration in which a flow condition or parameter is detected based on sensing a temperature of the heating element itself. One example is a heater element with a separate temperature sensor integral to the heater. Another example is a transistor acting as both a power dissipating heater element and where its semiconductor junction voltage or body diode voltage is also monitored to determine the temperature of that junction, However, a preferred embodiment, for achieving a low-cost design is to use a negative or positive temperature coefficient thermistor. The thermistor may be energized with sufficient current to generate a self-heating output, and simultaneously monitoring a resistance across the thermistor as a means of monitoring its temperature. This thermistor approach may be preferred since it can be implemented very inexpensively and provides a large temperature readout signal for further processing.

Thus, another aspect of the invention is a flow sensing device for sensing a fluid flow through a tubing, comprising a heating unit, the heating unit comprising a heating element and means for directly or indirectly sensing variation in a temperature of the heating element, wherein the device is arranged in use to hold the heating element in thermal communication with a lumen of the tubing, for transferring heat to fluid flowing in the tubing. The device further comprises a controller operatively coupled to the heating element and sensing means, and wherein the controller is adapted to detect a flow condition or parameter (e.g. stop/start condition, or presence of air in the tubing at the location of the heating element), based on: controlling the heating element to dissipate a heating power, and simultaneously using the sensing means to sense variations in the temperature of the heating element while the heating element is being controlled.

The heating unit may be a single unitary component.

In some embodiments, the heating unit comprises a self-sensing heating element, the sensing of the temperature of the heating element being achieved by sampling an electrical characteristic of the heating element. For example, by sampling a voltage across the heating element to detect a resistance of the heating element, the resistance being indicative of temperature. In some embodiments, the self-sensing heating element is a thermistor, and wherein heating is achieved by application of an electrical supply to the thermistor creating self-heating, and sensing is achieved by sampling an electrical characteristic of the thermistor while said electrical supply is being applied. For example, the thermistor is driven with a constant current, and a voltage across the thermistor is simultaneously sensed, whereby the voltage gives an indication of the resistance, and whereby the resistance gives an indication of temperature.

An experiment was performed in which a negative temperature coefficient (NTC) thermistor of 100 ohms at 25 degrees Centigrade was energized with 31.4 mA of constant current so that it dissipates approximately 100 mW of power at 25 degrees C, and the voltage across the thermistor was measured to determine its resistance using Ohm's Law. This resistance was then converted to temperature using the Steinhart-Hart equation as is well known by practitioners in the art. This thermistor was pressed against IV tubing in a fixture constructed from foam core boards. Water was used as the fluid in the tubing, and the flow rate was controlled by an infusion pump set for 100 ml/hour. The voltage across the thermistor was measured with a data acquisition system and the resulting voltage measurements were converted to resistance of the thermistor by dividing the reported voltage by the 31.4 mA of energizing current to get resistance, and further converted to temperature.

The results are shown in Fig. 29. The average power dissipation in the thermistor for this data was on the order of 50 mW at this elevated temperature. The initial 120 seconds of data acquisition show the thermistor heating to an asymptotic temperature of about 45 degrees C during the flow of the water. The water is flowing between the two points 502. The water flow was then stopped (point 504) and the temperature of the heating element climbs very distinctly until flow was resumed at 240 seconds. A sharp drop in temperature can be seen once flow resumes (point 506) and the water is carrying the heat away from the heater. It is thus clear that the temperature of the heater element changes with flow, and that if the heater element is also temperature sensing, then the presence or absence of flow can be determined.

Note that it is not necessary to explicitly calculate a numerical value for temperature in degrees or resistance in Ohms to employ this approach. The measurement of any parameter, or any combination of parameters, that shows a transition when flow starts or stops would be useful. For example, the data in Fig. 29 was calculated from raw analog-to-digital converter (ADC) counts, and the same transitions can be seen in these counts.

Also note that in the above experiment the power dissipated in the thermistor goes down as the resistance goes down since it is being energized at a constant current. If a constant power were being dissipated in the thermistor, rather than a constant current, as its resistance changes then there would be an even larger change in temperature.

One advantage to the use of a temperature sensing heater to detect fluid flow is that it is not necessary to position a circuit element across the tubing from the heater. This makes the mechanics of positioning heaters and temperature sensors simpler as all of the thermal elements can be in the same plane, such as next to the circuit board, avoiding the need to have a mechanism to position an opposing temperature element. This makes it easier for the design to accommodate a range of tubing diameters. A circuit to dissipate a constant power in the resistance of a thermistor preferably should not itself dissipate significant power beyond what is dissipated in the thermistor, so as to maximize battery life and avoid extraneous heat generating sources within the enclosure. This may be achieved by controlling current in the thermistor using pulse width modulation techniques that dissipate very little power, as opposed to a linear current control circuit which would dissipate power.

As discussed above, embodiments make use of a controller. The controller can be implemented in numerous ways, with software and/or hardware, to perform the various functions required. A processor is one example of a controller which employs one or more microprocessors that may be programmed using software or firmware (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

The controller referred to in this disclosure may comprise a single control/processing component or an assembly of control/processing components, some of which may be subcutaneous while others are outside the body corpus. Thus, steps described as carried out by the controller may be carried at by a plurality of control or processing components in some cases.

Examples of controller components that may be employed in various embodiments of the present disclosure include, but are not limited to, conventional microprocessors, application specific integrated circuits (ASICs), configurable logic devices and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. A device for sensing a fluid flow through a tubing line (12), comprising:
a heating element (22) and at least a first temperature sensor (T3, 24); and
a controller operatively coupled to the heating element (22) and first temperature sensor (T3, 24),
wherein the device is arranged in use to hold the heating element positioned in thermal communication with the fluid in the tubing at a first location (32), and the first temperature sensor positioned in thermal communication with the fluid in the tubing at a second location (34), wherein the second location is substantially radially opposite the first location across the lumen (5) of the tubing, and
wherein the controller is adapted to simultaneously:
control the heating element to dissipate heating power, and
detect, based on at least an output from the first temperature sensor, a binary flow stop/start condition indicative of whether fluid is stopped in the tubing or whether fluid is flowing through the tubing,
wherein the controller is adapted to detect a flow stop condition of the fluid based on detecting a temperature signal from the first temperature sensor, or a function or correlate thereof: (i) exceeding a threshold, (ii) exhibiting a rising trend for a threshold time duration or threshold number of signal sample points, or (iii) exhibiting a rise, measured over a specific time duration, which exceeds a threshold,
wherein the controller is adapted to detect a flow start condition of the fluid based on detecting a temperature signal from the first temperature sensor, or a function or correlate thereof: (i) falling below a threshold, (ii) exhibiting a declining trend for a threshold time duration or threshold number of signal sample points, or (iii) exhibiting a fall, measured over a specific time duration, exceeding a threshold, and
wherein the controller is further adapted, in at least one phase of operation, to adjust a heating power output of the heating element based upon changes in the detected flow stop/start condition, and
wherein said adjusting the heating power output in dependence upon changes in the detected flow stop/start condition comprises reducing the heating power output following detection of a flow stop condition, and
wherein
the device includes a housing (14) adapted to couple to an outside wall (20) of the tubing, the housing accommodating the heating element (22) and the at least first temperature sensor (24, T3), and wherein the housing, when coupled to the tubing, is adapted to hold the heating element in contact with an outside wall (20) of the tubing at the first location (32), and hold the first temperature sensor in contact with the outside wall of the tubing at the second location (34), or
the device includes at least a section of the tubing, and wherein the heating element (22) and the at least first temperature sensor (24) are each coupled to an outside wall of said section of tubing, at least partially integrated and/or embedded inside a wall of said section of tubing, or mounted inside the lumen of said section of tubing.

2. The device of claim 1,
wherein the controller is adapted to selectively operate the heating element in one of at least two heating power modes: a higher heating power mode and lower heating power mode, wherein at least an average heating power output in the higher heating power mode is greater than an average heating power output in the lower heating power mode, and
wherein said adjusting the heating power output in dependence upon changes in the detected flow stop/start condition comprises at least switching to the lower heating power mode following detection of a flow stop condition.

3. The device of claim 2, wherein, when the flow stop/start condition is in a non-zero flow state, the controller is adapted to operate the heating element in the higher power mode.

4. The device of any preceding claim, wherein, when the flow stop/start condition is a non-zero flow state, the controller is adapted to control the heating element to dissipate a substantially constant heating power.

5. The device any of claims 2-4, wherein the controller is adapted to reduce the power output of the heating element to the lower power mode responsive to detection of persistence of the flow stop condition for a threshold time period or a threshold number of temperature signal sample points, and/or responsive to detection of the output from the first temperature sensor, or a correlate thereof, exceeding a threshold level.

6. The device of claim 5, wherein, when the heating power output is in the lower power mode, the controller is adapted to detect transition of the fluid flow from the flow stop condition to the flow start condition based on:
detecting a negative inflection in an output from the first temperature sensor, or a correlate thereof, or
detecting a negative gradient in the first temperature sensor signal, or a correlate thereof, exceeding a pre-defined threshold gradient, or
detecting an output from the first temperature sensor, or a correlate thereof, falling below a threshold temperature.

7. The device of any of claims 2-6, wherein the controller is adapted to return the heating element to the higher heating power output mode following detection of a flow start condition.

8. The device of any preceding claim, wherein the device further includes one or more further temperature sensors arranged for sensing a temperature of fluid during use at a location upstream and/or downstream of the heating element, and wherein the flow stop/start condition is determined based on a function of respective outputs of the first temperature sensor and the one or more further temperature sensors.

9. The device of any preceding claim, wherein:
the device further includes at least one further temperature sensor (26, T5, T4, T2, T6); and
the device is arranged in use such that the at least one further temperature sensor is positioned for sensing either: an ambient temperature of the air in the environment of the device, or a temperature of the fluid at a location upstream from the heating element, and the controller is adapted to detect the flow stop/start condition based on outputs of both the first temperature sensor (24, T3) and the at least one further temperature sensor (26, T5, T4), based on compensating a temperature change measurement of the first temperature sensor using temperature change measurements of the at least one further temperature sensor,
and/or
the device is arranged in use such that the at least one further temperature sensor is positioned for sensing a temperature of the fluid at a location upstream from the heating element (22), and the controller is adapted to detect the flow stop/start condition based on a ratio or a difference between a signal from the first temperature sensor (T3), or a correlate thereof, and a signal from the further temperature sensor (T2, T6), or a correlate thereof.

10. The device of any preceding claim, wherein:
the device further includes at least one further temperature sensor (T1, T8);
the device is arranged in use such that the at least one further temperature sensor is positioned for sensing a temperature of the fluid at a location downstream from the heating element (22); and
wherein the controller is adapted to detect the flow stop/start condition based on outputs of both the first temperature sensor (T3), or a correlate thereof, and the at least one further temperature sensor (T1, T8), or a correlate thereof, and
preferably wherein the controller is adapted to detect at least a transition from a flow stop condition to a flow start condition based on a ratio or a difference between an output of the first temperature sensor, or a correlate thereof, and an output of the further temperature sensor, or a correlate thereof.

11. The device of any preceding claim, wherein:
the device comprises at least a first further temperature sensor (T2, T6) and a second further temperature sensor (T1, T8);
the device is arranged in use such that the first further temperature sensor (T2, T6) is positioned at a location longitudinally offset from the heating element (22) along the lumen of the tubing in a first direction, for sensing a temperature of the fluid either upstream or downstream of the heating element, and
the device is arranged in use such that the second further temperature sensor (T1, T8) is positioned longitudinally offset from the heating element (22) along the lumen of the tubing in a second direction, opposite to the first direction, for sensing a temperature of the fluid either downstream or upstream of the heating element.

12. The device of claim 11,
wherein the device further comprises an orientation sensing means, for example comprising an accelerometer or an inertial measurement unit (IMU) or inclinometer, for sensing an orientation of the device, and wherein the controller is adapted to determine which of the first and second further temperature sensors is located gravitationally higher than the other of the first and second further temperature sensors based on an output from the orientation sensing means, and
optionally wherein the controller is adapted to determine the flow stop/start condition based on a difference or ratio between an output from the gravitationally lower of the first and second further temperature sensors and an output of the first temperature sensor (T3).

13. The device of claim 12, wherein:
the orientation sensing means is adapted to generate an orientation output indicative of an angle of inclination of the device relative to a gravitational vertical direction;
following detection of a flow stop condition, the controller is adapted to determine a predicted convective heating influence on an output of the further temperature sensor which is gravitationally higher based on application of a model or function which defines mappings between inputs comprising: an angle of inclination of the device, and a time duration since a beginning of the flow stop condition, and an output comprising a predicted additional temperature component of the output of the further temperature sensor which is gravitationally higher; and
apply a correction/compensation to a temperature output from the gravitationally higher of the further temperature sensors based on the output from the model.

14. The device of claim 11, wherein the controller is adapted to:
compare the temperature signal outputs of the first and second further temperature sensors;
select, from the first and second further temperature sensors, the sensor with the lower temperature output;
determine the flow stop/start condition based on a difference or ratio between an output from the selected sensor and an output from the first temperature sensor (T3), or a correlate thereof, and based on a set of one or more thresholds.

15. A method for sensing fluid flow through a tubing line, comprising:
holding a heating element in thermal communication with fluid in the tubing at a first location;
simultaneously holding a temperature sensor in thermal communication with fluid in the tubing at a second location, wherein the second location is substantially radially opposite the first location across the lumen of the tubing;
controlling the heating element to dissipate heating power;
sensing a temperature output from the temperature sensor while the heating element is active; and
detecting, based on at least an output from the temperature sensor, a binary flow stop/start condition of fluid in the tubing indicative of whether fluid is stopped in the tubing or whether fluid is flowing through the tubing,
wherein the method comprises detecting a flow stop condition of the fluid based on detecting a temperature signal from the temperature sensor, or a function or correlate thereof: (i) exceeding a threshold, (ii) exhibiting a rising trend for a threshold time duration or threshold number of signal sample points, or (iii) exhibiting a rise, measured over a specific time duration, which exceeds a threshold, and
wherein the method comprises detecting a flow start condition of the fluid based on detecting a temperature signal from the temperature sensor, or a function or correlate thereof: (i) falling below a threshold, (ii) exhibiting a declining trend for a threshold time duration or threshold number of signal sample points, or (iii) exhibiting a fall, measured over a specific time duration, exceeding a threshold, and
wherein the method further comprises:
adjusting a heating power output of the heating element based upon changes in the detected flow stop/start condition,
wherein said adjusting the heating power output in dependence upon changes in the detected flow stop/start condition comprises reducing the heating power output following detection of a flow stop condition.

## Patentansprüche

1. Vorrichtung zum Erfassen eines Fluiddurchflusses durch eine Schlauchleitung (12), umfassend:
ein Heizelement (22) und mindestens einen ersten Temperatursensor (T3, 24); und
ein Steuergerät, das an das Heizelement (22) und den Temperatursensor (24, T3) wirkgekoppelt ist;
wobei die Vorrichtung im Gebrauch dazu angeordnet ist, das Heizelement in thermischer Kommunikation mit dem Fluid in dem Schlauch an einer ersten Stelle (32) positioniert und den ersten Temperatursensor in thermischer Kommunikation mit dem Fluid in dem Schlauch an einer zweiten Stelle (34) positioniert zu halten, wobei sich die zweite Stelle im Wesentlichen radial gegenüber der ersten Stelle über das Lumen (5) des Schlauchs befindet und wobei das Steuergerät angepasst ist, um gleichzeitig:
das Heizelement so zu steuern, dass es Heizleistung ableitet, und
basierend auf zumindest einer Ausgabe aus dem ersten Temperatursensor eine binäre Durchflussstopp-/Startbedingung zu erkennen, die dafür bezeichnend ist, ob Fluid in dem Schlauch gestoppt ist oder ob Fluid durch den Schlauch fließt,
wobei das Steuergerät dazu angepasst ist, eine Durchflussstoppbedingung des Fluids basierend auf dem Erkennen eines Temperatursignals von dem ersten Temperatursensor oder einer Funktion oder eines Korrelats davon zu erkennen: (i) Überschreiten eines Schwellenwerts, (ii) Aufweisen eines ansteigenden Trends für eine Schwellenzeitdauer oder Schwellenanzahl von Signalabtastpunkten oder (iii) Aufweisen eines Anstiegs, gemessen über eine bestimmte Zeitdauer, der einen Schwellenwert überschreitet,
wobei das Steuergerät dazu angepasst ist, eine Durchflussstartbedingung des Fluids basierend auf dem Erkennen eines Temperatursignals von dem ersten Temperatursensor oder einer Funktion oder eines Korrelats davon zu erkennen: (i) Unterschreiten eines Schwellenwerts, (ii) Aufweisen eines abnehmenden Trends für eine Schwellenzeitdauer oder Schwellenanzahl von Signalabtastpunkten oder (iii) Aufweisen eines Abfalls, gemessen über eine bestimmte Zeitdauer, der einen Schwellenwert überschreitet, und
wobei das Steuergerät ferner dazu angepasst ist, in mindestens einer Betriebsphase eine Heizleistungsabgabe des Heizelements basierend auf Änderungen in der erkannten Durchflussstopp-/Startbedingung einzustellen, und
wobei das Einstellen der Heizleistungsabgabe in Abhängigkeit von Änderungen in der erkannten Durchflussstopp-/Startbedingung das Reduzieren der Heizleistungsabgabe nach dem Erkennen einer Durchflussstoppbedingung umfasst, und
wobei die Vorrichtung ein Gehäuse (14) umfasst, das dazu angepasst ist, an eine Außenwand (20) des Schlauchs gekoppelt zu werden, wobei das Gehäuse das Heizelement (22) und den mindestens ersten Temperatursensor (24, T3) aufnimmt, und wobei das Gehäuse, wenn es an den Schlauch gekoppelt ist, dazu angepasst ist, das Heizelement in Kontakt mit einer Außenwand (20) des Schlauchs an der ersten Stelle (32) zu halten und den ersten Temperatursensor in Kontakt mit der Außenwand des Schlauchs an der zweiten Stelle (34) zu halten, oder
die Vorrichtung mindestens einen Abschnitt des Schlauchs beinhaltet und wobei das Heizelement (22) und der mindestens erste Temperatursensor (24) jeweils an eine Außenwand des Schlauchabschnitts gekoppelt sind, mindestens teilweise in eine Wand des Schlauchabschnitts integriert und/oder eingebettet sind oder in dem Inneren des Lumens des Schlauchabschnitts montiert sind.

2. Vorrichtung nach Anspruch 1,
wobei das Steuergerät dazu angepasst ist, das Heizelement selektiv in einem von mindestens zwei Heizleistungsmodi zu betreiben: einem Modus mit höherer Heizleistung und einem Modus mit niedrigerer Heizleistung, wobei mindestens eine durchschnittliche Heizleistungsabgabe in dem Modus mit höherer Heizleistung größer als eine durchschnittliche Heizleistungsabgabe in dem Modus mit niedrigerer Heizleistung ist, und
wobei das Einstellen der Heizleistungsabgabe in Abhängigkeit von Änderungen in der erkannten Durchflussstopp-/Startbedingung zumindest das Umschalten in den Modus mit geringerer Heizleistung nach dem Erkennen einer Durchflussstoppbedingung umfasst.

3. Vorrichtung nach Anspruch 2, wobei, wenn sich die Durchflussstopp-/Startbedingung in einem Nicht-Null-Durchflusszustand befindet, das Steuergerät dazu angepasst ist, das Heizelement in dem Modus mit höherer Leistung zu betreiben.

4. Vorrichtung nach einem vorhergehenden Anspruch, wobei, wenn die Durchflussstopp-/Startbedingung ein Nicht-Null-Durchflusszustand ist, das Steuergerät dazu angepasst ist, das Heizelement so zu steuern, dass es eine im Wesentlichen konstante Heizleistung ableitet.

5. Vorrichtung nach einem der Ansprüche 2 bis 4, wobei das Steuergerät dazu angepasst ist, die Leistungsabgabe des Heizelements in den Modus mit niedrigerer Leistung als Reaktion auf das Erkennen des Fortbestehens der Durchflussstoppbedingung für einen Schwellenzeitraum oder eine Schwellenanzahl von Temperatursignalabtastpunkten und/oder als Reaktion auf das Erkennen der Ausgabe aus dem ersten Temperatursensor oder eines Korrelats davon, die oder das einen Schwellenpegel überschreitet, zu reduzieren.

6. Vorrichtung nach Anspruch 5, wobei, wenn sich die Heizleistungsabgabe in dem Modus mit geringerer Leistung befindet, das Steuergerät dazu angepasst ist, einen Übergang des Fluiddurchflusses von der Durchflussstoppbedingung zu der Durchflussstartbedingung basierend auf Folgendem zu erkennen:
Erkennen einer negativen Biegung in einer Ausgabe aus dem ersten Temperatursensor oder einem Korrelat davon oder
Erkennen eines negativen Gradienten in dem ersten Temperatursensorsignal oder einem Korrelat davon, der oder das einen vordefinierten Schwellengradienten überschreitet, oder
Erkennen, dass eine Ausgabe von dem ersten Temperatursensor oder einem Korrelat davon unter eine Schwellentemperatur fällt.

7. Vorrichtung nach einem der Ansprüche 2 bis 6, wobei das Steuergerät dazu angepasst ist, das Heizelement, nach dem Erkennen einer Durchflussstartbedingung, in den Modus mit höherer Heizleistungsabgabe zurückzuführen.

8. Vorrichtung nach einem vorhergehenden Anspruch, wobei die Vorrichtung ferner einen oder mehrere weitere Temperatursensoren beinhaltet, die zum Erfassen einer Temperatur von Fluid während der Verwendung an einer Stelle stromaufwärts und/oder stromabwärts des Heizelements angeordnet sind, und wobei die Durchflussstopp-/Startbedingung basierend auf einer Funktion jeweiliger Ausgaben des ersten Temperatursensors und des einen oder der mehreren weiteren Temperatursensoren bestimmt wird.

9. Vorrichtung nach einem vorhergehenden Anspruch, wobei:
die Vorrichtung ferner mindestens einen weiteren Temperatursensor (26, T5, T4, T2, T6) beinhaltet; und
die Vorrichtung im Gebrauch so angeordnet ist, dass der mindestens eine weitere Temperatursensor zum Erfassen entweder einer Umgebungstemperatur der Luft in der Umgebung der Vorrichtung oder einer Temperatur des Fluids an einer Stelle stromaufwärts des Heizelements positioniert ist, und das Steuergerät dazu angepasst ist, die Durchflussstopp-/Startbedingung basierend auf Ausgaben sowohl des ersten Temperatursensors (24, T3) als auch des mindestens einen weiteren Temperatursensors (26, T5, T4) basierend auf dem Kompensieren einer Temperaturänderungsmessung des ersten Temperatursensors unter Verwendung von Temperaturänderungsmessungen des mindestens einen weiteren Temperatursensors zu erkennen, und/oder
die Vorrichtung im Gebrauch so angeordnet ist, dass der mindestens eine weitere Temperatursensor zum Erfassen einer Temperatur des Fluids an einer Stelle stromaufwärts des Heizelements (22) positioniert ist, und das Steuergerät dazu angepasst ist, die Durchflussstopp-/Startbedingung basierend auf einem Verhältnis oder einer Differenz zwischen einem Signal aus dem ersten Temperatursensor (T3) oder einem Korrelat davon und einem Signal aus dem weiteren Temperatursensor (T2, T6) oder einem Korrelat davon zu erkennen.

10. Vorrichtung nach einem vorhergehenden Anspruch, wobei:
die Vorrichtung ferner mindestens einen weiteren Temperatursensor (T1, T8) beinhaltet;
die Vorrichtung im Gebrauch so angeordnet ist, dass der mindestens eine weitere Temperatursensor zum Erfassen einer Temperatur des Fluids an einer Stelle stromabwärts des Heizelements (22) positioniert ist; und
wobei das Steuergerät dazu angepasst ist, die Durchflussstopp-/Startbedingung basierend auf Ausgaben sowohl des ersten Temperatursensors (T3) oder eines Korrelats davon als auch des mindestens einen weiteren Temperatursensors (T1, T8) oder eines Korrelats davon zu erkennen, und
vorzugsweise wobei das Steuergerät dazu angepasst ist, mindestens einen Übergang von einer Durchflussstoppbedingung zu einer Durchflussstartbedingung basierend auf einem Verhältnis oder einer Differenz zwischen einer Ausgabe des ersten Temperatursensors oder einem Korrelat davon und einer Ausgabe des weiteren Temperatursensors oder einem Korrelat davon zu erkennen.

11. Vorrichtung nach einem vorhergehenden Anspruch, wobei:
die Vorrichtung mindestens einen ersten weiteren Temperatursensor (T2, T6) und einen zweiten weiteren Temperatursensor (T1, T8) umfasst;
die Vorrichtung im Gebrauch so angeordnet ist, dass der erste weitere Temperatursensor (T2, T6) an einer Stelle längs versetzt von dem Heizelement (22) entlang des Lumens des Schlauchs in einer ersten Richtung positioniert ist, um eine Temperatur des Fluids entweder stromaufwärts oder stromabwärts des Heizelements zu erfassen, und
die Vorrichtung im Gebrauch so angeordnet ist, dass der zweite weitere Temperatursensor (Tl, T8) in Längsrichtung versetzt von dem Heizelement (22) entlang des Lumens des Schlauchs in einer zweiten Richtung entgegengesetzt zu der ersten Richtung positioniert ist, um eine Temperatur des Fluids entweder stromabwärts oder stromaufwärts des Heizelements zu erfassen.

12. Vorrichtung nach Anspruch 11,
wobei die Vorrichtung ferner eine Orientierungserfassungseinrichtung umfasst, die beispielsweise einen Beschleunigungsmesser oder eine Trägheitsmesseinheit (IMU) oder einen Neigungsmesser umfasst, um eine Orientierung der Vorrichtung zu erfassen, und wobei das Steuergerät dazu angepasst ist, basierend auf einer Ausgabe der Orientierungserfassungseinrichtung zu bestimmen, welcher von dem ersten und zweiten weiteren Temperatursensor sich gravitativ höher als der andere von dem ersten und zweiten weiteren Temperatursensor befindet,
optional wobei das Steuergerät dazu angepasst ist, die Durchflussstopp-/Startbedingung basierend auf einer Differenz oder einem Verhältnis zwischen einer Ausgabe des gravitativ niedrigeren des ersten und zweiten weiteren Temperatursensors und einer Ausgabe des ersten Temperatursensors (T3) zu bestimmen.

13. Vorrichtung nach Anspruch 12, wobei:
die Orientierungserfassungseinrichtung dazu angepasst ist, eine Orientierungsausgabe zu erzeugen, die für einen Neigungswinkel der Vorrichtung in Bezug auf eine vertikale Gravitationsrichtung bezeichnend ist;
nach dem Erkennen einer Durchflussstoppbedingung das Steuergerät dazu angepasst ist, einen vorhergesagten konvektiven Erwärmungseinfluss auf eine Ausgabe des weiteren Temperatursensors, der gravitativ höher ist basierend auf der Anwendung eines Modells oder einer Funktion zu bestimmen, das oder die Abbildungen zwischen
Eingaben definiert, die Folgendes umfassen: einen Neigungswinkel der Vorrichtung, und eine Zeitdauer seit einem Beginn der Durchflussstoppbedingung, und eine Ausgabe, die eine vorhergesagte zusätzliche Temperaturkomponente der Ausgabe des weiteren Temperatursensors umfasst, der gravitativ höher ist; und
eine Korrektur/Kompensation auf eine Temperaturausgabe von dem gravitativ höheren der weiteren Temperatursensoren basierend auf der Ausgabe von dem Modell anzuwenden.

14. Vorrichtung nach Anspruch 11, wobei das Steuergerät zu Folgendem angepasst ist:
Vergleichen der Temperatursignalausgaben des ersten und zweiten weiteren Temperatursensors;
Auswählen, aus dem ersten und zweiten weiteren Temperatursensor, des Sensors mit der niedrigeren Temperaturausgabe;
Bestimmen der Durchflussstopp-/Startbedingung basierend auf einer Differenz oder einem Verhältnis zwischen einer Ausgabe aus dem ausgewählten Sensor und einer Ausgabe aus dem ersten Temperatursensor (T3) oder einem Korrelat davon und basierend auf einem Satz von einem oder mehreren Schwellenwerten.

15. Verfahren zum Erfassen eines Fluiddurchflusses durch eine Schlauchleitung, umfassend:
Halten eines Heizelements in thermischer Kommunikation mit Fluid in dem Schlauch an einer ersten Stelle;
gleichzeitiges Halten eines Temperatursensors in thermischer Kommunikation mit Fluid in dem Schlauch an einer zweiten Stelle, wobei sich die zweite Stelle im Wesentlichen radial gegenüber der ersten Stelle über das Lumen des Schlauchs befindet;
Steuern des Heizelements derart, dass es Heizleistung ableitet;
Erfassen einer Temperaturausgabe aus dem Temperatursensor, während das Heizelement aktiv ist;
Erkennen, basierend auf zumindest einer Ausgabe aus dem Temperatursensor, einer binären Durchflussstopp-/Startbedingung von Fluid in dem Schlauch, der dafür bezeichnend ist, ob Fluid in dem Schlauch gestoppt ist oder ob Fluid durch den Schlauch fließt,
wobei das Verfahren das Erkennen einer Durchflussstoppbedingung des Fluids basierend auf dem Erkennen eines Temperatursignals aus dem Temperatursensor oder einer Funktion oder eines Korrelats davon umfasst: (i) Überschreiten eines Schwellenwerts, (ii) Aufweisen eines ansteigenden Trends für eine Schwellenzeitdauer oder Schwellenanzahl von Signalabtastpunkten oder (iii) Aufweisen eines Anstiegs, gemessen über eine bestimmte Zeitdauer, der einen Schwellenwert überschreitet, und
wobei das Verfahren das Erkennen einer Durchflussstartbedingung des Fluids basierend auf dem Erkennen eines Temperatursignals aus dem Temperatursensor oder einer Funktion oder eines Korrelats davon umfasst: (i) Unterschreiten eines Schwellenwerts, (ii) Aufweisen eines abnehmenden Trends für eine Schwellenzeitdauer oder einer Schwellenwert-
anzahl von Signalabtastpunkten oder (iii) Aufweisen eines Abfalls, gemessen über eine bestimmte Zeitdauer, der einen Schwellenwert überschreitet, und
wobei das Verfahren ferner Folgendes umfasst:
Einstellen einer Heizleistungsabgabe des Heizelements basierend auf Änderungen in der erkannten Durchflussstopp-/Startbedingung,
wobei das Einstellen der Heizleistungsabgabe in Abhängigkeit von Änderungen in der erkannten Durchflussstopp-/Startbedingung das Reduzieren der Heizleistungsabgabe nach dem Erkennen einer Durchflussstoppbedingung umfasst.

## Revendications

1. Dispositif pour la détection d'un écoulement de fluide à travers une conduite tubulaire (12), comprenant :
un élément chauffant (22) et au moins un premier capteur de température (T3, 24) ; et
un dispositif de commande couplé fonctionnellement à l'élément chauffant (22) et au premier capteur de température (T3, 24),
dans lequel le dispositif est agencé, lors de l'utilisation, pour maintenir l'élément chauffant positionné en communication thermique avec le fluide dans le tube à un premier emplacement (32), et le premier capteur de température positionné en communication thermique avec le fluide dans le tube à un second emplacement (34), dans lequel le second emplacement est sensiblement radialement opposé au premier emplacement à travers la lumière (5) du tube, et dans lequel le dispositif de commande est adapté pour, simultanément :
commander l'élément chauffant pour dissiper une puissance de chauffage, et
détecter, sur la base d'au moins une sortie provenant du premier capteur de température, une condition binaire d'arrêt/démarrage d'écoulement indiquant si un fluide est à l'arrêt dans le tube ou si un fluide s'écoule à travers le tube,
dans lequel le dispositif de commande est adapté pour détecter une condition d'arrêt d'écoulement du fluide sur la base de la détection d'un signal de température provenant du premier capteur de température, ou d'une fonction ou d'un corrélat de celui-ci : (i) dépassant un seuil, (ii) présentant une tendance à la hausse pendant une durée seuil ou un nombre seuil de points d'échantillonnage de signal, ou (iii) présentant une hausse, mesurée sur une durée spécifique, qui dépasse un seuil,
dans lequel le dispositif de commande est adapté pour détecter une condition de démarrage d'écoulement du fluide sur la base de la détection d'un signal de température provenant du premier capteur de température, ou d'une fonction ou d'un corrélat de celui-ci : (i) tombant en dessous d'un seuil, (ii) présentant une tendance à la baisse pendant une durée seuil ou un nombre seuil de points d'échantillonnage de signal, ou (iii) présentant une baisse, mesurée sur une durée spécifique, dépassant un seuil, et
dans lequel le dispositif de commande est en outre adapté, dans au moins une phase de fonctionnement, pour régler une sortie de puissance de chauffage de l'élément chauffant sur la base de changements de la condition d'arrêt/démarrage d'écoulement détectée, et
dans lequel ledit réglage de la sortie de puissance de chauffage en fonction de changements de la condition d'arrêt/démarrage d'écoulement détectée comprend la réduction de la sortie de puissance de chauffage après la détection d'une condition d'arrêt d'écoulement, et
dans lequel le dispositif comprend un boîtier (14) adapté pour être couplé à une paroi extérieure (20) du tube, le boîtier recevant l'élément chauffant (22) et l'au moins premier capteur de température (24, T3), et dans lequel le boîtier, lorsqu'il est couplé au tube, est adapté pour maintenir l'élément chauffant en contact avec une paroi extérieure (20) du tube au premier emplacement (32), et maintenir le premier capteur de température en contact avec la paroi extérieure du tube au second emplacement (34), ou
le dispositif comprend au moins une section du tube, et dans lequel l'élément chauffant (22) et l'au moins premier capteur de température (24) sont chacun couplés à une paroi extérieure de ladite section de tube, au moins partiellement intégrés et/ou noyés à l'intérieur d'une paroi de ladite section de tube, ou montés à l'intérieur de la lumière de ladite section de tube.

2. Dispositif de la revendication 1,
dans lequel le dispositif de commande est adapté pour faire fonctionner sélectivement l'élément chauffant dans l'un d'au moins deux modes de puissance de chauffage : un mode de puissance de chauffage supérieure et un mode de puissance de chauffage inférieure, dans lequel au moins une sortie de puissance de chauffage moyenne dans le mode de puissance de chauffage supérieure est supérieure à une sortie de puissance de chauffage moyenne dans le mode de puissance de chauffage inférieure, et
dans lequel ledit réglage de la sortie de puissance de chauffage en fonction de changements de la condition d'arrêt/démarrage d'écoulement détectée comprend au moins la commutation vers le mode de puissance de chauffage inférieure après la détection d'une condition d'arrêt d'écoulement.

3. Dispositif de la revendication 2, dans lequel, lorsque la condition d'arrêt/démarrage d'écoulement est dans un état d'écoulement non nul, le dispositif de commande est adapté pour faire fonctionner l'élément chauffant dans le mode de puissance supérieure.

4. Dispositif d'une quelconque revendication précédente, dans lequel, lorsque la condition d'arrêt/démarrage d'écoulement est un état d'écoulement non nul, le dispositif de commande est adapté pour commander l'élément chauffant afin de dissiper une puissance de chauffage sensiblement constante.

5. Dispositif de l'une quelconque des revendications 2 à 4, dans lequel le dispositif de commande est adapté pour réduire la sortie de puissance de l'élément chauffant au mode de puissance inférieure en réponse à la détection de la persistance de la condition d'arrêt d'écoulement pendant une période seuil ou un nombre seuil de points d'échantillonnage de signal de température, et/ou en réponse à la détection du dépassement d'un niveau seuil par la sortie provenant du premier capteur de température, ou par un corrélat de celle-ci.

6. Dispositif de la revendication 5, dans lequel, lorsque la sortie de puissance de chauffage est dans le mode de puissance inférieure, le dispositif de commande est adapté pour détecter une transition de l'écoulement de fluide de la condition d'arrêt d'écoulement à la condition de démarrage d'écoulement sur la base de :
la détection d'une inflexion négative dans une sortie provenant du premier capteur de température, ou d'un corrélat de celle-ci, ou
la détection d'un gradient négatif dans le signal de premier capteur de température, ou d'un corrélat de celui-ci, dépassant un gradient seuil prédéfini, ou
la détection d'une sortie provenant du premier capteur de température, ou d'un corrélat de celle-ci, tombant en dessous d'une température seuil.

7. Dispositif de l'une quelconque des revendications 2 à 6, dans lequel le dispositif de commande est adapté pour ramener l'élément chauffant au mode de sortie de puissance de chauffage supérieure après la détection d'une condition de démarrage d'écoulement.

8. Dispositif d'une quelconque revendication précédente, dans lequel le dispositif comprend en outre un ou plusieurs capteurs de température supplémentaires agencés pour détecter une température de fluide, lors de l'utilisation, à un emplacement en amont et/ou en aval de l'élément chauffant, et dans lequel la condition d'arrêt/démarrage d'écoulement est déterminée sur la base d'une fonction de sorties respectives du premier capteur de température et du ou des capteurs de température supplémentaires.

9. Dispositif d'une quelconque revendication précédente, dans lequel :
le dispositif comprend en outre au moins un capteur de température supplémentaire (26, T5, T4, T2, T6) ; et
le dispositif est agencé, lors de l'utilisation, de sorte que l'au moins un capteur de température supplémentaire soit positionné pour détecter soit : une température ambiante de l'air dans l'environnement du dispositif, soit une température du fluide à un emplacement en amont de l'élément chauffant, et le dispositif de commande est adapté pour détecter la condition d'arrêt/démarrage d'écoulement sur la base de sorties à la fois du premier capteur de température (24, T3) et de l'au moins un capteur de température supplémentaire (26, T5, T4), sur la base de la compensation d'une mesure de changement de température du premier capteur de température à l'aide de mesures de changement de température de l'au moins un capteur de température supplémentaire, et/ou
le dispositif est agencé, lors de l'utilisation, de sorte que l'au moins un capteur de température supplémentaire soit positionné pour détecter une température du fluide à un emplacement en amont de l'élément chauffant (22), et le dispositif de commande est adapté pour détecter la condition d'arrêt/démarrage d'écoulement sur la base d'un rapport ou d'une différence entre un signal provenant du premier capteur de température (T3), ou d'un corrélat de celui-ci, et un signal provenant du capteur de température supplémentaire (T2, T6), ou d'un corrélat de celui-ci.

10. Dispositif d'une quelconque revendication précédente, dans lequel :
le dispositif comprend en outre au moins un capteur de température supplémentaire (T1, T8) ;
le dispositif est agencé, lors de l'utilisation, de sorte que l'au moins un capteur de température supplémentaire soit positionné pour détecter une température du fluide à un emplacement en aval de l'élément chauffant (22) ; et
dans lequel le dispositif de commande est adapté pour détecter la condition d'arrêt/démarrage d'écoulement sur la base de sorties à la fois du premier capteur de température (T3), ou d'un corrélat de celui-ci, et de l'au moins un capteur de température supplémentaire (T1, T8), ou d'un corrélat de celui-ci, et
de préférence, dans lequel le dispositif de commande est adapté pour détecter au moins une transition d'une condition d'arrêt d'écoulement à une condition de démarrage d'écoulement sur la base d'un rapport ou d'une différence entre une sortie du premier capteur de température, ou un corrélat de celle-ci, et une sortie du capteur de température supplémentaire, ou un corrélat de celle-ci.

11. Dispositif d'une quelconque revendication précédente, dans lequel :
le dispositif comprend au moins un premier capteur de température supplémentaire (T2, T6) et un second capteur de température supplémentaire (T1, T8) ;
le dispositif est agencé, lors de l'utilisation, de sorte que le premier capteur de température supplémentaire (T2, T6) soit positionné à un emplacement longitudinalement décalé par rapport à l'élément chauffant (22) le long de la lumière du tube dans une première direction, pour détecter une température du fluide soit en aval, soit en amont de l'élément chauffant, et
le dispositif est agencé, lors de l'utilisation, de sorte que le second capteur de température supplémentaire (T1, T8) soit positionné de manière décalée longitudinalement par rapport à l'élément chauffant (22) le long de la lumière du tube, dans une seconde direction, opposée à la première direction, pour détecter une température du fluide soit en aval soit en amont de l'élément chauffant.

12. Dispositif de la revendication 11,
dans lequel le dispositif comprend en outre un moyen de détection d'orientation, comprenant par exemple un accéléromètre ou une unité de mesure inertielle (IMU) ou un inclinomètre, pour détecter une orientation du dispositif, et dans lequel le dispositif de commande est adapté pour déterminer lequel des premier et second capteurs de température supplémentaires est situé plus haut que l'autre des premier et second capteurs de température supplémentaires par rapport à la gravité, sur la base d'une sortie provenant du moyen de détection d'orientation, et
éventuellement, dans lequel le dispositif de commande est adapté pour déterminer la condition d'arrêt/démarrage d'écoulement sur la base d'une différence ou d'un rapport entre une sortie provenant d'un capteur qui est situé plus bas par rapport à la gravité parmi les premier et second capteurs de température supplémentaires et une sortie du premier capteur de température (T3).

13. Dispositif de la revendication 12, dans lequel :
le moyen de détection d'orientation est adapté pour générer une sortie d'orientation indiquant un angle d'inclinaison du dispositif par rapport à une direction verticale gravitationnelle ;
après la détection d'une condition d'arrêt d'écoulement, le dispositif de commande est adapté pour déterminer une influence de chauffage par convection prédite sur une sortie du capteur de température supplémentaire qui est situé plus haut par rapport à la gravité, sur la base de l'application d'un modèle ou d'une fonction qui définit des correspondances entre des entrées comprenant : un angle d'inclinaison du dispositif, et une durée écoulée depuis le début de la condition d'arrêt d'écoulement, et une sortie comprenant une composante de température supplémentaire prédite de la sortie du capteur de température supplémentaire qui est situé plus haut par rapport à la gravité ; et
appliquer une correction/compensation à une sortie de température provenant du capteur qui est situé plus haut par rapport à la gravité parmi les capteurs de température supplémentaires, sur la base de la sortie provenant du modèle.

14. Dispositif de la revendication 11, dans lequel le dispositif de commande est adapté pour :
comparer les sorties de signal de température des premier et second capteurs de température supplémentaires ;
sélectionner, à partir des premier et second capteurs de température supplémentaires, le capteur présentant la sortie de température inférieure ;
déterminer la condition d'arrêt/démarrage d'écoulement sur la base d'une différence ou d'un rapport entre une sortie provenant du capteur sélectionné et une sortie provenant du premier capteur de température (T3), ou un corrélat de celle-ci, et sur la base d'un ensemble d'un ou plusieurs seuils.

15. Procédé pour la détection d'écoulement de fluide à travers une conduite tubulaire, comprenant :
le maintien d'un élément chauffant en communication thermique avec un fluide dans le tube à un premier emplacement ;
le maintien simultané d'un capteur de température en communication thermique avec un fluide dans le tube à un second emplacement, dans lequel le second emplacement est sensiblement radialement opposé au premier emplacement à travers la lumière du tube ;
la commande de l'élément chauffant pour dissiper une puissance de chauffage ;
la détection d'une sortie de température provenant du capteur de température lorsque l'élément chauffant est actif ; et
la détection, sur la base d'au moins une sortie provenant du capteur de température, d'une condition binaire d'arrêt/démarrage d'écoulement de fluide dans le tube indiquant si un fluide est à l'arrêt dans le tube ou si un fluide s'écoule à travers le tube,
dans lequel le procédé comprend la détection d'une condition d'arrêt d'écoulement du fluide sur la base de la détection d'un signal de température provenant du capteur de température, ou d'une fonction ou d'un corrélat de celui-ci : (i) dépassant un seuil, (ii) présentant une tendance à la hausse pendant une durée seuil ou un nombre seuil de points d'échantillonnage de signal, ou (iii) présentant une hausse, mesurée sur une durée spécifique, qui dépasse un seuil, et
dans lequel le procédé comprend la détection d'une condition de démarrage d'écoulement du fluide sur la base de la détection d'un signal de température provenant du capteur de température, ou d'une fonction ou d'un corrélat de celui-ci : (i) tombant en dessous d'un seuil, (ii) présentant une tendance à la baisse pendant une durée seuil ou un nombre seuil de points d'échantillonnage de signal, ou (iii) présentant une baisse, mesurée sur une durée spécifique, dépassant un seuil, et
dans lequel le procédé comprend en outre :
le réglage d'une sortie de puissance de chauffage de l'élément chauffant sur la base de changements de la condition d'arrêt/démarrage d'écoulement détectée,
dans lequel ledit réglage de la sortie de puissance de chauffage en fonction de changements de la condition d'arrêt/démarrage d'écoulement détectée comprend la réduction de la sortie de puissance de chauffage après la détection d'une condition d'arrêt d'écoulement.
